(19) Europäisches Patentamt
European Patent Office
Office européen des brevets

(11) **EP 4 364 746 A1**

(12) **EUROPEAN PATENT APPLICATION**
published in accordance with Art. 153(4) EPC

(43) Date of publication:
**08.05.2024 Bulletin 2024/19**

(21) Application number: **22832192.3**

(22) Date of filing: **30.06.2022**

(51) International Patent Classification (IPC):
*A61K 35/17* (2015.01)      *C07K 14/54* (2006.01)

(52) Cooperative Patent Classification (CPC):
**A61K 39/464412; A61K 39/4611; A61K 39/4631;**
**A61K 39/464419; C07K 14/54;** A61K 2239/26;
A61K 2239/31; A61K 2239/38

(86) International application number:
**PCT/CN2022/103037**

(87) International publication number:
**WO 2023/274380 (05.01.2023 Gazette 2023/01)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority:  **01.07.2021  CN 202110749536**

(71) Applicant: **Ningbo T-MAXIMUM**
**Biopharmaceuticals Co., Ltd.**
**Ningbo, Zhejiang 315336 (CN)**

(72) Inventors:
• **SHANG, Xiaoyun**
**Hangzhou Bay New Area Ningbo, Zhejiang**
**315336 (CN)**
• **LI, Jialu**
**Hangzhou Bay New Area Ningbo, Zhejiang**
**315336 (CN)**
• **JIANG, Haijuan**
**Hangzhou Bay New Area Ningbo, Zhejiang**
**315336 (CN)**
• **WANG, Dan**
**Hangzhou Bay New Area Ningbo, Zhejiang**
**315336 (CN)**

• **MA, Shaowen**
**Hangzhou Bay New Area Ningbo, Zhejiang**
**315336 (CN)**
• **SHEN, Hui**
**Hangzhou Bay New Area Ningbo, Zhejiang**
**315336 (CN)**
• **XU, Fanli**
**Hangzhou Bay New Area Ningbo, Zhejiang**
**315336 (CN)**
• **WANG, Shichen**
**Hangzhou Bay New Area Ningbo, Zhejiang**
**315336 (CN)**
• **CHEN, Weijie**
**Hangzhou Bay New Area Ningbo, Zhejiang**
**315336 (CN)**

(74) Representative: **Weickmann & Weickmann**
**PartmbB**
**Postfach 860 820**
**81635 München (DE)**

Remarks:
The complete document including Reference
Table(s) and the Sequence Listing(s) can be
downloaded from the EPO website

(54) **UNIVERSAL CAR-T CELL TARGETING IL13Ra2, PREPARATION METHOD THEREFOR AND APPLICATION THEREOF**

(57)    The present application relates to a modified immune effector cell, wherein the functions of a T cell antigen receptor (TCR) and major histocompatibility complexes (MHCI, MHCII) in the modified immune effector cell are inhibited in a T cell, and the modified immune effector cell comprises a chimeric antigen receptor (CAR) targeting IL13Rα2. The modified immune effector cell of the present application knocks out TCR and HLA-A genes expressed by the cell while recognizing surface antigens of tumor cells, so that multiple effects of improving the anti-tumor effect of CAR-T cells, prolonging the survival time of the cells, and reducing the immune rejection response caused by allogeneic cell therapy are achieved.

EP 4 364 746 A1

**Description**

**TECHNICAL FIELD**

The present application relates to the field of biomedicine, and in particular to a universal CAR-T cell targeting IL13Rα2, a preparation method therefor, and use thereof.

**BACKGROUND**

IL-13Rα2 is highly expressed in various tumors, such as malignant gliomas, pancreatic cancer, ovarian cancer, breast cancer, colon cancer, and the like. Among them, glioma refers to a tumor originating from neuroglial cells, and is the most common primary intracranial tumor. The incidence of primary malignant brain tumors has increased year by year in the past 30 years. Among primary malignant central nervous system tumors, glioblastoma (GBM, WHO grade IV) has the highest incidence rate, accounting for 46.1%. In glioma, the expression of IL-13Rα2 is positively correlated with the grade of glioma. High-grade glioma has high expression of IL-13Rα2, and low-grade or normal brain tissues have low expression or no expression of IL-13Rα2. Moreover, the high expression of IL-13Rα2 is associated with poor prognosis. In gliomas, the high expression of IL-13Rα2 promotes proliferation, migration, and invasion of glioma cells, and this effect is achieved by activating the SRC/PI3K/ATK/mTOR pathway. The treatment of glioma mainly includes surgical resection of tumor, and is combined with radiotherapy, chemotherapy, and other multimodality treatment methods. The chemotherapy for glioma has been a combination regimen applying a single drug and multiple drugs. However, the treatment effect on the existing glioma is not good overall. Adults with high-grade glioma have a 1-year survival rate of 30% and a 5-year survival rate of 13%, and the median survival time for adults with anaplastic glioma and GBM is 2-3 years and 1 year, respectively. Therefore, a new method for treating glioma is established in clinical need.

Katarzyna C. Pituch's research team in the United States and Baylor College of Medicine jointly conducted a preclinical study of IL-13Rα2-targeted CAR-T. The study found that in an *in vitro* tumor cell model killing experiment, IL-13Rα2 CAR-T cells showed a specific killing function on tumor cells with positive IL-13Rα2 expression; meanwhile, the research team found that the survival rate of a tumor-bearing mouse experimental group administrated IL-13Rα2 CAR-T was significantly higher than that of other experimental groups. In addition, Christine E. Brown's team reported a case receiving IL-13Rα2 CAR-T treatment in the NEJM journal. A patient with recurrent malignant gliomas whose primary lesion was located on the right temporal lobe participated in the researchers' clinical study. The patient received 6 intralesional injections of IL-13Rα2 CAR-T in sequence, and the whole treatment lasted for about 7.5 months. Then, the illness state of the patient was controlled, the lesion did not progress or recur. The patient did not have grade 3 or higher adverse events in the whole treatment period, and the number of immune cells and the level of cytokines in the cerebrospinal fluid were remarkably increased, showing the good safety and the clinical treatment application prospect of IL-13Rα2 CAR-T.

However, autologous T cells of patients have difficulty expanding *in vitro* or reduced functions, resulting in an insufficient amount or poor quality of the CAR-T cell products prepared. Universal CAR-T cells are T cells isolated from healthy donors, and the prepared CAR-T cells not only have high amplification efficiency and strong activity, but also have an improved infection positive rate. However, the universal CAR-T also faces the problems of graft versus host disease (GVHD) and immune rejection. The CRISPR/Cas9 system is the most commonly used gene editing method, and can be used for producing T cells with TCR deficiency and HLA class I molecule deficiency, and for reducing immune rejection caused by allogeneic cell therapy. Compared with a traditional CAR-T cell product, for the allogeneic universal CAR-T cells, the preparation cost is greatly reduced, and the preparation period is shortened. The universal CAR-T not only expands the recognition range of antigens, but also can change immunosuppressive microenvironment through gene knockout, and is applied to the treatment of malignant hematologic tumors and solid tumors.

**SUMMARY**

The present invention aims to prepare a universal CAR-T cell targeting IL-13Rα2, which knocks out TCR and HLA-A genes expressed by the cell while recognizing surface antigens of tumor cells, so that multiple effects of improving the anti-tumor effect of the CAR-T cell, prolonging the survival time of the cell, and reducing the immune rejection response caused by allogeneic cell therapy are achieved.

In one aspect, the present application provides an immune effector cell, wherein the functions of a T cell antigen receptor (TCR) and major histocompatibility complexes (MHCI, MHCII) in the immune effector cell are inhibited in a cell, and the immune effector cell comprises a chimeric antigen receptor (CAR) targeting IL13Rα2.

In certain embodiments, the chimeric antigen receptor (CAR) targeting IL13Rα2 comprises a targeting moiety comprising at least one complementarity-determining region (CDR) of an antibody heavy chain variable region (VH), wherein the VH comprises an amino acid sequence set forth in SEQ ID NO: 18.

In certain embodiments, the VH comprises an amino acid sequence set forth in SEQ ID NO: 16 or SEQ ID NO: 17.

In certain embodiments, the chimeric antigen receptor (CAR) targeting IL13Rα2 comprises a targeting moiety comprising a VH, wherein the VH comprises a heavy chain complementarity-determining region 1 (HCDR1), a heavy chain complementarity-determining region 2 (HCDR2), and a heavy chain complementarity-determining region 3 (HCDR3), and the HCDR3 comprises an amino acid sequence set forth in SEQ ID NO: 3.

In certain embodiments, the HCDR2 comprises an amino acid sequence set forth in SEQ ID NO: 2.

In certain embodiments, the HCDR1 comprises an amino acid sequence set forth in SEQ ID NO: 1.

In certain embodiments, the VH comprises: the HCDR1 comprising the amino acid sequence set forth in SEQ ID NO: 1, the HCDR2 comprising the amino acid sequence set forth in SEQ ID NO: 2, and the HCDR3 comprising the amino acid sequence set forth in SEQ ID NO: 3.

In certain embodiments, the VH comprises a heavy chain framework region 1 (HFR1), a heavy chain framework region 2 (HFR2), a heavy chain framework region 3 (HFR3), and a heavy chain framework region 4 (HFR4), and the HFR1 comprises an amino acid sequence set forth in SEQ ID NO: 4.

In certain embodiments, the HFR1 comprises an amino acid sequence set forth in SEQ ID NO: 8 or SEQ ID NO: 12.

In certain embodiments, the HFR2 comprises an amino acid sequence set forth in SEQ ID NO: 5.

In certain embodiments, the HFR2 comprises an amino acid sequence set forth in SEQ ID NO: 9 or SEQ ID NO: 13.

In certain embodiments, the HFR3 comprises an amino acid sequence set forth in SEQ ID NO: 6.

In certain embodiments, the HFR3 comprises an amino acid sequence set forth in SEQ ID NO: 10 or SEQ ID NO: 14.

In certain embodiments, the HFR4 comprises an amino acid sequence set forth in SEQ ID NO: 7.

In certain embodiments, the HFR4 comprises an amino acid sequence set forth in SEQ ID NO: 11 or SEQ ID NO: 15.

In certain embodiments, the VH comprises HFR1, HFR2, HFR3, and HFR4, and the HFR1, HFR2, HFR3, and HFR4 are selected from

i) the HFR1 comprising the amino acid sequence set forth in SEQ ID NO: 8, the HFR2 comprising the amino acid sequence set forth in SEQ ID NO: 9, the HFR3 comprising the amino acid sequence set forth in SEQ ID NO: 10, and the HFR4 comprising the amino acid sequence set forth in SEQ ID NO: 11; and

ii) the HFR1 comprising the amino acid sequence set forth in SEQ ID NO: 12, the HFR2 comprising the amino acid sequence set forth in SEQ ID NO: 13, the HFR3 comprising the amino acid sequence set forth in SEQ ID NO: 14, and the HFR4 comprising the amino acid sequence set forth in SEQ ID NO: 15.

In certain embodiments, the VH comprises an amino acid sequence set forth in SEQ ID NO: 18.

In certain embodiments, the VH comprises an amino acid sequence set forth in SEQ ID NO: 16 or SEQ ID NO: 17.

In certain embodiments, the targeting moiety further comprises at least one CDR of an antibody light chain variable region (VL), wherein the VL comprises an amino acid sequence set forth in SEQ ID NO: 36.

In certain embodiments, the VL comprises an amino acid sequence set forth in SEQ ID NO: 34 or SEQ ID NO: 35.

In certain embodiments, the targeting moiety comprises at least one CDR of the VH and at least one CDR of the VL, and the VH and the VL are selected from:

i) the VH comprising the amino acid sequence set forth in SEQ ID NO: 16, and the VL comprising the amino acid sequence set forth in SEQ ID NO: 34; and

ii) the VH comprising the amino acid sequence set forth in SEQ ID NO: 17, and the VL comprising the amino acid sequence set forth in SEQ ID NO: 35.

In certain embodiments, the chimeric antigen receptor (CAR) targeting IL13Rα2 comprises a targeting moiety comprising a VL, wherein the VL comprises a light chain complementarity-determining region 1 (LCDR1), a light chain complementarity-determining region 2 (LCDR2), and a light chain complementarity-determining region 3 (LCDR3), and the LCDR1 comprises an amino acid sequence set forth in SEQ ID NO: 19.

In certain embodiments, the LCDR2 comprises an amino acid sequence set forth in SEQ ID NO: 20.

In certain embodiments, the LCDR3 comprises an amino acid sequence set forth in SEQ ID NO: 21.

In certain embodiments, the VL comprises: the LCDR1 comprising the amino acid sequence set forth in SEQ ID NO: 19, the LCDR2 comprising the amino acid sequence set forth in SEQ ID NO: 20, and the LCDR3 comprising the amino acid sequence set forth in SEQ ID NO: 21.

In certain embodiments, the VL comprises a light chain framework region 1 (LFR1), a light chain framework region 2 (LFR2), a light chain framework region 3 (LFR3), and a light chain framework region 4 (LFR4), and the LFR1 comprises an amino acid sequence set forth in SEQ ID NO: 22.

In certain embodiments, the LFR1 comprises an amino acid sequence set forth in SEQ ID NO: 26 or SEQ ID NO: 30.

In certain embodiments, the LFR2 comprises an amino acid sequence set forth in SEQ ID NO: 23.

In certain embodiments, the LFR2 comprises an amino acid sequence set forth in SEQ ID NO: 27 or SEQ ID NO: 31.

In certain embodiments, the LFR3 comprises an amino acid sequence set forth in SEQ ID NO: 24.

In certain embodiments, the LFR3 comprises an amino acid sequence set forth in SEQ ID NO: 28 or SEQ ID NO: 32.

In certain embodiments, the LFR4 comprises an amino acid sequence set forth in SEQ ID NO: 25.

In certain embodiments, the LFR4 comprises an amino acid sequence set forth in SEQ ID NO: 29 or SEQ ID NO: 33.

In certain embodiments, the VL comprises LFR1, LFR2, LFR3, and LFR4, and the LFR1, LFR2, LFR3, and LFR4 are selected from

i) the LFR1 comprising the amino acid sequence set forth in SEQ ID NO: 26, the LFR2 comprising the amino acid sequence set forth in SEQ ID NO: 27, the LFR3 comprising the amino acid sequence set forth in SEQ ID NO: 28, and the LFR4 comprising the amino acid sequence set forth in SEQ ID NO: 29; and

ii) the LFR1 comprising the amino acid sequence set forth in SEQ ID NO: 30, the LFR2 comprising the amino acid sequence set forth in SEQ ID NO: 31, the LFR3 comprising the amino acid sequence set forth in SEQ ID NO: 32, and the LFR4 comprising the amino acid sequence set forth in SEQ ID NO: 33.

In certain embodiments, the VL comprises an amino acid sequence set forth in SEQ ID NO: 36.

In certain embodiments, the VL comprises an amino acid sequence set forth in SEQ ID NO: 34 or SEQ ID NO: 35.

In certain embodiments, the targeting moiety comprises the VH comprising the amino acid sequence set forth in SEQ ID NO: 18 and the VL comprising the amino acid sequence set forth in SEQ ID NO: 36.

In certain embodiments, the VH and the VL are selected from:

i) the VH comprising the amino acid sequence set forth in SEQ ID NO: 16, and the VL comprising the amino acid sequence set forth in SEQ ID NO: 34; and

ii) the VH comprising the amino acid sequence set forth in SEQ ID NO: 17, and the VL comprising the amino acid sequence set forth in SEQ ID NO: 35.

In certain embodiments, the targeting moiety includes a full-length antibody, a Fab, a single-chain variable fragment (scFv), or a single-domain antibody (VHH).

In certain embodiments, the targeting moiety includes the scFv.

In certain embodiments, the targeting moiety comprises an amino acid sequence set forth in SEQ ID NO: 41 or SEQ ID NO: 44.

In certain embodiments, the targeting moiety comprises an amino acid sequence set forth in SEQ ID NO: 39, SEQ ID NO: 40, SEQ ID NO: 42, or SEQ ID NO: 43.

In certain embodiments, the chimeric antigen receptor (CAR) targeting IL13Rα2 comprises a targeting moiety comprising at least one complementarity-determining region (CDR) of an antibody heavy chain variable region (VH), wherein the VH comprises an amino acid sequence set forth in SEQ ID NO: 56.

In certain embodiments, the chimeric antigen receptor (CAR) targeting IL13Rα2 comprises a targeting moiety comprising a VH, wherein the VH comprises a heavy chain complementarity-determining region 1 (HCDR1), a heavy chain complementarity-determining region 2 (HCDR2), and a heavy chain complementarity-determining region 3 (HCDR3), and the HCDR3 comprises an amino acid sequence set forth in SEQ ID NO: 55.

In certain embodiments, the HCDR2 comprises an amino acid sequence set forth in SEQ ID NO: 54.

In certain embodiments, the HCDR1 comprises an amino acid sequence set forth in SEQ ID NO: 53.

In certain embodiments, the VH comprises: the HCDR1 comprising the amino acid sequence set forth in SEQ ID NO: 53, the HCDR2 comprising the amino acid sequence set forth in SEQ ID NO: 54, and the HCDR3 comprising the amino acid sequence set forth in SEQ ID NO: 55.

In certain embodiments, the VH comprises an amino acid sequence set forth in SEQ ID NO: 56.

In certain embodiments, the targeting moiety further comprises at least one CDR of an antibody light chain variable region (VL), wherein the VL comprises an amino acid sequence set forth in SEQ ID NO: 60.

In certain embodiments, the targeting moiety comprises at least one CDR of the VH and at least one CDR of the VL, the VH comprises the amino acid sequence set forth in SEQ ID NO: 56, and the VL comprises the amino acid sequence set forth in SEQ ID NO: 60.

In certain embodiments, the chimeric antigen receptor (CAR) targeting IL13Rα2 comprises a targeting moiety comprising a VL, wherein the VL comprises a light chain complementarity-determining region 1 (LCDR1), a light chain complementarity-determining region 2 (LCDR2), and a light chain complementarity-determining region 3 (LCDR3), and the LCDR1 comprises an amino acid sequence set forth in SEQ ID NO: 57.

In certain embodiments, the LCDR2 comprises an amino acid sequence set forth in SEQ ID NO: 58.

In certain embodiments, the LCDR3 comprises an amino acid sequence set forth in SEQ ID NO: 59.

In certain embodiments, the VL comprises: the LCDR1 comprising the amino acid sequence set forth in SEQ ID NO: 57, the LCDR2 comprising the amino acid sequence set forth in SEQ ID NO: 58, and the LCDR3 comprising the amino acid sequence set forth in SEQ ID NO: 59.

In certain embodiments, the VL comprises an amino acid sequence set forth in SEQ ID NO: 60.

In certain embodiments, the targeting moiety comprises the VH comprising the amino acid sequence set forth in SEQ ID NO: 56 and the VL comprising the amino acid sequence set forth in SEQ ID NO: 60.

In certain embodiments, the targeting moiety includes a full-length antibody, a Fab, a single-chain variable fragment (scFv), or a single-domain antibody (VHH).

In certain embodiments, the targeting moiety includes the scFv.

In certain embodiments, the targeting moiety comprises an amino acid sequence set forth in SEQ ID NO: 61 or SEQ ID NO: 62.

In certain embodiments, the CAR comprises a transmembrane domain, wherein the transmembrane domain comprises a transmembrane domain derived from one or more proteins selected from the group consisting of: CD8A, CD8B, CD28, CD3ε (CD3e), 4-1BB, CD4, CD27, CD7, PD-1, TRAC, TRBC, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L (CD154), TIM1, CD226, DR3, CD45, CD80, CD86, CD9, CD16, CD22, CD33, CD37, CD64, and SLAM.

In certain embodiments, the transmembrane domain comprises a transmembrane domain derived from CD8A.

In certain embodiments, the transmembrane domain comprises an amino acid sequence set forth in any one of SEQ ID NO: 69 to SEQ ID NO: 117.

In certain embodiments, the CAR comprises an intracellular co-stimulatory signaling domain, wherein the intracellular co-stimulatory signaling domain comprises an intracellular co-stimulatory signaling domain derived from one or more proteins selected from the group consisting of: CD28, 4-1BB (CD137), CD27, CD2, CD7, CD8A, CD8B, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88.

In certain embodiments, the intracellular co-stimulatory signaling domain is derived from a co-stimulatory signaling domain of 4-1BB.

In certain embodiments, the intracellular co-stimulatory signaling domain comprises an amino acid sequence set forth in any one of SEQ ID NO: 118 to SEQ ID NO: 150.

In certain embodiments, the CAR comprises an intracellular signaling domain, wherein the intracellular signaling domain comprises an intracellular signaling domain derived from one or more proteins selected from the group consisting of: CD3ζ, CD3δ, CD3γ, CD3ε, CD79a, CD79b, FceRIγ, FceRIβ, FcyRIIa, bovine leukemia virus gp30, Epstein-Barr virus (EBV) LMP2A, simian immunodeficiency virus PBj14 Nef, DAP10, DAP-12, and a domain comprising at least one ITAM.

In certain embodiments, the intracellular signaling domain comprises a signaling domain derived from CD3ζ.

In certain embodiments, the intracellular signaling domain comprises an amino acid sequence set forth in any one of SEQ ID NO: 134, SEQ ID NO: 138, SEQ ID NO: 139, and SEQ ID NO: 151 to SEQ ID NO: 161.

In certain embodiments, the CAR comprises a hinge region between the targeting moiety and the transmembrane domain, and the hinge region comprises a hinge region derived from one or more proteins selected from the group consisting of: CD28, IgG1, IgG4, IgD, 4-1BB, CD4, CD27, CD7, CD8A, PD-1, ICOS, OX40, NKG2D, NKG2C, FcεRIγ, BTLA, GITR, DAP10, TIM1, SLAM, CD30, and LIGHT.

In certain embodiments, the hinge region comprises a hinge region derived from CD8A.

In certain embodiments, the hinge region comprises an amino acid sequence set forth in any one of SEQ ID NO: 162 to SEQ ID NO: 183.

In certain embodiments, a non-targeting moiety of the chimeric antigen receptor comprises a transmembrane domain of CD8A molecule, a hinge region of CD8A, an intracellular co-stimulatory signaling domain of 4-1BB, and an intracellular signaling domain of CD3ζ.

In certain embodiments, the non-targeting moiety of the chimeric antigen receptor comprises an amino acid sequence set forth in SEQ ID NO: 67.

In certain embodiments, the chimeric antigen receptor further comprises a signal peptide fragment, and the C-terminus of the signal peptide fragment is linked to the N-terminus of the targeting moiety.

In certain embodiments, the signal peptide fragment includes a CD8A signal peptide fragment.

In certain embodiments, the signal peptide fragment comprises an amino acid sequence set forth in SEQ ID NO: 184.

In certain embodiments, the chimeric antigen receptor comprises an amino acid sequence set forth in any one of SEQ ID NO: 45 and SEQ ID NO: 46.

In certain embodiments, the immune effector cell includes a human cell.

In certain embodiments, the immune effector cell includes a T cell, a B cell, a natural killer cell (NK cell), a macrophage, an NKT cell, a monocyte, a dendritic cell, a granulocyte, a lymphocyte, a leukocyte, and/or a peripheral blood mononuclear cell.

In certain embodiments, the immune effector cell includes an autologous or non-autologous immune effector cell.

In certain embodiments, the immune effector cell comprises a modified immune effector cell, wherein the modification comprises down-regulation of the expression and/or activity of one or more of immune rejection-related genes.

In certain embodiments, the immune rejection-related gene is selected from one or more of the following groups: TRAC, TRBC, HLA-A, HLA-B, B2M, and CIITA.

In certain embodiments, the expression and/or activity of the TRAC gene and the HLA-A gene in the modified immune effector cell is down-regulated as compared to a corresponding unmodified cell.

In certain embodiments, the expression and/or activity of the CIITA gene in the modified immune effector cell is not down-regulated as compared to the corresponding unmodified cell.

In certain embodiments, the expression and/or activity of the B2M gene in the modified immune effector cell is not down-regulated as compared to the corresponding unmodified cell.

In certain embodiments, the expression and/or activity of the TRAC gene and the HLA-A gene in the modified immune effector cell is down-regulated as compared to a corresponding wild-type cell.

In certain embodiments, the expression and/or activity of the B2M gene in the modified immune effector cell is not down-regulated as compared to the corresponding wild-type cell.

In certain embodiments, the expression and/or activity of the CIITA gene in the modified immune effector cell is not down-regulated as compared to the corresponding wild-type cell.

In certain embodiments, the down-regulation of the expression level and/or activity of the gene includes down-regulating the expression and/or activity of a nucleic acid molecule encoding the gene; and/or down-regulating the expression and/or activity of a protein product encoded by the gene.

In certain embodiments, the modification comprises: gene knockout, gene mutation, and/or gene silencing.

In certain embodiments, the modification comprises knocking out either of two TRAC alleles and knocking out either of two HLA-A alleles in the immune effector cell.

In certain embodiments, the modification comprises knocking out the two TRAC alleles and knocking out either of the two HLA-A alleles in the immune cell.

In certain embodiments, the modification comprises knocking out an exon of the TRAC gene and knocking out an exon of the HLA-A gene in the immune cell.

In certain embodiments, the modification comprises administering to the immune effector cell one or more substances selected from the group consisting of: antisense RNA, siRNA, shRNA, and a CRISPR/Cas9 system.

In certain embodiments, the modification comprises administering to the immune effector cell the CRISPR/Cas9 system.

In certain embodiments, the modification further comprises administering to the immune effector cell sgRNA targeting an exon portion of the TRAC gene.

In certain embodiments, the sgRNA targeting the exon portion of the TRAC gene comprises a nucleotide sequence set forth in any one of SEQ ID NO: 186 to SEQ ID NO: 200.

In certain embodiments, the modification comprises administering to the immune effector cell sgRNA targeting an exon portion of the HLA-A gene.

In certain embodiments, the sgRNA targeting the exon portion of the HLA-A gene comprises a nucleotide sequence set forth in any one of SEQ ID NO: 201 to SEQ ID NO: 241.

In certain embodiments, the modification further comprises administering to the cell a Cas enzyme.

In certain embodiments, the Cas enzyme includes a Cas9 protein.

In certain embodiments, the antisense RNA comprises a nucleotide sequence set forth in any one of SEQ ID NO: 242 to SEQ ID NO: 245.

In certain embodiments, the immune effector cell is an HLA-B homozygous cell.

In certain embodiments, the HLA-B homozygote includes HLA-B*40 homozygote, HLA-B* 15 homozygote, HLA-B*46 homozygote, HLA-B* 13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

In certain embodiments, the immune effector cell is an HLA-A homozygous or heterozygous cell.

In certain embodiments, the HLA-A homozygote or heterozygote includes HLA-A*02 homozygote, HLA-A* 11 homozygote, HLA-A* 02/A* 11 heterozygote, or HLA-A*24 homozygote.

In another aspect, the present application provides a method for preparing the aforementioned immune effector cell, which comprises: modifying the immune effector cell before/after introducing a polynucleotide sequence encoding the aforementioned CAR targeting IL 13Rα2 or a vector comprising the polynucleotide sequence encoding the aforementioned CAR targeting IL13Rα2 into the immune effector cell, wherein the modification comprises down-regulation of the expression and/or activity of one or more of immune rejection-related genes.

In certain embodiments, the vector is an expression vector.

In certain embodiments, the vector is selected from a DNA vector, an RNA vector, a plasmid, a lentiviral vector, an adenoviral vector, an adeno-associated viral vector, and a retroviral vector.

In certain embodiments, the immune rejection-related gene is selected from one or more of the following groups: TRAC, TRBC, HLA-A, HLA-B, B2M, and CIITA.

In certain embodiments, the expression and/or activity of the TRAC gene and the HLA-A gene in the immune effector cell is down-regulated as compared to the expression and/or activity of a corresponding gene in a corresponding unmodified cell.

In certain embodiments, the expression and/or activity of the CIITA gene is not down-regulated as compared to the expression and/or activity of the corresponding gene in the corresponding unmodified cell.

In certain embodiments, the expression and/or activity of the B2M gene is not down-regulated as compared to the expression and/or activity of the corresponding gene in the corresponding unmodified cell.

In certain embodiments, the expression and/or activity of the TRAC gene and the HLA-A gene in the immune effector cell is down-regulated as compared to a corresponding wild-type cell.

In certain embodiments, the expression and/or activity of the CIITA gene is not down-regulated as compared to the corresponding wild-type cell.

In certain embodiments, the expression and/or activity of the B2M gene is not down-regulated as compared to the corresponding wild-type cell.

In certain embodiments, the down-regulation of the expression level and/or activity of the gene includes down-regulating the expression and/or activity of a nucleic acid molecule encoding the gene; and/or down-regulating the expression and/or activity of a protein product encoded by the gene.

In certain embodiments, the modification comprises: gene knockout, gene mutation, and/or gene silencing.

In certain embodiments, the modification comprises knocking out either of two TRAC alleles and knocking out either of two HLA-A alleles in the immune effector cell.

In certain embodiments, the modification comprises knocking out the two TRAC alleles and knocking out either of the two HLA-A alleles in the immune cell.

In certain embodiments, the modification comprises knocking out an exon of the TRAC gene and knocking out an exon of the HLA-A gene in the immune cell.

In certain embodiments, the modification comprises administering to the immune effector cell one or more substances selected from the group consisting of: antisense RNA, siRNA, shRNA, and a CRISPR/Cas9 system.

In certain embodiments, the modification comprises administering to the immune effector cell the CRISPR/Cas9 system.

In certain embodiments, the modification comprises administering to the immune effector cell sgRNA targeting an exon portion of the TRAC gene.

In certain embodiments, the sgRNA targeting the exon portion of the TRAC gene comprises a nucleotide sequence set forth in any one of SEQ ID NO: 186 to SEQ ID NO: 200.

In certain embodiments, the modification comprises administering to the immune effector cell sgRNA targeting an exon portion of the HLA-A gene.

In certain embodiments, the sgRNA targeting the exon portion of the HLA-A gene comprises a nucleotide sequence set forth in any one of SEQ ID NO: 201 to SEQ ID NO: 241.

In certain embodiments, the modification further comprises administering to the cell a Cas enzyme.

In certain embodiments, the Cas enzyme includes a Cas9 protein.

In certain embodiments, the antisense RNA comprises a nucleotide sequence set forth in any one of SEQ ID NO: 242 to SEQ ID NO: 245.

In certain embodiments, the immune effector cell includes a human cell.

In certain embodiments, the immune effector cell includes a T cell, a B cell, a natural killer cell (NK cell), a macrophage, an NKT cell, a monocyte, a dendritic cell, a granulocyte, a lymphocyte, a leukocyte, and/or a peripheral blood mononuclear cell.

In certain embodiments, the immune effector cell includes an autologous or non-autologous immune effector cell.

In certain embodiments, the cell is an HLA-B homozygous cell.

In certain embodiments, the HLA-B homozygote includes HLA-B*40 homozygote, HLA-B* 15 homozygote, HLA-B*46 homozygote, HLA-B* 13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

In certain embodiments, the cell is an HLA-A homozygous or heterozygous cell.

In certain embodiments, the HLA-A homozygote or heterozygote includes HLA-A*02 homozygote, HLA-A* 11 homozygote, HLA-A* 02/A* 11 heterozygote, or HLA-A*24 homozygote.

In another aspect, the present application further provides use of the aforementioned immune effector cell in the preparation of a CAR-T cell.

In another aspect, the present application further provides a pharmaceutical composition comprising the aforementioned immune effector cell and optionally a pharmaceutically acceptable carrier.

In another aspect, the present application further provides use of the aforementioned immune effector cell and/or the aforementioned pharmaceutical composition in the treatment of a disease or disorder associated with the expression of IL13Rα2.

In certain embodiments, the disease or disorder associated with the expression of IL13Rα2 includes a disease or disorder associated with up-regulation of the expression of IL13Rα2.

In certain embodiments, the disease or disorder associated with the expression of IL13Rα2 includes cancer.

In certain embodiments, the cancer includes an IL13Rα-positive tumor.

In certain embodiments, the IL13Rα-positive tumor includes malignant gliomas, colorectal cancer, cervical cancer, pancreatic cancer, cutaneous melanoma, ovarian cancer, breast cancer, adrenocortical carcinoma, thymoma, uterine cancer, bladder cancer, esophageal cancer, head and neck squamous cell carcinoma, or gastric cancer.

In another aspect, the present application further provides use of the aforementioned immune effector cell and/or the aforementioned pharmaceutical composition in the preparation of a medicament for treating a disease or disorder associated with the expression of IL13Rα2.

In certain embodiments, the disease or disorder associated with the expression of IL13Rα2 includes a disease or disorder associated with up-regulation of the expression of IL13Rα2.

In certain embodiments, the disease or disorder associated with the expression of IL13Rα2 includes cancer.

In certain embodiments, the cancer includes an IL13Rα-positive tumor.

In certain embodiments, the IL13Rα-positive tumor includes malignant gliomas, colorectal cancer, cervical cancer, pancreatic cancer, cutaneous melanoma, ovarian cancer, breast cancer, adrenocortical carcinoma, thymoma, uterine cancer, bladder cancer, esophageal cancer, head and neck squamous cell carcinoma, or gastric cancer.

In another aspect, the present application further provides a method for preventing or treating a disease or disorder associated with the expression of IL13Rα2, which comprises administering to a subject in need thereof an effective amount of the aforementioned immune effector cell and/or the aforementioned pharmaceutical composition.

In certain embodiments, the disease or disorder associated with the expression of IL13Rα2 includes a disease or disorder associated with up-regulation of the expression of IL13Rα2.

In certain embodiments, the disease or disorder associated with the expression of IL13Rα2 includes cancer.

In certain embodiments, the cancer includes an IL13Rα-positive tumor.

In certain embodiments, the IL13Rα-positive tumor includes malignant gliomas, colorectal cancer, cervical cancer, pancreatic cancer, cutaneous melanoma, ovarian cancer, breast cancer, adrenocortical carcinoma, thymoma, uterine cancer, bladder cancer, esophageal cancer, head and neck squamous cell carcinoma, or gastric cancer.

Other aspects and advantages of the present application will be readily apparent to those skilled in the art from the following detailed description. Only exemplary embodiments of the present application have been shown and described in the following detailed description. As will be recognized by those skilled in the art, the content of the present application enables those skilled in the art to make changes to the specific embodiments disclosed without departing from the spirit and scope of the invention to which the present application pertains. Accordingly, descriptions in the drawings and specification of the present application are only illustrative rather than restrictive.

## BRIEF DESCRIPTION OF THE DRAWINGS

Specific features of the invention to which the present application pertains are set forth in appended claims. Features and advantages of the invention to which the present application pertains will be better understood by reference to the exemplary embodiments and drawings described in detail below. The drawings are briefly described as follows:

FIG. 1 shows a schematic structural diagram of anti-IL13Rα2 CAR described in the present application;

FIG. 2 shows a construction strategy of anti-IL13Rα2 UCAR-T cells described in the present application;

FIGs. 3A-3C show phenotype assay results of the anti-IL13Rα2 UCAR-T cells (murine) described in the present application;

FIG. 4 shows a killing result of the anti-IL13Rα2 UCAR-T cells (murine) described in the present application on target cells;

FIGs. 5A-5C show cytokine secretion assay results of the IL13Rα2 UCAR-T cells (murine) described in the present application co-cultured with the target cells;

FIG. 6 shows an *in vivo* anti-tumor effect of the anti-IL13Rα2 UCAR-T cells (murine) described in the present application;

FIG. 7 shows an *in vivo* half-life assay result of the anti-IL13Rα2 UCAR-T cells described in the present application;

FIG. 8 shows an *in vitro* GVHD result of the anti-IL13Rα2 UCAR-T cells described in the present application;

FIG. 9 shows an *in vitro* rejection response result of the anti-IL13Rα2 UCAR-T cells described in the present application;

FIGs. 10A-10D show *in vivo* GVHD results of the anti-IL13Rα2 UCAR-T cells described in the present application;

FIGs. 11-12 show *in vitro* rejection response results of the anti-IL13Rα2 UCAR-T cells described in the present application;

FIG. 13 shows off target analysis of the anti-IL13Rα2 UCAR-T cells described in the present application;

FIG. 14 shows chromosome translocation analysis of the anti-IL13Rα2 UCAR-T cells described in the present application;

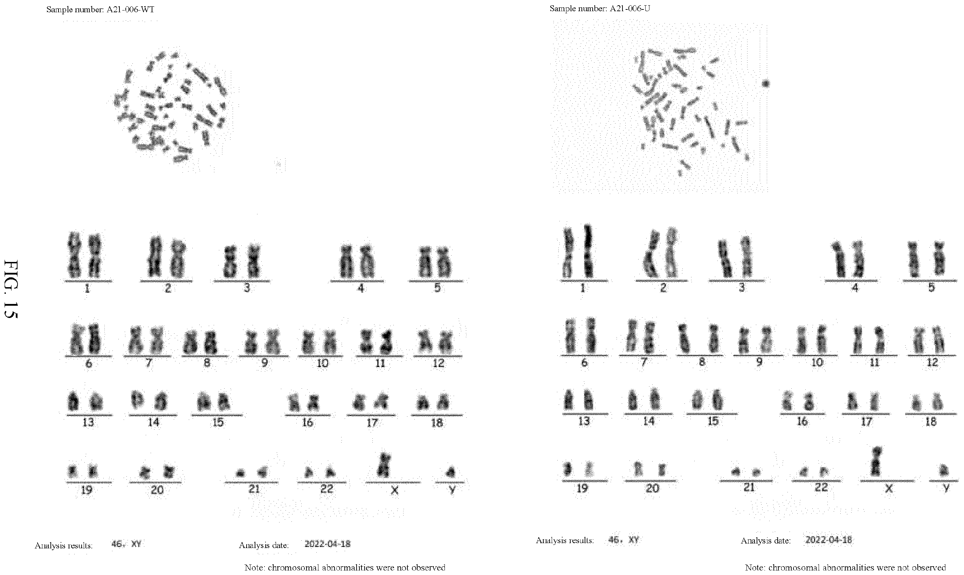

FIG. 15 shows karyotype analysis of the anti-IL13Rα2 UCAR-T cells described in the present application;

FIG. 16 shows residual Cas9 analysis of the anti-IL13Rα2 UCAR-T cells described in the present application;

FIG. 17 shows Sanger sequencing results of TRAC gene after Sg9RNA editing in the present application;

FIG. 18 shows TA cloning assay results of the TRAC gene after Sg9RNA editing in the present application;

FIG. 19 shows flow cytometry assay results of the TRAC gene after Sg9RNA editing in the present application;

FIG. 20 shows the Sanger sequencing results of HLA-A02 gene after Sg2RNA editing in the present application;

FIG. 21 shows the Sanger sequencing results of HLA-A02 gene after Sg5RNA editing in the present application;

FIG. 22 shows the Sanger sequencing results of HLA-A11 gene after Sg21RNA editing in the present application;

FIG. 23 shows the Sanger sequencing results of HLA-A11 gene after Rsg2RNA editing in the present application;

FIGs. 24A-24B show results of simultaneous knockout of HLA-A02 and TRAC in the modified immune effector cells of the present application;

FIGs. 25A-25B show protein levels of HLA-A02 and TRAC in the modified immune effector cells of the present application;

FIG. 26 shows mRNA levels of TRAC, HLA-A, B2M, and CIITA in the modified immune effector cells of the present application;

FIGs. 27A-27B show protein levels of B2M and CIITA in the modified immune effector cells of the present application;

FIGs. 28A-28D show protein levels of TRAC, HLA-A, B2M, and CIITA in the modified immune effector cells of the present application;

FIGs. 29A-29B show the knockout of TRAC and HLA-A at mRNA levels in the modified immune effector cells of the present application;

FIGs. 30A-30B show protein levels of CD69 and CD137 in the modified immune effector cells of the present application;

FIG. 31 shows the co-culture of the modified immune effector cells of the present application and NK cells;

FIG. 32 shows the expression level of IFN-$\gamma$ in the modified immune effector cells of the present application;

FIGs. 33A-33D show protein levels of TRAC, HLA-A, B2M, and CIITA in the modified immune effector cells of the present application;

FIG. 34 shows the infection efficiency of the modified immune effector cells of the present application on CARs;

FIG. 35 shows amplification folds of the modified immune effector cells of the present application;

FIG. 36 shows a killing effect of the modified immune effector cells of the present application on CD19-positive target cells;

FIG. 37 shows a dosing regimen for administering the modified immune effector cells of the present application; and

FIG. 38 shows a killing effect of the modified immune effector cells of the present application on tumors in mice.

## DETAILED DESCRIPTION

The embodiments of the present invention are described below with reference to specific examples, and other advantages and effects of the present invention will be readily apparent to those skilled in the art from the disclosure of the present specification.

### Definitions of Terms

In the present application, the term "chimeric antigen receptor" or "CAR" generally refers to a group of polypeptides, generally two types in the simplest embodiment, which, when in an immune effector cell, provide the cell with specificity for a target cell (generally a cancer cell) and produce an intracellular signal. In some embodiments, the CAR comprises at least one extracellular antigen-binding domain (such as a VHH, a scFv, or a portion thereof), a transmembrane domain, and a cytoplasmic signaling domain (also referred to herein as an "intracellular signaling domain") that comprises a functional signaling domain derived from a stimulatory molecule and/or a co-stimulatory molecule as defined below. In some embodiments, the group of polypeptides are in the same polypeptide chain (e.g., comprising a chimeric fusion protein). In some embodiments, the group of polypeptides are not contiguous with each other, e.g., in different polypeptide chains. In some aspects, the group of polypeptides includes a dimerization switch that can couple the polypeptides to each other in the presence of a dimerization molecule, e.g., can couple an antigen-binding domain to an intracellular signaling domain. In one aspect, the stimulatory molecule of the CAR is a $\zeta$ chain associated with a T cell receptor complex. In one aspect, the cytoplasmic signaling domain comprises a primary signaling domain (e.g., a primary signaling domain of CD3-$\zeta$). In one aspect, the cytoplasmic signaling domain further comprises one or more functional signaling domains derived from at least one co-stimulatory molecule as defined below. In one aspect, the co-stimulatory molecule may be selected from 4-1BB (i.e., CD137), CD27, ICOS, and/or CD28. In one aspect, the CAR comprises a chimeric fusion protein, which may comprise an extracellular antigen recognition domain, a transmembrane domain, and an intracellular signaling domain comprising a functional signaling domain derived from a stimulatory molecule. In one aspect, the CAR comprises a chimeric fusion protein, which may comprise an extracellular antigen recognition domain, a transmembrane domain, and an intracellular signaling domain comprising a functional signaling domain derived from a co-stimulatory molecule and a functional signaling domain derived from a stimulatory molecule. In one aspect, the CAR comprises a chimeric fusion protein, which may comprise an extracellular antigen recognition domain, a transmembrane domain, and an intracellular signaling domain comprising a functional signaling domain derived from one or more co-stimulatory molecules and a functional signaling domain derived from a stimulatory molecule.

In one aspect, the CAR comprises a chimeric fusion protein, which may comprise an extracellular antigen recognition domain, a transmembrane domain, and an intracellular signaling domain comprising at least two functional signaling domains derived from one or more co-stimulatory molecules and a functional signaling domain derived from a stimulatory molecule. In one aspect, the CAR comprises an optional leader sequence at the amino-terminus (N-ter) of the CAR fusion protein. In one aspect, the CAR further comprises a leader sequence at the N-terminus of the extracellular antigen recognition domain, wherein the leader sequence is optionally cleaved from the antigen recognition domain (e.g., VHH) during cell processing and localizes the CAR to the cell membrane.

In the present application, the term "interleukin-13 receptor subunit $\alpha$-2 (IL-13R$\alpha$2)" (also referred to as CD213A2, 213A2 cluster of differentiation) has a common and conventional meaning and can include, but is not limited to, for example, membrane-bound proteins encoded by the IL-13RA2 gene in humans. IL-13R$\alpha$2 is closely associated with the subunit IL-13R$\alpha$1 of the interleukin-13 receptor complex. IL-13R$\alpha$2 generally binds to IL-13 with high affinity, but lacks any prominent cytoplasmic domain, and does not appear to act as a signal mediator. However, IL-13R$\alpha$2 is able to regulate the effects of both IL-13 and IL-4, although it cannot bind directly to the latter. It has been reported that IL-13R$\alpha$2 plays a role in the internalization of IL-13.

In the present application, the term "antibody" generally refers to being used in the broadest sense and specifically encompasses monoclonal antibodies, polyclonal antibodies, dimers, polymers, multispecific antibodies (e.g., bispecific antibodies), and antibody fragments so long as they exhibit the desired biological activity (Miller et al., (2003) Jour. of Immunology 170: 4854-4861). The antibody may be a murine antibody, a human antibody, a humanized antibody, or a chimeric antibody, or derived from other species.

A full-length antibody typically refers to an antibody that consists of two "full-length antibody heavy chains" and two "full-length antibody light chains". The "Full-length antibody heavy chain" generally refers to a polypeptide consisting of, from the N-terminus to the C-terminus, an antibody heavy chain variable domain (VH), an antibody constant heavy chain domain 1 (CH1), an antibody hinge region (HR), an antibody heavy chain constant domain 2 (CH2), and an antibody heavy chain constant domain 3 (CH3), abbreviated as VH-CH1-HR-CH2-CH3; and in the case of antibodies of the IgE subclass, it optionally further comprises an antibody heavy chain constant domain 4 (CH4). In some embodiments, the "full-length antibody heavy chain" is a polypeptide consisting of, from the N-terminus to the C-terminus, VH, CH1, HR, CH2, and CH3. The "full-length antibody light chain" is generally a polypeptide consisting of, from the N-terminus to the C-terminus, an antibody light chain variable domain (VL) and an antibody light chain constant domain (CL), abbreviated as VL-CL. The antibody light chain constant domain (CL) may be $\kappa$(kappa) or $\lambda$(lambda). The two full-length antibody chains are linked together by inter-polypeptide disulfide bonds between the CL domain and the CH1 domain and between the hinge regions of the full-length antibody heavy chains. Examples of typical full-length antibodies are natural antibodies such as IgG (e.g., IgG1 and IgG2), IgM, IgA, IgD, and IgE.

In the present application, the term "antigen-binding fragment" (also referred to herein as a "targeting moiety" or "antigen-binding moiety") generally refers to a portion of an antibody molecule, which comprises amino acids responsible for specific binding between an antibody and an antigen. The portion of the antigen specifically recognized and bound by the antibody is referred to as an "epitope" described above. The antigen-binding domain may typically comprise an antibody light chain variable region (VL) and an antibody heavy chain variable region (VH); however, it does not necessarily comprise both. An Fd fragment, for example, has two VH regions and generally retains some of the antigen-binding functions of the intact antigen-binding domain. Examples of antigen-binding fragments of antibodies include: (1) a Fab fragment, a monovalent fragment having a VL, a VH, a constant light chain (CL) and a CH1 domain; (2) an F(ab')$_2$ fragment, a bivalent fragment having two Fab fragments linked by a disulfide bridge at the hinge region; (3) an Fd fragment, having two VH and CH1 domains; (4) an Fv fragment, having VL and VH domains of a single arm of an antibody; (5) a dAb fragment (Ward et al., "Binding Activities of a Repertoire of Single Immunoglobulin Variable Domains Secreted From Escherichia coli", Nature 341: 544-546 (1989), which is incorporated herein by reference in its entirety), having a VH domain; (6) an isolated complementarity-determining region (CDR); (7) a single-chain Fv (scFv), e.g., derived from an scFV-library. Although the two domains of the Fv fragment, VL and VH, are encoded by separate genes, they may be joined by a recombinant method using a synthetic linker that allows them to be prepared as a single protein chain in which the VL and VH regions pair to form monovalent molecules (referred to as single-chain Fv (scFv)) (see, e.g., Huston et al., "Protein Engineering of Antibody Binding Sites: Recovery of Specific Activity in an Anti-Digoxin Single-Chain Fv Analogue Produced in Escherichia coli", Proc. Natl. Acad. Sci. USA 85: 5879-5883 (1988)); and (8) VHH, which relates to variable antigen-binding domains of heavy chain antibodies from Camelidae (camel, dromedary, llama, alpaca, etc.) (see Nguyen V. K. et al., 2000, The EMBO Journal, 19, 921-930; Muyldermans S., 2001, J Biotechnol., 74, 277-302 and a review of Vanlandschoot P. et al., 2011, Antiviral Research 92, 389-407). VHH may also be referred to as nanobody (Nb) and/or single-domain antibody. These antibody fragments are obtained using conventional techniques known to those skilled in the art, and assessed for the function in the same manner as for intact antibodies. An antigen-binding fragment targeting IL13R$\alpha$2 is also described in International Patent Application Publications WO2014072888A1 and WO2021041725A1, each of which is incorporated herein by reference in its entirety.

In the present application, the term "single-domain antibody" or "VHH" generally refers to a class of antibodies that lack an antibody light chain and have only a heavy chain variable region. In certain cases, the single-domain antibody may be derived from Bactrian camels, dromedaries, alpacas, llamas, nurse sharks, smooth dogfishes or rays (see, e.g., Kang Xiaozhen et al., Chinese Journal of Biotechnology, 2018, 34(12): 1974-1984). For example, the single-domain antibody may be derived from alpacas. The single-domain antibody may consist of a heavy chain variable region (VH). The term "heavy chain variable region" generally refers to the amino-terminal domain of the heavy chain of an antigen-binding fragment. The heavy chain variable region may be further divided into hypervariable regions termed complementarity-determining regions (CDRs), which are scattered over more conserved regions termed framework regions (FRs). Each heavy chain variable region may consist of three CDRs and four FRs arranged from the amino-terminus to the carboxyl-terminus in the following order: FR1, CDR1, FR2, CDR2, FR3, CDR3, and FR4. The heavy chain variable region comprises a binding domain that interacts with an antigen.

In the present application, the term "single-chain variable fragment" or "scFv" has a common and conventional meaning and can include, but is not limited to, for example, a fusion protein comprising the heavy chain variable region (VH) and the light chain variable region (VL) of an immunoglobulin, which are linked to each other with a short linker peptide. Without limitation, the linker may comprise glycine (for flexibility) as well as hydrophilic amino acids (e.g., serine or threonine) (for solubility). The linker may link the N-terminus of the VH to the C-terminus of the VL, or may link the C-terminus of the VH to the N-terminus of the VL. In some alternatives, a ligand binding domain present on the CAR is a single-chain variable fragment (scFv). The CAR of the present application may be configured in a VH-VL or VL-VH configuration with variations in the linker, hinge, transmembrane domain, co-stimulatory domain, and/or signaling domain, and the CAR still retains its efficacy. In some embodiments, the scFv domain present on the CAR is specific for IL-13$\alpha$2 receptor (IL13R$\alpha$2) present on a tumor cell.

The CAR of the present application may comprise linker residues between the various domains added for proper spacing and conformation of a molecule, for example, a linker comprising an amino acid sequence that links the VH domain to the VL domain and provides a spacer function compatible with the interaction of the two sub-binding domains, so that the resulting polypeptide retains specific binding affinity for the same target molecule as an antibody comprising the same light and heavy chain variable regions. The CAR of the present application may comprise one, two, three, four, or five or more linkers. In particular embodiments, the linker has a length of about 1 to about 25 amino acids, about 5 to about 20 amino acids, or about 10 to about 20 amino acids, or any intervening length of amino acids. Illustrative examples of linkers include glycine polymers; glycine-serine polymers; glycine-alanine polymers; alanine-serine polymers; other flexible linkers known in the art, such as a Whitlow linker. The glycine and the glycine-serine polymers are relatively unstructured and therefore may act as neutral tethers between domains of a fusion protein (e.g., the CAR of the present application).

In the present application, the term "complementarity-determining region" (CDR) generally refers to a complementarity-determining region within a variable region of an antigen-binding fragment. In the present application, there are 3 CDRs present in the heavy chain variable region, and the CDRs are designated HCDR1, HCDR2 and HCDR3 for each variable region. The exact boundaries of those CDRs have been defined differently according to different systems. The system described by Kabat (Kabat et al., Sequences of Proteins of Immunological Interest, National Institutes of Health, Bethesda, Md. (1987) and (1991)) provides not only a clear residue numbering system applicable to any variable region of an antigen-binding fragment, but also precise residue boundaries defining 3 CDRs. Those CDRs may be referred to as Kabat CDRs. Chothia and colleagues (Chothia and Lesk, J. Mol. Biol., 196: 901-917 (1987) and Chothia et al., Nature 342: 877-883(1989)) found that although there is large diversity at the amino acid sequence level, certain sub-portions within Kabat CDRs take almost identical peptide backbone conformations. Those sub-portions were designated L1, L2 and L3 or H1, H2 and H3, wherein "L" and "H" refer to the light and heavy chain regions, respectively. Those regions may be referred to as Chothia CDRs, which have boundaries that overlap with Kabat CDRs. Other boundaries defining CDRs that overlap with Kabat CDRs have been described by Padlan (FASEB J. 9: 133-139 (1995)) and MacCallum (J Mol Biol 262 (5): 732-45 (1996)). In addition, other CDR boundary definitions may not strictly follow one of the above systems, but will nevertheless overlap with Kabat CDRs, although they may be shortened or lengthened according to predictions or experimental findings that a particular residue or a particular group of residues, or even the entire CDRs, do not significantly affect the antigen binding. In the present application, the IMGT numbering scheme is used.

In the present application, the term "FR" generally refers to the more highly conserved portions of antibody variable domains, which are referred to as framework regions. For example, the variable domains of natural heavy and light chains may each comprise four FR regions, namely four in VH (H-FR1, H-FR2, H-FR3, and H-FR4), and four in VL (L-FR1, L-FR2, L-FR3, and L-FR4). A "framework region" generally refers to a portion of the antibody variable region recognized in the art that is present between the more divergent (i.e., hypervariable) CDRs. Such framework regions are typically referred to as frameworks 1 to 4 (FR1, FR2, FR3, and FR4) and provide a backbone for presenting six CDRs (three from the heavy chain and three from the light chain) in the three-dimensional space to form an antigen-binding surface.

In the present application, the term "homology" may generally be equivalent to sequence "identity". A homologous sequence may include an amino acid sequence that may be at least 80%, 85%, 90%, 99.1%, 99.2%, 99.3%, 99.4%, 99.5%, 99.6%, 99.7%, 99.8%, or 99.9% identical to a subject sequence. Generally, the homolog will comprise the same active site, etc. as the subject amino acid sequence. Homology may be considered in terms of similarity (i.e., amino acid residues having similar chemical properties/functions), or may be expressed in terms of sequence identity. In the present application, reference to a sequence having a percent identity of any one of the SEQ ID NOs of an amino acid sequence or a nucleotide sequence refers to a sequence having the percent identity over the entire length of the referenced SEQ ID NO.

**[0174]** To determine sequence identity, sequence alignments can be performed by various means known to those skilled in the art, e.g., using BLAST, BLAST-2, ALIGN, NEEDLE, or Megalign (DNASTAR) software, etc. Those skilled in the art can determine appropriate parameters for alignment, including any algorithms required to achieve optimal alignment over the full length of the sequences being compared.

In the present application, the term "$K_D$" is used interchangeably with "KD" and generally refers to a dissociation equilibrium constant, in M (mol/L), of a particular antibody-antigen interaction. $K_D$ can be calculated from the concentration of substance AB and the concentration of substance A and substance B resulting from its dissociation: $K_D$ = c(A) × c(B)/c(AB). It can be seen from this equation that a larger $K_D$ indicates more dissociation and weaker affinity between substances A and B; and conversely, a smaller $K_D$ indicates less dissociation and stronger affinity between substances A and B.

In the present application, the term "isolated nucleic acid molecule" generally refers to an isolated form of nucleotides, deoxyribonucleotides or ribonucleotides or analogs thereof of any length, isolated from their natural environment, or artificially synthesized.

In the present application, the term "vector" generally refers to a nucleic acid molecule capable of self-replicating in a suitable host, which transfers an inserted nucleic acid molecule into a host cell and/or between host cells. The vector may include vectors primarily for the insertion of DNA or RNA into a cell, vectors primarily for the replication of DNA or RNA, and vectors primarily for the expression of transcription and/or translation of DNA or RNA. The vector further includes vectors having a variety of the above-described functions. The vector may be a polynucleotide capable of being transcribed and translated into a polypeptide when introduced into a suitable host cell. Generally, the vector can produce the desired expression product by culturing an appropriate host cell containing the vector.

In the present application, the term "viral vector" is used broadly to refer to a nucleic acid molecule (e.g., transfer plasmid) or viral particle that mediates the transfer of nucleic acids. The nucleic acid molecule includes virus-derived nucleic acid elements that generally facilitate the transfer or integration of the nucleic acid molecules into the genome of a cell. The viral particle generally includes various viral components and sometimes further includes host cell components in addition to nucleic acids. The viral vector may refer to a virus or viral particle capable of transferring nucleic acids into a cell, or the transferred nucleic acid itself.

In the present application, the term "lentivirus" generally refers to a group (or genus) of complex retroviruses. Exemplary lentiviruses include, but are not limited to: human immunodeficiency virus (HIV; including HIV type 1 and HIV type 2); visna-maedivirus (VMV); caprine arthritis-encephalitis virus (CAEV); equine infectious anemia virus (EIAV); feline immunodeficiency virus (FIV); bovine immunodeficiency virus (BIV); and simian immunodeficiency virus (SIV). In one embodiment, an HIV-based vector backbone (i.e., HIV cis-acting sequence element) is preferred. In particular embodiments, the lentivirus is used to deliver a polynucleotide comprising the CAR to a cell.

In the present application, the term "host cell" or "cell" generally refers to an individual cell, cell line, or cell culture that may contain or has contained a vector comprising the isolated nucleic acid molecule described in the present application, or that is capable of expressing the isolated antigen-binding fragment described in the present application. The host cell may comprise progeny of a single host cell. Due to natural, accidental or deliberate mutations, progeny cells may not necessarily be identical in morphology or in genome to the original parent cell, but are capable of expressing the isolated antigen-binding fragment described herein. The host cell may be obtained by transfecting cells with the vector described herein *in vitro.* The host cell may be a prokaryotic cell (e.g., *E. coli*) or a eukaryotic cell (e.g., a yeast cell, a COS cell, a Chinese hamster ovary (CHO) cell, a HeLa cell, an HEK293 cell, a COS-1 cell, an NS0 cell, or a myeloma cell). For example, the host cell may be an *E. coli* cell. For example, the host cell may be a yeast cell. For example, the host cell may be a mammalian cell. For example, the mammalian cell may be a CHO-K1 cell.

In the present application, the term "T cell" or "T lymphocyte" may be any T cell, such as a cultured T cell, e.g., a primary T cell, or a T cell from the cultured T cell line, e.g., Jurkat, and SupTI, or a T cell obtained from a mammal (preferably a primate, species including monkeys, dogs, or humans). If obtained from a mammal, the T cells may be obtained from a number of sources including, but not limited to, blood, bone marrow, lymph nodes, thymus, or other tissues or fluids. The T cell may also be enriched or purified. The T cell may be obtained by maturing a hematopoietic stem cell into a T cell *in vitro* or *in vivo.* In exemplary aspects, the T cell is a human T cell. In exemplary aspects, the T cell is a T cell isolated from a human. The T cell may be any type of T cell, including NKT cells, and may have any developmental stage, including but not limited to CD4+/CD8+ double positive T cells; CDA+ helper T cells; e.g., Th1 and Th2 cells, CD8+ T cells (e.g., cytotoxic T cells); peripheral blood mononuclear cells (PBMCs); peripheral blood leukocytes (PBLs); tumor infiltrating cells (TILs); memory T cells; untreated T cells, and the like. Preferably, the T cell is a CD8+ T cell or a CD4+ T cell. In some alternatives, the T cell is allogeneic (from different donors of the same species) to the recipient subject that receives the cell or cell to be received (e.g., the cells are in the form of a therapeutic composition); in some alternatives, the T cell is autologous (the donor and recipient are the same); in some alternatives, the T cell is syngeneic (the donor and recipient are different, but are homozygotic twins).

In the present application, the term "immune effector cell" generally refers to an immune cell involved in an immune response and performing an effector function. For example, the performing an effector function may include clearing foreign antigens, promoting an immune effector response, or the like. The immune effector cell may include plasma cells, T cells, B cells, natural killer (NK) cells, natural killer T (NKT) cells, mast cells, and myeloid-derived phagocytes.

The immune effector cell of the present application may be autologous/autogeneic ("self") or non-autologous ("non-self", e.g., allogeneic, syngeneic, or xenogeneic). In the present application, the term "autologous" generally refers to cells from the same subject. "Allogeneic" generally means that cells are of the same species as but genetically different from the cells to which they are compared. "Syngeneic" generally means that cells are from different subjects but genetically identical to the cells to which they are compared. "Xenogeneic" generally means that cells are of different species from the cell to which they are compared. In some embodiments, the cells of the present application are autologous or allogeneic.

In the present application, the term "modification" generally refers to changing the state or structure of the cell and/or a change in the state or structure of the cell. The change is generally compared to the state or structure of the corresponding unmodified cell. The change may include a change in endogenous gene expression level or function, for example, a down-regulation, up-regulation, or non-expression of the endogenous gene expression level of the cell by genetic engineering means, which may include homologous recombination, CRISPR/Cas9 system gene editing, or the like; the change may also include a change in cellular protein expression, structure, or function, for example, a change in the expression, structure, or function of the corresponding protein achieved by the change in the expression level or function of the endogenous gene, such as a change in the expression, structure or function of a protein achieved by the regulation of protein translation and post-translational modification; the change may also include the introduction of foreign genes, the expression of foreign proteins, and the like.

In the present application, the term "TRAC" generally refers to a T cell receptor alpha constant. A T cell receptor (TCR) generally refers to a specific receptor located on the surface of the T cell, which is capable of recognizing antigens that bind to major histocompatibility complex (MHC) molecules. TCRs are generally composed of two different protein chains (i.e., heterodimers). In humans, TCRs in most T cells are composed of one α chain and one β chain (encoded by TRA and TRB, respectively), and this class of T cells is referred to as αβ T cells; and in a few T cells, TCRs are composed of γ chain and δ chain (encoded by TRG and TRD, respectively), and this class of T cells is referred to as γδ T cells. Generally, αβ T cells account for about 95% of the total T cells, γδ T cells account for about 5% of the total T cells, and the ratios vary during ontogenesis and in diseased states (e.g., leukemia), and also differ among species. Each chain constituting TCRs comprises a variable region and a constant region. In humans, the gene encoding α chain (TRA, e.g., information as shown by HGNC: 12027) is located on chromosome 14 and consists of multiple gene fragments, including a variable fragment (V), a joining fragment (J), and a constant fragment (C). The TRAC gene generally refers to a gene sequence encoding the T cell receptor α chain constant region (C) (e.g., information as shown by HGNC: 12029) and is located on chromosome 14 (14q11.2; 14:22,547,505-22,552,131). Generally, one of the genes of the variable fragments (V) encoding the N-fragment of the antigen recognition domain is rearranged with one of the joining fragments (1) to produce a functional V-region exon, which is transcribed and linked to the constant region (C) by splicing, thereby forming a coding sequence of the T cell receptor α chain.

In the present application, the term "major histocompatibility complex antigen" ("MHC", also referred to as "human leukocyte antigen" ("HLA") in humans) generally refers to a protein expressed on the surface of a cell that confers a unique antigenic identity to the cell. MHC/HLA antigens are target molecules that are recognized by T cells and NK cells as being derived from the same source of hematopoietic stem cells as immune effector cells ("self') or as being derived from another source of hematopoietic repopulating cells ("non-self'). Two major classes of HLA antigens are recognized: HLA class I and HLA class II. HLA class I antigens (A, B, and C in humans) allow each cell to be recognized as "self", while HLA class II antigens (DR, DP, and DQ in humans) are involved in reactions between lymphocytes and antigen-presenting cells. Both have been implicated in the rejection of transplanted organs. An important aspect of the HLA gene system is its polymorphism. Each gene for MHC class I (A, B, and C) and MHC class II (DP, DQ, and DR) exists in different alleles. HLA alleles are designated by numbers and subscripts. For example, two unrelated individuals may carry class I HLA-B genes B5 and Bw41, respectively. Allelic products differ in one or more amino acids of the α and/or β domains. A number of specific antibodies or nucleic acid reagents are used to type HLA haplotypes of individuals using leukocytes that express class I and class II molecules. Genes commonly used for HLA typing are six MHC class I and II proteins, i.e., two alleles for each of HLA-A, HLA-B, and HLA-DR. The HLA genes are clustered in a "super locus" present on chromosome position 6p21, wherein the "super locus" encodes 6 classical transplantation HLA genes and at least 132 protein-coding genes that play important roles in the regulation of the immune system as well as some other fundamental molecular and cellular processes. The complete locus measures roughly 3.6 Mb with at least 224 loci. One effect of such clustering is that a "haplotype", i.e., a group of alleles present on a single chromosome, is inherited from one parent and tends to be inherited as a group. The group of alleles inherited from each parent form a haplotype, in which some alleles tend to be associated together. Identifying haplotypes of a patient may help predict the probability of finding a matching donor and help formulate a search strategy, because some alleles and haplotypes are more common than others and they are distributed at different frequencies in different racial and ethnic groups.

In the present application, "HLA-A" generally refers to a class of human leukocyte antigen polypeptide chains encoded by an HLA-A gene located on human chromosome 6p21.3 (e.g., information as shown by HGNC:4931). HLA-A is one of the three major polypeptide types that constitute MHC class I molecules on the surface of human cells, and others further include HLA-B and HLA-C. A heterodimer composed of an α chain encoded by the HLA-A gene and a β chain encoded by a B2M gene (β2-microglobulin) is an HLA-A class MHC I molecule. The α chain encoded by the HLA-A gene may comprise an α1 domain, an α2 domain, an α3 domain, a transmembrane region, and a cytoplasmic region, wherein the α1 domain and the α2 domain may bind to a peptide fragment so as to present the peptide fragment to an immune cell by the MHC I molecule (e.g., HLA-A class). In humans, similar to most mammals, the α chain of the MHC I molecule is polymorphic, and there are many variations in the primary structure thereof. As of December 2013, there are 2432 known HLA-A alleles in total, which encode 1740 active proteins and 117 inactive proteins. In the present application, HLA-A alleles may include sequence information on different HLA-A alleles recorded in the IMGT/HLA database version 3.38.0 (https://www.ebi.ac.uk/ipd/imgt/hla/) and designated by the WHO HLA Factor Nomenclature Committee.

In the present application, the term "HLA-B" generally refers to a part of the gene family of human leukocyte antigen (HLA) complexes. HLA is a human version of the major histocompatibility complex (MHC), and MHC is a gene family present in many species. The complex genes are divided into three basic groups: class I, class II, and class III. In humans, the HLA-B gene and the two related genes HLA-A and HLA-C are the major genes of MHC class I. The HLA-B gene is located in the cell band 21.3 of the short (p) arm of chromosome 6 from base pairs 31,353,871 to 31,357,211. HLA-B is one of the three major HLAs that should be matched between the donor and recipient. They are HLA-A, HLA-B (both are MHC class I), and HLA-DR (MHC class II). If two tissues have the same genes encoding the three HLAs, the possibility and severity of rejection are minimized. Hundreds of versions (alleles) of HLA-B are known, each version having a specific number (e.g., HLA-B27). Closely related alleles are grouped together, for example, at least 28 very similar alleles are subtypes of HLA-B27. These subtypes are designated as HLA-B*2701 to HLA-B*2728.

In the present application, the term "HLA-matched" refers to a donor-recipient pair in which none of the HLA antigens are mismatched between the donor and recipient, such as a donor providing a hematopoietic stem cell graft to a recipient in need of hematopoietic stem cell transplantation therapy. HLA-matched (i.e., in which all 6 alleles are matched) donor-recipient pairs have a reduced risk of graft rejection, because endogenous T cells and NK cells are less likely to recognize the incoming graft as foreign, and are thus less likely to generate an immune response against the graft.

In the present application, the term "HLA-mismatched" refers to a donor-recipient pair in which at least one HLA antigen (particularly with respect to HLA-A, HLA-B, and HLA-DR) is mismatched between the donor and recipient, such as a donor providing a hematopoietic stem cell graft to a recipient in need of hematopoietic stem cell transplantation therapy. In some embodiments, one haplotype is matched, and the other is mismatched. HLA-mismatched donor-recipient pairs may have an increased risk of graft rejection relative to HLA-matched donor-recipient pairs, because endogenous T cells and NK cells are more likely to recognize the incoming graft as foreign in the case of HLA-mismatched donor-recipient pairs, and such T cells and NK cells are thus more likely to generate an immune response against the graft.

In the present application, the term "B2M" generally refers to β2-microglobulin, which is one of the components of MHC class I molecules. β2 micro globulin (also referred to as β chain) may form an MHC class I molecule with an α chain encoded by HLA. B2M is generally expressed in all nucleated cells. In humans, β2 microglobulin is encoded by the B2M gene located at 15q21.1 (e.g., information as shown by HGNC: 914).

In the present application, the term "CIITA" generally refers to a transactivator of a class II major histocompatibility complex (MHC II). The transactivator may be a protein having an acidic transcriptional activation domain, 4 LRRs (leucine-rich repeats), and a GTP binding domain. The protein may be located in the cell nucleus, act as a positive regulator of the gene transcription of the class II major histocompatibility complex (MHC II), and be referred to as a "master control factor" for the expression of these genes. The protein may also bind to GTP and utilize the binding to GTP to transport itself into the cell nucleus, where it generally functions by acetyltransferase (AT) activity in a coactivator-like manner. In humans, the protein is encoded by a gene located at 16p13.13 (e.g., information as shown by HGNC:7067), and several transcript variants encoding different isoforms can be produced.

In the present application, the term "wild-type cell" generally refers to a cell that naturally occurs or is of natural origin.

In the present application, the term "nucleic acid" or "polynucleotide" or "nucleic acid molecule" generally refers to deoxyribonucleic acid (DNA) or ribonucleic acid (RNA) and polymers thereof in either single-stranded or double-stranded form. Unless specifically limited, the term may include nucleic acids comprising analogs of natural nucleotides that have similar binding properties as the reference nucleic acid (e.g., with sequence information shown) and are metabolized in a manner similar to naturally occurring nucleotides. Unless otherwise indicated, the sequence of a nucleic acid may include conservatively modified variants thereof (e.g., degenerate codon substitutions), alleles, orthologs, SNPs, and complementary sequences, as well as the sequences explicitly indicated.

In the present application, the term "expression" generally refers to the transcription and/or translation of a particular nucleotide sequence.

In the present application, the term "gene mutation" generally refers to a change in the composition or arrangement order of base pairs occurring in the structure of genes, such as a point mutation caused by a single base change, or deletion, duplication, insertion, and the like of a plurality of bases.

In the present application, the term "gene silencing" generally refers to the prevention of the expression of certain genes by regulatory mechanisms. The gene silencing may primarily include two types: one is transcriptional gene silencing (TGS) caused by factors such as DNA methylation, heterochromatization, and position effect at the transcriptional level, and the other is post-transcriptional gene silencing (PTGS), which is the effect on gene expression at the post-transcriptional level by specific intervention in the target RNA. Generally, when a gene is silenced, the expression of the corresponding gene is down-regulated/reduced. When a gene is knocked out, it is generally not expressed. For example, the expression of a specific gene in a cell disappears when all alleles of the specific gene are knocked out. Gene silencing is generally considered to be a gene knockdown mechanism, and methods commonly used to silence genes may be, for example, RNAi and the like.

In the present application, the term "endogenous" refers to any substance derived from or produced within an organism, a cell, a tissue, or a system.

In the present application, the term "exogenous" refers to any substance introduced from or produced outside of an organism, a cell, a tissue, or a system.

In the present application, the term "antisense RNA" generally refers to a single-stranded RNA complementary to a transcript mRNA (messenger RNA). The antisense RNA may inhibit the expression of genes by binding to mRNA. For example, the binding of the antisense RNA to the target mRNA results in an increased sensitivity of the double-stranded RNA molecule to RNA enzyme III, and causes the degradation of the double-stranded RNA molecule. For example, the antisense RNA binds to an upstream non-coding region of mRNA, thereby directly inhibiting the translation of the target mRNA.

In the present application, the term "siRNA" generally refers to the abbreviation of small interfering RNA or short interfering RNA. siRNA is a class of double-stranded, non-coding RNA molecules that are about 18-28 base pairs in length and may cause the degradation of mRNA by the complementary binding to mRNA, thereby interfering with the expression of a specific gene. In certain embodiments, siRNA may be a product obtained by treating a long double-stranded RNA or shRNA with Dicer enzyme. In certain embodiments, siRNA enters a cell to form an RNA-induced silencing complex (RISC) with other proteins. The sense strand is degraded, and the antisense strand may bind to a complementary targeting sequence, thereby achieving gene silencing.

In the present application, the term "shRNA" generally refers to the abbreviation of short hairpin RNA, i.e., "short hairpin RNA". shRNA generally comprises two short inverted repeats separated by a stem-loop sequence to form a hairpin structure. Generally, shRNA may further comprise 5-6 T bases as transcription terminators for RNA polymerase III. In certain embodiments, shRNA may enter a cell via a viral vector or plasmid, and be transcribed under the action of polymerase II or polymerase III. The transcripts are exported from the cell nucleus (generally via Exportin 5), and then transported to RISC after Dicer treatment. The sense strand is degraded, and the antisense strand may bind to a complementary targeting sequence, thereby achieving gene silencing.

In the present application, the term "CRISPR/Cas system" generally refers to a group of molecules comprising an RNA-guided nuclease or other effector molecules and a gRNA molecule, the molecules are capable of directing and realizing modification of a nucleic acid by the RNA-guided nuclease or other effector molecules at a target sequence, e.g., causing degradation of the target sequence. In certain embodiments, the CRISPR system comprises gRNA and a Cas protein, e.g., Cas9 protein. A system comprising Cas9 or a functional mutant thereof is referred to herein as "Cas9 system" or "CRISPR/Cas9 system". In certain embodiments, the gRNA molecule and Cas molecule may be complexed to form a ribonucleoprotein (RNP) complex.

In the present application, the terms "gRNA molecule", "guide RNA", "instruction RNA", "direct RNA", "guide RNA module", and "gRNA" can be used interchangeably and generally refer to a nucleic acid molecule capable of facilitating the specific guidance of the RNA-guided nuclease or other effector molecules (generally complexed with a gRNA molecule) onto the target sequence. In certain embodiments, the guidance is achieved by the hybridization of a portion of gRNA with DNA (e.g., via a gRNA guide domain) and by the binding of a portion of the gRNA molecule to the RNA-guided nuclease or other effector molecule (e.g., at least via gRNAtracr). In certain embodiments, the gRNA molecule consists of a single, contiguous polynucleotide molecule, referred to herein as a "single guide RNA", "sgRNA", or the like. In other embodiments, the gRNA molecule consists of multiple (e.g., two) polynucleotide molecules that are capable of association by themselves (typically by hybridization), referred to herein as a "dual guide RNA", "dgRNA", or the like.

In the present application, the term "Cas protein" generally refers to an enzyme responsible for cleaving DNA in the CRISPR/Cas system. The enzyme may include enzymes from types I, II, and III CRISPR/Cas systems, e.g., Cas3, Cas9, and Cas10.

In the present application, the term "Cas9 protein" generally refers to an enzyme responsible for cleaving DNA, which is from the bacterial type II CRISPR/Cas system. Cas9 may include wild-type proteins and functional mutants thereof.

In the present application, the term "allele" generally refers to a form of a gene sequence at a locus that may have different variations. The locus is also referred to as a gene site or site and refers to a fixed position on a chromosome, e.g., where a gene is located. The arrangement of a locus in the genome is referred to as a genetic map.

In the present application, the term "homozygote" generally refers to a genotype individual in which two alleles of homologous chromosomes are identical at the same locus. A pair of opposing genes may have individuals with two genotypes, AA and aa.

In the present application, the term "heterozygote" generally refers to a genotype individual in which two alleles at the same site on homologous chromosomes in a diploid are not identical, such as Aa. Heterozygous genotypes are generally more adaptive than homozygous dominant or homozygous recessive genotypes, and such phenomenon is referred to as heterozygote advantage.

In the present application, the terms "tumor" and "cancer" are used interchangeably and generally refer to a disease characterized by rapid and uncontrolled growth of abnormal cells. Cancer cells can spread to other parts of the body locally or through the bloodstream and lymphatic system. Examples of various cancers are described herein and include, but are not limited to, breast cancer, prostate cancer, ovarian cancer, cervical cancer, skin cancer, pancreatic cancer, colorectal cancer, kidney cancer, liver cancer, brain cancer, lymphoma, leukemia, lung cancer, and the like. The term "cancer" or "tumor" includes premalignant and malignant cancers and tumors, and also encompasses solid tumors and non-solid tumors.

Interleukin-13 receptor $\alpha$2 (IL-13R$\alpha$2) is found at high levels in many cancer cells, including pancreatic cancer, breast cancer, and ovarian cancer, or malignant gliomas such as glioblastoma. IL-13R$\alpha$2 may be also overexpressed in most of human patients with high-grade astrocytomas (see PLoS One. 2013 Oct 16; 8 (10): e77719; expressly incorporated herein by reference in its entirety). Furthermore, reducing the amount of IL13RA2 expression in cancer cells can significantly slow down tumor growth in models (Breast Cancer Research, 2015, 17 (1); expressly incorporated herein by reference in its entirety). Few types of normal tissues are expected to express IL13R$\alpha$2, and in such cases, IL13R$\alpha$2 is only expressed at a low level. In the case of glioblastoma multiforme (GBM), high expression of IL13R$\alpha$2 may be a prognostic marker of tumor progression and poor survival of a patient.

In the present application, the term "pharmaceutically acceptable" generally refers to those compounds, materials, compositions, and/or dosage forms which are commensurate with a reasonable benefit/risk ratio and suitable, within the scope of sound medical judgment, for use in contact with the tissues of human beings and animals without excessive toxicity, irritation, allergic response, or other problems or complications.

In the present application, the term "pharmaceutically acceptable carrier" generally refers to any of those carriers conventionally used and is limited only by physical or chemical factors (such as solubility and lack of reactivity with active binding agents) and by the route of administration. The pharmaceutically acceptable carrier, such as a vehicle, an adjuvant, an excipient, and a diluent, described herein is well known to those skilled in the art and readily available to the public. In one aspect, the pharmaceutically acceptable carrier is one that is chemically inert to an active ingredient of a pharmaceutical composition and one that does not have adverse side effects or toxicity under the conditions of use. In some embodiments, the carrier does not produce an adverse, allergic, or other untoward reaction when administered to an animal or human. In some aspects, the pharmaceutical composition does not comprise pyrogens and other impurities that may be harmful to humans or animals. The pharmaceutically acceptable carrier includes any and all solvents, dispersion media, coatings, antibacterial and antifungal agents, isotonic and absorption delaying agents, and the like; the use of the pharmaceutically acceptable carrier is well known in the art.

The acceptable carriers, excipients, or stabilizers are non-toxic to recipients and are preferably inert at the doses and concentrations employed, and include buffers, such as phosphate, citrate, or other organic acids; antioxidants, such as ascorbic acid; low molecular weight polypeptides; proteins, such as serum albumin, gelatin, or immunoglobulin; hydrophilic polymers, such as polyvinylpyrrolidone; amino acids, such as glycine, glutamine, asparagine, arginine, or lysine; monosaccharides, disaccharides, and other carbohydrates including glucose, mannose, or dextrin; chelating agents, such as EDTA; sugar alcohols, such as mannitol or sorbitol; salt-forming counter-ions, such as sodium; and/or non-ionic surfactants, such as Tween, Pluronics, or polyethylene glycol (PEG).

In the present application, the term "effective amount" or "effective dose" generally refers to an amount sufficient to achieve, or at least partially achieve, a desired effect. "Therapeutically effective amount" or "therapeutically effective dose" of a drug or therapeutic agent is generally any amount of drug that promotes the regression of a disease (as evidenced by a decrease in the severity of symptoms of the disease, an increase in the frequency and duration of the asymptomatic phase of the disease, or the prevention of damage or disability due to the development of the disease) when used alone or in combination with another therapeutic agent.

"Therapeutically effective amount" or "effective amount" of an anti-IL-13R$\alpha$2 CAR-T cell is also an amount or dose that has a therapeutically beneficial effect over any toxic or deleterious effects, such as CRS, of the anti-IL-13R$\alpha$2 CAR-T cell. The term "therapeutically effective amount" includes an amount effective to "treat" a subject (e.g., a patient). In one embodiment, the therapeutically effective dose is the minimum effective dose (MED) of the anti-IL-13R$\alpha$2 CAR-T cell for treating multiple myeloma in the subject. In one embodiment, the therapeutically effective dose is the maximum tolerated dose (MTD) of the anti-IL-13R$\alpha$2 CAR-T cell that does not cause the subject to have unresolved CRS.

In the present application, the term "up-regulation of the expression" generally refers to an increase in the expression of a nucleic acid at the mRNA level or an increase in the expression of the nucleic acid at the polypeptide level. The term may also relate to post-translational modifications required for increased polypeptide activity and/or functions, e.g., addition of sugar moieties, phosphorylation, and the like.

In the present application, the term "comprise" or "comprising" generally means including, summarizing, containing or encompassing. In certain cases, the term also means "being" or "consisting of... ".

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

In the present application, the term "subject" generally refers to a human or non-human animal, including but not limited to cats, dogs, horses, pigs, cows, sheep, rabbits, mice, rats, monkeys, and the like.

## Detailed Description of the Invention

Some embodiments of the method and the composition provided herein relate to chimeric antigen receptors (CARs) capable of specifically binding to interleukin-13 receptor subunit $\alpha$-2 (IL-13R$\alpha$2) or configured to specifically bind to interleukin-13 receptor subunit $\alpha$-2 (IL-13R$\alpha$2). Some embodiments include nucleic acids encoding such CARs, immune effector cells comprising such CARs, and treatment methods that utilize the immune effector cells having such CARs, e.g., by providing these cells for a subject in need thereof as a medicament, for example, for treating or inhibiting diseases or disorders mediated by interleukin-13 receptor subunit $\alpha$-2 (IL-13R$\alpha$2), such as cancers (e.g., IL-13Ra2-positive malignant tumors, including glioma and glioblastoma).

## Immune effector cell

In one aspect, the present application provides an immune effector cell, wherein the functions of a T cell antigen receptor (TCR) and major histocompatibility complexes (MHCI, MHCII) in the immune effector cell are inhibited in a cell, and the immune effector cell comprises a chimeric antigen receptor (CAR) targeting IL13R$\alpha$2.

In certain embodiments, the chimeric antigen receptor (CAR) targeting IL13R$\alpha$2 comprises a targeting moiety comprising at least one complementarity-determining region (CDR) of an antibody heavy chain variable region (VH), wherein the VH comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 18.

In certain embodiments, the VH comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 16 or SEQ ID NO: 17. For example, the VH may comprise an amino acid sequence set forth in SEQ ID NO: 16 or SEQ ID NO: 17.

In certain embodiments, the chimeric antigen receptor (CAR) targeting IL13R$\alpha$2 comprises a targeting moiety comprising a VH, wherein the VH comprises a heavy chain complementarity-determining region 1 (HCDR1), a heavy chain complementarity-determining region 2 (HCDR2), and a heavy chain complementarity-determining region 3 (HCDR3), and the HCDR3 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 3. For example, the HCDR3 may comprise an amino acid sequence set forth in SEQ ID NO: 3.

In certain embodiments, the HCDR2 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 2. For example, the HCDR2 may comprise an amino acid sequence set forth in SEQ ID NO: 2.

In certain embodiments, the HCDR1 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 1. For example, the HCDR1 may comprise an amino acid sequence set forth in SEQ ID NO: 1.

In certain embodiments, the VH comprises: the HCDR1 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 1, the HCDR2 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 2, and the HCDR3 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 3.

For example, the functions of a T cell antigen receptor (TCR) and major histocompatibility complexes (MHCI, MHCII) in the immune effector cell are inhibited in a cell, and the immune effector cell comprises a chimeric antigen receptor (CAR) targeting IL13Rα2. The chimeric antigen receptor (CAR) targeting IL13Rα2 comprises a targeting moiety comprising an antibody heavy chain variable region (VH), and the VH may comprise: the HCDR1 comprising the amino acid sequence set forth in SEQ ID NO: 1, the HCDR2 comprising the amino acid sequence set forth in SEQ ID NO: 2, and the HCDR3 comprising the amino acid sequence set forth in SEQ ID NO: 3.

In certain embodiments, the VH comprises a heavy chain framework region 1 (HFR1), a heavy chain framework region 2 (HFR2), a heavy chain framework region 3 (HFR3), and a heavy chain framework region 4 (HFR4), wherein the HFR1 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 4.

In certain embodiments, the HFR1 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 8 or SEQ ID NO: 12.

In certain embodiments, the HFR2 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 5.

In certain embodiments, the HFR2 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 9 or SEQ ID NO: 13.

In certain embodiments, the HFR3 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 6.

In certain embodiments, the HFR3 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 10 or SEQ ID NO: 14.

In certain embodiments, the HFR4 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 7.

In certain embodiments, the HFR4 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 11 or SEQ ID NO: 15.

In certain embodiments, the VH comprises HFR1, HFR2, HFR3, and HFR4, and the HFR1, HFR2, HFR3, and HFR4 are selected from

i) the HFR1 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 8, the HFR2 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 9, the HFR3 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 10, and the HFR4 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 11; and

ii) the HFR1 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 12, the HFR2 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 13, the HFR3 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 14, and the HFR4 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 15.

In certain embodiments, the VH comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 18.

For example, the functions of the T cell antigen receptor (TCR) and the major histocompatibility complexes (MHCI, MHCII) in the immune effector cell are inhibited in the cell, and the immune effector cell comprises a chimeric antigen receptor (CAR) targeting IL13Rα2. The chimeric antigen receptor (CAR) targeting IL13Rα2 comprises a targeting moiety comprising an antibody heavy chain variable region (VH), and the VH may comprise the amino acid sequence set forth in SEQ ID NO: 18.

In certain embodiments, the VH comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 16 or SEQ ID NO: 17.

In certain embodiments, the targeting moiety further comprises at least one CDR of an antibody light chain variable region (VL), wherein the VL comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 36.

In certain embodiments, the VL comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 34 or SEQ ID NO: 35.

In certain embodiments, the targeting moiety comprises at least one CDR of the VH and at least one CDR of the VL, and the VH and the VL are selected from:

i) a VH comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 16, and a VL comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 34; and

ii) a VH comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 17, and a VL comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 35.

In certain embodiments, the chimeric antigen receptor (CAR) targeting IL13Rα2 comprises a targeting moiety comprising a VL, wherein the VL comprises a light chain complementarity-determining region 1 (LCDR1), a light chain complementarity-determining region 2 (LCDR2), and a light chain complementarity-determining region 3 (LCDR3), and the LCDR1 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 19. For example, the LCDR1 may comprise an amino acid sequence set forth in SEQ ID NO: 19.

In certain embodiments, the LCDR2 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 20. For example, the LCDR2 may comprise an amino acid sequence set forth in SEQ ID NO: 20.

In certain embodiments, the LCDR3 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 21. For example, the LCDR3 may comprise an amino acid sequence set forth in SEQ ID NO: 21.

In certain embodiments, the VL comprises the LCDR1 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 19, the LCDR2 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 20, and the LCDR3 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 21.

For example, the functions of the T cell antigen receptor (TCR) and the major histocompatibility complexes (MHCI, MHCII) in the immune effector cell are inhibited in the cell, and the immune effector cell comprises a chimeric antigen receptor (CAR) targeting IL13Rα2. The chimeric antigen receptor (CAR) targeting IL13Rα2 comprises a targeting moiety comprising a VH and a VL, wherein the VH may comprise: the HCDR1 comprising the amino acid sequence set forth in SEQ ID NO: 1, the HCDR2 comprising the amino acid sequence set forth in SEQ ID NO: 2, and the HCDR3 comprising the amino acid sequence set forth in SEQ ID NO: 3; and the VL may comprise the LCDR1 comprising the amino acid sequence set forth in SEQ ID NO: 19, the LCDR2 comprising the amino acid sequence set forth in SEQ ID NO: 20, and the LCDR3 comprising the amino acid sequence set forth in SEQ ID NO: 21.

In certain embodiments, the VL comprises a light chain framework region 1 (LFR1), a light chain framework region 2 (LFR2), a light chain framework region 3 (LFR3), and a light chain framework region 4 (LFR4), and the LFR1 comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 22.

In certain embodiments, the LFR1 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 26 or SEQ ID NO: 30.

In certain embodiments, the LFR2 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 23.

In certain embodiments, the LFR2 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 27 or SEQ ID NO: 31.

In certain embodiments, the LFR3 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 24.

In certain embodiments, the LFR3 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 28 or SEQ ID NO: 32.

In certain embodiments, the LFR4 comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 25.

In certain embodiments, the LFR4 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 29 or SEQ ID NO: 33.

In certain embodiments, the VL comprises LFR1, LFR2, LFR3, and LFR4, and the LFR1, LFR2, LFR3, and LFR4 are selected from

i) the LFR1 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 26, the LFR2 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 27, the LFR3 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 28, and the LFR4 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 29; and

ii) the LFR1 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 30, the LFR2 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 31, the LFR3 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 32, and the LFR4 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 33.

In certain embodiments, the VL comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 36.

For example, the functions of the T cell antigen receptor (TCR) and the major histocompatibility complexes (MHCI, MHCII) in the immune effector cell are inhibited in the cell, and the immune effector cell comprises a chimeric antigen receptor (CAR) targeting IL13Rα2. The chimeric antigen receptor (CAR) targeting IL13Rα2 comprises a targeting moiety comprising a VH and a VL, wherein the VH may comprise the amino acid sequence set forth in SEQ ID NO: 18, and the VL may comprise the amino acid sequence set forth in SEQ ID NO: 36.

In certain embodiments, the VL comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 34 or SEQ ID NO: 35.

In certain embodiments, the targeting moiety comprises a VH and a VL, wherein the VH comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 18, and the VL comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 36.

For example, the VH and VL may be selected from:

i) the VH comprising the amino acid sequence set forth in SEQ ID NO: 16, and the VL comprising the amino acid sequence set forth in SEQ ID NO: 34; and

ii) the VH comprising the amino acid sequence set forth in SEQ ID NO: 17, and the VL comprising the amino acid sequence set forth in SEQ ID NO: 35.

In certain embodiments, the targeting moiety includes a full-length antibody, a Fab, a single-chain variable fragment (scFv), or a single-domain antibody (VHH). For example, the targeting moiety includes the scFv.

In certain embodiments, the targeting moiety comprises a linker polypeptide between the VH and the VL.

In certain embodiments, the linker polypeptide is selected from a Gly-Ser linker (SEQ ID NO: 37), a Whitlow linker (SEQ ID NO: 38), and a linker comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 37 or SEQ ID NO: 38.

In certain embodiments, the targeting moiety comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 41 or SEQ ID NO: 44.

For example, the targeting moiety may comprise the amino acid sequence set forth in SEQ ID NO: 41 or SEQ ID NO: 44.

For another example, the targeting moiety may comprise an amino acid sequence set forth in SEQ ID NO: 39, SEQ ID NO: 40, SEQ ID NO: 42, or SEQ ID NO: 43.

For another example, the functions of the T cell antigen receptor (TCR) and the major histocompatibility complexes (MHCI, MHCII) in the immune effector cell are inhibited in the cell, and the immune effector cell comprises a chimeric antigen receptor (CAR) targeting IL13Rα2. The chimeric antigen receptor (CAR) targeting IL13Rα2 comprises a targeting moiety, and the targeting moiety may comprise the amino acid sequence set forth in SEQ ID NO: 39, SEQ ID NO: 40, SEQ ID NO: 42, or SEQ ID NO: 43.

In certain embodiments, the chimeric antigen receptor (CAR) targeting IL13Rα2 comprises a targeting moiety comprising at least one complementarity-determining region (CDR) of an antibody heavy chain variable region (VH), wherein the VH comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 56. For example, the VH comprises the amino acid sequence set forth in SEQ ID NO: 56.

In certain embodiments, the chimeric antigen receptor (CAR) targeting IL13Rα2 comprises a targeting moiety comprising a VH, wherein the VH comprises a heavy chain complementarity-determining region 1 (HCDR1), a heavy chain complementarity-determining region 2 (HCDR2), and a heavy chain complementarity-determining region 3 (HCDR3), and the HCDR3 comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 55. For example, the HCDR3 may comprise the amino acid sequence set forth in SEQ ID NO: 55.

In certain embodiments, the HCDR2 comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 54. For example, the HCDR2 may comprise the amino acid sequence set forth in SEQ ID NO: 54.

In certain embodiments, the HCDR1 comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 53. For example, the HCDR1 may comprise the amino acid sequence set forth in SEQ ID NO: 53.

In certain embodiments, the VH comprises: the HCDR1 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 53, the HCDR2 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 54, and the HCDR3 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 55.

For example, the VH may comprise: the HCDR1 comprising the amino acid sequence set forth in SEQ ID NO: 53, the HCDR2 comprising the amino acid sequence set forth in SEQ ID NO: 54, and the HCDR3 comprising the amino acid sequence set forth in SEQ ID NO: 55.

In certain embodiments, the VH comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 56. For example, the VH may comprise the amino acid sequence set forth in SEQ ID NO: 56.

For another example, the functions of the T cell antigen receptor (TCR) and the major histocompatibility complexes (MHCI, MHCII) in the immune effector cell are inhibited in the cell, and the immune effector cell comprises a chimeric antigen receptor (CAR) targeting IL13Rα2. The chimeric antigen receptor (CAR) targeting IL13Rα2 comprises a targeting moiety comprising a VH, and the VH may comprise the amino acid sequence set forth in SEQ ID NO: 56.

In certain embodiments, the targeting moiety further comprises at least one CDR of an antibody light chain variable region (VL), wherein the VL comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 60.

In certain embodiments, the targeting moiety comprises at least one CDR of a VH and at least one CDR of a VL, wherein the VH comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 56, and the VL comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 60.

In certain embodiments, the chimeric antigen receptor (CAR) targeting IL13Rα2 comprises a targeting moiety comprising a VL, wherein the VL comprises a light chain complementarity-determining region 1 (LCDR1), a light chain complementarity-determining region 2 (LCDR2), and a light chain complementarity-determining region 3 (LCDR3), and the LCDR1 comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 57. For example, the LCDR1 comprises the amino acid sequence set forth in SEQ ID NO: 57.

In certain embodiments, the LCDR2 comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 58. For example, the LCDR2 comprises the amino acid sequence set forth in SEQ ID NO: 58.

In certain embodiments, the LCDR3 comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 59. For example, the LCDR3 comprises the amino acid sequence set forth in SEQ ID NO: 59.

In certain embodiments, the VL comprises the LCDR1 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 57, the LCDR2 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 58, and the LCDR3 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 59.

For example, the functions of the T cell antigen receptor (TCR) and the major histocompatibility complexes (MHCI, MHCII) in the immune effector cell are inhibited in the cell, and the immune effector cell comprises a chimeric antigen receptor (CAR) targeting IL13Rα2. The chimeric antigen receptor (CAR) targeting IL13Rα2 comprises a targeting moiety comprising a VH and a VL, wherein the VH may comprise: the HCDR1 comprising the amino acid sequence set forth in SEQ ID NO: 53, the HCDR2 comprising the amino acid sequence set forth in SEQ ID NO: 54, and the HCDR3 comprising the amino acid sequence set forth in SEQ ID NO: 55; and the VL may comprise: the LCDR1 comprising the amino acid sequence set forth in SEQ ID NO: 57, the LCDR2 comprising the amino acid sequence set forth in SEQ ID NO: 58, and the LCDR3 comprising the amino acid sequence set forth in SEQ ID NO: 59.

In certain embodiments, the VL comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 60.

In certain embodiments, the targeting moiety comprises a VH and a VL, wherein the VH comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 56, and the VL comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 60.

For example, the functions of the T cell antigen receptor (TCR) and the major histocompatibility complexes (MHCI, MHCII) in the immune effector cell are inhibited in the cell, and the immune effector cell comprises a chimeric antigen receptor (CAR) targeting IL13Rα2. The chimeric antigen receptor (CAR) targeting IL13Rα2 comprises a targeting moiety comprising a VH and a VL, wherein the VH may comprise the amino acid sequence set forth in SEQ ID NO: 56, and the VL may comprise the amino acid sequence set forth in SEQ ID NO: 60.

In certain embodiments, the targeting moiety includes a full-length antibody, a Fab, a single-chain variable fragment (scFv), or a single-domain antibody (VHH).

In certain embodiments, the targeting moiety includes the scFv.

In certain embodiments, the targeting moiety comprises a linker polypeptide between the VH and the VL.

In certain embodiments, the linker polypeptide is selected from a Gly-Ser linker (SEQ ID NO: 37), a Whitlow linker (SEQ ID NO: 38), and a linker comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 37 or SEQ ID NO: 38.

In certain embodiments, the targeting moiety comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 61 or SEQ ID NO: 62.

For example, the functions of the T cell antigen receptor (TCR) and the major histocompatibility complexes (MHCI, MHCII) in the immune effector cell are inhibited in the cell, and the immune effector cell comprises a chimeric antigen receptor (CAR) targeting IL13Rα2. The chimeric antigen receptor (CAR) targeting IL13Rα2 comprises a targeting moiety, and the targeting moiety may comprise the amino acid sequence set forth in SEQ ID NO: 61 or SEQ ID NO: 62.

In certain embodiments, the CAR comprises a transmembrane domain, wherein the transmembrane domain comprises a transmembrane domain derived from one or more proteins selected from the group consisting of: CD8A, CD8B, CD28, CD3ε (CD3e), 4-1BB, CD4, CD27, CD7, PD-1, TRAC, TRBC, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L (CD154), TIM1, CD226, DR3, CD45, CD80, CD86, CD9, CD16, CD22, CD33, CD37, CD64, and SLAM.

In certain embodiments, the transmembrane domain comprises a transmembrane domain derived from CD8A. For example, the transmembrane domain may comprise a transmembrane domain derived from CD8A.

In certain embodiments, the transmembrane domain comprises an amino acid sequence having at least about 90%, about 91%, about 92%, about 93%, about 94%, about 95%, about 96%, about 97%, about 98%, about 99%, or about 99.5% identity to an amino acid sequence set forth in any one of SEQ ID NO: 69 to SEQ ID NO: 117. For example, the transmembrane domain may comprise the amino acid sequence set forth in SEQ ID NO: 69.

In certain embodiments, the CAR comprises an intracellular co-stimulatory signaling domain, wherein the intracellular co-stimulatory signaling domain comprises an intracellular co-stimulatory signaling domain derived from one or more proteins selected from the group consisting of: CD28, 4-1BB (CD137), CD27, CD2, CD7, CD8A, CD8B, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88.

In certain embodiments, the intracellular co-stimulatory signaling domain is derived from a co-stimulatory signaling domain of 4-1BB.

In certain embodiments, the intracellular co-stimulatory signaling domain comprises an amino acid sequence having at least about 90%, about 91%, about 92%, about 93%, about 94%, about 95%, about 96%, about 97%, about 98%, about 99%, or about 99.5% identity to the amino acid sequence set forth in any one of SEQ ID NO: 118 to SEQ ID NO: 150. For example, the intracellular co-stimulatory signaling domain may comprise an amino acid sequence set forth in SEQ ID NO: 119.

In certain embodiments, the CAR comprises an intracellular signaling domain, wherein the intracellular signaling domain comprises an intracellular signaling domain derived from one or more proteins selected from the group consisting of: CD3ζ, CD3δ, CD3γ, CD3ε, CD79a, CD79b, FceRIγ, FceRIβ, FcyRIIa, bovine leukemia virus gp30, Epstein-Barr virus (EBV) LMP2A, simian immunodeficiency virus PBj14 Nef, DAP10, DAP-12, and a domain comprising at least one ITAM.

In certain embodiments, the intracellular signaling domain comprises a signaling domain derived from CD3ζ.

In certain embodiments, the intracellular signaling domain comprises an amino acid sequence having at least about 90%, about 91%, about 92%, about 93%, about 94%, about 95%, about 96%, about 97%, about 98%, about 99%, or about 99.5% identity to an amino acid sequence set forth in any one of SEQ ID NO: 134, SEQ ID NO: 138, SEQ ID NO: 139, and SEQ ID NO: 151 to SEQ ID NO: 161. For example, the intracellular signaling domain may comprise the amino acid sequence set forth in SEQ ID NO: 151.

In certain embodiments, the CAR comprises a hinge region between the targeting moiety and the transmembrane domain, and the hinge region comprises a hinge region derived from one or more proteins selected from the group consisting of: CD28, IgG1, IgG4, IgD, 4-1BB, CD4, CD27, CD7, CD8A, PD-1, ICOS, OX40, NKG2D, NKG2C, FcεRIγ, BTLA, GITR, DAP10, TIM1, SLAM, CD30, and LIGHT.

In certain embodiments, the hinge region comprises a hinge region derived from CD8A.

In certain embodiments, the hinge region comprises an amino acid sequence having at least about 90%, about 91%, about 92%, about 93%, about 94%, about 95%, about 96%, about 97%, about 98%, about 99%, or about 99.5% identity to the amino acid sequence set forth in any one of SEQ ID NO: 162 to SEQ ID NO: 183. For example, the hinge region may comprise an amino acid sequence set forth in SEQ ID NO: 170.

In certain embodiments, a non-targeting moiety of the chimeric antigen receptor comprises a transmembrane domain of CD8A molecule, a hinge region of CD8A, an intracellular co-stimulatory signaling domain of 4-1BB, and an intracellular signaling domain of CDSζ.

In certain embodiments, the non-targeting moiety of the chimeric antigen receptor comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 67. For example, the non-targeting moiety of the chimeric antigen receptor may comprise the amino acid sequence set forth in SEQ ID NO: 67.

In certain embodiments, the chimeric antigen receptor further comprises a signal peptide fragment, and the C-terminus of the signal peptide fragment is linked to the N-terminus of the targeting moiety. For example, the chimeric antigen receptor may include a CAR comprising a signal peptide, an anti-IL13Rα2 scFv, a CD8A hinge domain, a CD8A transmembrane domain, a 4-1BB co-stimulatory domain, and a CD3ζ primary signaling domain.

In certain embodiments, the signal peptide fragment includes a CD8A signal peptide fragment.

In certain embodiments, the signal peptide fragment comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 184. For example, the signal peptide fragment may comprise the amino acid sequence set forth in SEQ ID NO: 184.

In certain embodiments, the chimeric antigen receptor comprises an amino acid sequence having at least about 90%, about 91%, about 92%, about 93%, about 94%, about 95%, about 96%, about 97%, about 98%, about 99%, or about 99.5% identity to an amino acid sequence set forth in SEQ ID NO: 45 or SEQ ID NO: 46. For example, the chimeric antigen receptor may comprise the amino acid sequence set forth in any one of SEQ ID NO: 45 and SEQ ID NO: 46.

In certain embodiments, the immune effector cell includes a human cell.

In certain embodiments, the immune effector cell includes a T cell, a B cell, a natural killer cell (NK cell), a macrophage, an NKT cell, a monocyte, a dendritic cell, a granulocyte, a lymphocyte, a leukocyte, and/or a peripheral blood mononuclear cell. For example, the immune effector cell may be a T cell, such as a human T cell.

In certain embodiments, the immune effector cell includes an autologous or non-autologous immune effector cell. For example, the immune effector cell may be a non-autologous human T cell.

In certain embodiments, the immune effector cell comprises a modified immune effector cell, wherein the modification comprises down-regulation of the expression and/or activity of one or more of immune rejection-related genes.

In certain embodiments, the immune rejection-related gene is selected from one or more of the following groups:

TRAC, TRBC, HLA-A, HLA-B, B2M, and CIITA.

In certain embodiments, the expression and/or activity of the TRAC gene and the HLA-A gene in the modified immune effector cell is down-regulated as compared to a corresponding unmodified cell.

In certain embodiments, the expression and/or activity of the CIITA gene in the modified immune effector cell is not down-regulated as compared to the corresponding unmodified cell.

In certain embodiments, the expression and/or activity of the B2M gene in the modified immune effector cell is not down-regulated as compared to the corresponding unmodified cell.

For example, in the modified immune effector cell, the expression and/or activity of the TRAC and HLA-A genes may be down-regulated, and the expression and/or activity of the CIITA and B2M genes may not be down-regulated as compared to the corresponding unmodified cell.

In certain embodiments, the expression and/or activity of the TRAC gene and the HLA-A gene in the modified immune effector cell is down-regulated as compared to a corresponding wild-type cell.

In certain embodiments, the expression and/or activity of the B2M gene in the modified immune effector cell is not down-regulated as compared to the corresponding wild-type cell.

In certain embodiments, the expression and/or activity of the CIITA gene in the modified immune effector cell is not down-regulated as compared to the corresponding wild-type cell.

For example, in the modified immune effector cell, the expression and/or activity of the TRAC and HLA-A genes may be down-regulated, and the expression and/or activity of the CIITA and B2M genes may not be down-regulated as compared to the corresponding wild-type cell.

In certain embodiments, the down-regulation of the expression level and/or activity of the gene includes down-regulating the expression and/or activity of a nucleic acid molecule encoding the gene; and/or down-regulating the expression and/or activity of a protein product encoded by the gene.

In certain embodiments, the modification comprises: gene knockout, gene mutation, and/or gene silencing.

In certain embodiments, the modification comprises knocking out either of two TRAC alleles and knocking out either of two HLA-A alleles in the immune effector cell.

In certain embodiments, the modification comprises knocking out the two TRAC alleles and knocking out either of the two HLA-A alleles in the immune cell.

In certain embodiments, the modification comprises knocking out an exon of the TRAC gene and knocking out an exon of the HLA-A gene in the immune cell.

In certain embodiments, the modification comprises administering to the immune effector cell one or more substances selected from the group consisting of: antisense RNA, siRNA, shRNA, and a CRISPR/Cas9 system.

In certain embodiments, the modification comprises administering to the immune effector cell the CRISPR/Cas9 system.

In certain embodiments, the modification further comprises administering to the immune effector cell sgRNA targeting an exon portion of the TRAC gene.

In certain embodiments, the sgRNA targeting the exon portion of the TRAC gene comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 186 to SEQ ID NO: 200.

In certain embodiments, the modification comprises administering to the immune effector cell sgRNA targeting an exon portion of the HLA-A gene.

In certain embodiments, the sgRNA targeting the exon portion of the HLA-A gene comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 201 to SEQ ID NO: 241.

In certain embodiments, the modification further comprises administering to the cell a Cas enzyme.

In certain embodiments, the Cas enzyme includes a Cas9 protein.

In certain embodiments, the antisense RNA comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 242 to SEQ ID NO: 245.

In certain embodiments, the immune effector cell is an HLA-B homozygous cell.

In certain embodiments, the HLA-B homozygote includes HLA-B*40 homozygote, HLA-B* 15 homozygote, HLA-B*46 homozygote, HLA-B* 13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

In certain embodiments, the immune effector cell is an HLA-A homozygous or heterozygous cell.

In certain embodiments, the HLA-A homozygote or heterozygote includes HLA-A*02 homozygote, HLA-A* 11 homozygote, HLA-A* 02/A* 11 heterozygote, or HLA-A*24 homozygote.

For example, the immune effector cell may be a human T cell, and the human T cell may be an HLA-B homozygous cell.

**Preparation method for immune effector cells**

In another aspect, the present application provides a method for preparing the aforementioned immune effector cell, which comprises: modifying the immune effector cell before/after introducing a polynucleotide sequence encoding the aforementioned CAR targeting IL13Rα2 or a vector comprising the polynucleotide sequence encoding the aforementioned CAR targeting IL13Rα2 into the immune effector cell, wherein the modification comprises down-regulation of the expression and/or activity of one or more of immune rejection-related genes.

For example, the method for preparing an immune effector cell may comprise:

(1) introducing the aforementioned nucleic acid molecule or the aforementioned vector into an immune effector cell; and
(2) modifying the immune effector cell, wherein the modification comprises down-regulation of the expression and/or activity of one or more of immune rejection-related genes.

For example, the method for preparing an immune effector cell may comprise:

(1) collecting peripheral blood of healthy people, separating PBMCs, adding CD3 magnetic beads according to a proportion for incubation, and sorting CD3+ T cells; uniformly mixing CD3/CD28 antibody-coupled magnetic beads, measuring an appropriate amount of magnetic bead suspension according to the calculated amount, adding the magnetic bead suspension into a T cell culture system, activating the T cells, and performing overnight culture;

(2) infecting the T cells according to the titer of IL13Rα2 CAR virus;

(3) simultaneously knocking out TRAC and HLA-A genes; and

(4) sorting CD3-negative T cells: adding CD3 magnetic beads according to a proportion, and collecting CD3-T cells (cells not bound to the magnetic beads).

In certain embodiments, the vector is an expression vector.
In certain embodiments, the vector is selected from a DNA vector, an RNA vector, a plasmid, a lentiviral vector, an adenoviral vector, an adeno-associated viral vector, and a retroviral vector.
In certain embodiments, the vector further comprises an EF-1α promoter.
In certain embodiments, the vector further comprises a woodchuck hepatitis virus post-transcriptional regulatory element (WPRE).
In certain embodiments, the immune rejection-related gene is selected from one or more of the following groups: TRAC, TRBC, HLA-A, HLA-B, B2M, and CIITA. For example, the immune rejection-related genes may include TRAC and/or HLA-A.
In certain embodiments, the expression and/or activity of the TRAC gene and the HLA-A gene in the immune effector cell is down-regulated as compared to the expression and/or activity of a corresponding gene in a corresponding unmodified cell.
In certain embodiments, the expression and/or activity of the CIITA gene is not down-regulated as compared to the expression and/or activity of the corresponding gene in the corresponding unmodified cell.
In certain embodiments, the expression and/or activity of the B2M gene is not down-regulated as compared to the expression and/or activity of the corresponding gene in the corresponding unmodified cell.
For example, in the modified immune effector cell, the expression and/or activity of the TRAC and HLA-A genes may be down-regulated, and the expression and/or activity of the CIITA and B2M genes may not be down-regulated as compared to the corresponding unmodified cell.
In certain embodiments, the expression and/or activity of the TRAC gene and the HLA-A gene in the immune effector cell is down-regulated as compared to a corresponding wild-type cell.
In certain embodiments, the expression and/or activity of the CIITA gene is not down-regulated as compared to the corresponding wild-type cell.
In certain embodiments, the expression and/or activity of the B2M gene is not down-regulated as compared to the corresponding wild-type cell.
In certain embodiments, the down-regulation of the expression level and/or activity of the gene includes down-regulating the expression and/or activity of a nucleic acid molecule encoding the gene; and/or down-regulating the expression and/or activity of a protein product encoded by the gene.

For example, in the modified immune effector cell, the expression and/or activity of the TRAC and HLA-A genes may be down-regulated, and the expression and/or activity of the CIITA and B2M genes may not be down-regulated as compared to the corresponding wild-type cell.

In certain embodiments, the modification comprises: gene knockout, gene mutation, and/or gene silencing.

In certain embodiments, the modification comprises knocking out either of two TRAC alleles and knocking out either of two HLA-A alleles in the immune effector cell.

In certain embodiments, the modification comprises knocking out the two TRAC alleles and knocking out either of the two HLA-A alleles in the immune cell.

In certain embodiments, the modification comprises knocking out an exon of the TRAC gene and knocking out an exon of the HLA-A gene in the immune cell.

In certain embodiments, the modification comprises administering to the immune effector cell one or more substances selected from the group consisting of: antisense RNA, siRNA, shRNA, and a CRISPR/Cas9 system.

In certain embodiments, the modification comprises administering to the immune effector cell the CRISPR/Cas9 system.

In certain embodiments, the modification comprises administering to the immune effector cell sgRNA targeting an exon portion of the TRAC gene.

In certain embodiments, the sgRNA targeting the exon portion of the TRAC gene comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 186 to SEQ ID NO: 200.

In certain embodiments, the modification comprises administering to the immune effector cell sgRNA targeting an exon portion of the HLA-A gene.

In certain embodiments, the sgRNA targeting the exon portion of the HLA-A gene comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 201 to SEQ ID NO: 241.

In certain embodiments, the modification further comprises administering to the cell a Cas enzyme.

In certain embodiments, the Cas enzyme includes a Cas9 protein.

In certain embodiments, the antisense RNA comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 242 to SEQ ID NO: 245.

In certain embodiments, the immune effector cell includes a human cell.

In certain embodiments, the immune effector cell includes a T cell, a B cell, a natural killer cell (NK cell), a macrophage, an NKT cell, a monocyte, a dendritic cell, a granulocyte, a lymphocyte, a leukocyte, and/or a peripheral blood mononuclear cell. For example, the immune effector cell may be a T cell, such as a human T cell.

In certain embodiments, the immune effector cell includes an autologous or non-autologous immune effector cell.

In certain embodiments, the cell is an HLA-B homozygous cell. For example, the immune effector cell may be a human cell, and the human cell may be an HLA-B homozygous cell. For another example, the immune effector cell may be a human T cell, and the human cell may be an HLA-B homozygous cell.

In certain embodiments, the HLA-B homozygote includes HLA-B*40 homozygote, HLA-B* 15 homozygote, HLA-B*46 homozygote, HLA-B*13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

In certain embodiments, the cell is an HLA-A homozygous or heterozygous cell.

In certain embodiments, the HLA-A homozygote or heterozygote includes HLA-A*02 homozygote, HLA-A* 11 homozygote, HLA-A*02/A*11 heterozygote, or HLA-A*24 homozygote.

## Use, **pharmaceutical composition, and treatment method**

In another aspect, the present application further provides use of the aforementioned immune effector cell in the preparation of a CAR-T cell.

In another aspect, the present application further provides a pharmaceutical composition comprising the aforementioned immune effector cell and optionally a pharmaceutically acceptable carrier.

In another aspect, the present application further provides use of the aforementioned immune effector cell and/or the aforementioned pharmaceutical composition in the treatment of a disease or disorder associated with the expression of IL13Rα2.

In certain embodiments, the disease or disorder associated with the expression of IL13Rα2 includes a disease or disorder associated with up-regulation of the expression of IL13Rα2.

In certain embodiments, the disease or disorder associated with the expression of IL13Rα2 includes cancer.

In certain embodiments, the cancer includes an IL13Rα-positive tumor.

In certain embodiments, the IL13Rα-positive tumor includes malignant gliomas, colorectal cancer, cervical cancer, pancreatic cancer, cutaneous melanoma, ovarian cancer, breast cancer, adrenocortical carcinoma, thymoma, uterine cancer, bladder cancer, esophageal cancer, head and neck squamous cell carcinoma, or gastric cancer.

In another aspect, the present application further provides use of the aforementioned immune effector cell and/or the aforementioned pharmaceutical composition in the preparation of a medicament for treating a disease or disorder associated with the expression of IL13Rα2.

In certain embodiments, the disease or disorder associated with the expression of IL13Rα2 includes a disease or disorder associated with up-regulation of the expression of IL13Rα2.

In certain embodiments, the disease or disorder associated with the expression of IL13Rα2 includes cancer.

In certain embodiments, the cancer includes an IL13Rα-positive tumor.

In certain embodiments, the IL13Rα-positive tumor includes malignant gliomas, colorectal cancer, cervical cancer, pancreatic cancer, cutaneous melanoma, ovarian cancer, breast cancer, adrenocortical carcinoma, thymoma, uterine cancer, bladder cancer, esophageal cancer, head and neck squamous cell carcinoma, or gastric cancer.

In another aspect, the present application further provides a method for preventing or treating a disease or disorder associated with the expression of IL13Rα2, which comprises administering to a subject in need thereof an effective amount of the aforementioned immune effector cell and/or the aforementioned pharmaceutical composition.

In certain embodiments, the disease or disorder associated with the expression of IL13Rα2 includes a disease or disorder associated with up-regulation of the expression of IL13Rα2.

In certain embodiments, the disease or disorder associated with the expression of IL13Rα2 includes cancer.

In certain embodiments, the cancer includes an IL13Rα-positive tumor.

In certain embodiments, the IL13Rα-positive tumor includes malignant gliomas, colorectal cancer, cervical cancer, pancreatic cancer, cutaneous melanoma, ovarian cancer, breast cancer, adrenocortical carcinoma, thymoma, uterine cancer, bladder cancer, esophageal cancer, head and neck squamous cell carcinoma, or gastric cancer.

Without being bound by any theory, the following examples are intended only to illustrate the chimeric antigen receptor, immune effector cell, preparation method, use, etc., of the present application, and are not intended to limit the scope of the present application.

**Examples**

**Example 1**

**1.1 Design of Anti-IL13Rα2 Chimeric Antigen Receptor (CAR)**

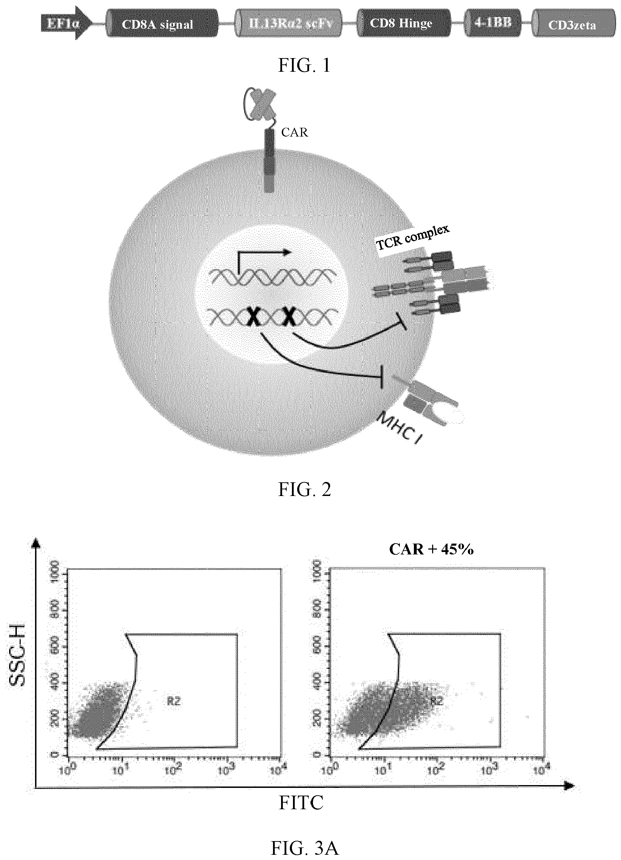

As shown in FIG. 1, the anti-IL13Rα2 CAR VLVH (murine) structure comprised: an IL13Rα2 antigen-binding region (derived from an anti-IL13Rα2 murine monoclonal antibody Scfv), a CD8A extracellular hinge region, a CD8A transmembrane region, a 4-1BB intracellular co-stimulatory domain, and a CD3ζ activation signal domain. The DNA sequence of the structure was set forth in SEQ ID NO: 51, and the amino acid sequence thereof was set forth in SEQ ID NO: 45.

The anti-IL13Rα2 CAR VHVL (humanized) structure comprised: an IL13Rα2 antigen-binding region (derived from an anti-IL13Rα2 humanized monoclonal antibody Scfv), a CD8A extracellular hinge region, a CD8A transmembrane region, a 4-1BB intracellular co-stimulatory domain, and a CD3ζ activation signal domain. The DNA sequence of the structure was set forth in SEQ ID NO: 52, and the amino acid sequence thereof was set forth in SEQ ID NO: 46.

**1.2 Lentiviral Vector Construction of IL13Rα2 CAR**

According to the sequence information on IL13Rα2 and the structure of the CAR vector, an IL13Rα2 CAR lentiviral expression vector was constructed, with the vector schematic shown in FIG. 1. The optimization was performed: a commercial lentiviral expression vector pCDH-CMV-MCS-EF1-copGFP was selected as a backbone, and element modification was performed on the basis of the vector. First, an ampicillin resistance gene β-lactamase on the vector was replaced with aminoglycoside phosphotransferase derived from Tn5 to enable the vector to have kanamycin resistance. Secondly, we deleted the CMV promoter and its adjacent downstream multiple cloning site, which were potentially threatening in *in vivo* applications. Finally, the copGFP gene that was started to express by the EF1 promoter in the original vector was deleted; a SalI enzyme digestion site was retained, and a SmaI enzyme digestion site was added to the 5' end of SalI for vector construction to form a final target vector. The added SmaI enzyme digestion site was a single enzyme digestion site of the final target vector, and other sequence portions of the vector did not have the enzyme digestion site. After the optimization, the chimeric antigen receptor lentiviral expression vector was constructed, and lentiviral packaging was performed after confirming that the sequence was correct by Sanger sequencing.

**1.3 Design of Guide RNA**

Corresponding gene sequences were searched and downloaded through a website https://www.ncbi.nlm.nih.gov/; the gene sequences were opened by using SnapGene software, and sgRNAs could be designed on different exons of target genes. The sgRNA of the CRISPR/Cas9 system used in this example was designed following a non-restrictive principle of: 5'-NNN(20)-NGG-3', NGG being referred to as a protospacer adjacent motif (PAM), wherein N represented A, T, C, or G. Since many sgRNAs could be designed on the same exon and the sgRNA composed of 20 nucleotide sequences could repeatedly occur in a genome, the design and evaluation of sgRNAs were performed using the website http://crispr.cos.uni-heidelberg.de. An exon sequence was pasted to the website, and the sgRNAs were designed and subjected to a prediction evaluation. The higher the score in the evaluation was, the higher the editing efficiency and the lower the off-target risk could exist. The sgRNAs with higher scores were selected for assay. The sgRNAs targeting the TRAC gene were set forth in SEQ ID NO: 186 to SEQ ID NO: 200, the sgRNAs targeting the HLA-A02 gene were set forth in SEQ ID NO: 201 to SEQ ID NO: 222, the sgRNAs targeting the HLA-A11 gene were set forth in SEQ ID NO: 223 to SEQ ID NO: 233, and the sgRNAs targeting the HLA-A24 gene were set forth in SEQ ID NO: 234 to SEQ ID NO: 241, which were synthesized by GenScript Biotech Corporation.

**Example 2**

**2.1 Donor Selection**

HLA-B homozygotes which matched with HLA-B typing of a receptor were selected based on the HLA-B typing of the receptor.
First, the donor source was based on HLA-B homozygotes in the population, and one of the alleles of HLA-B in the patient was consistent with the HLA-B homozygotes in the donor, so that cells from these donors could cover a high number of patient populations, and the rejection response caused by inconsistencies in HLA-B subtypes was reduced. HLA-B, such as B*40 homozygote, B*15 homozygote, B*46 homozygote, B*13 homozygote, B*51 homozygote, B*58 homozygote, B*07 homozygote, B*35 homozygote, B*44 homozygote, B*52 homozygote, B*57 homozygote, B*54 homozygote, and B*55 homozygote, was mainly selected, which had relatively high frequency in the population. HLA-A, such as A*02 homozygote, A* 11 homozygote, and A*02/A11 heterozygote, was selected, which had relatively high frequency in the population.

**2.2 Preparation of CD3+ T Cells**

(1) Isolation of PBMCs from peripheral blood

Peripheral blood was collected from healthy donors and diluted with PBS buffer at a rate of 1:1. A cell isolation solution (Ficoll) with a blood volume of 1/3 after dilution was first added into a new 50 mL centrifuge tube; then the blood cell dilution was added very slowly along the tube wall, and the mixture was centrifuged at room temperature for 20 min at 800 g (for the centrifuge, the acceleration speed was set as 1, and the deceleration speed was set as 0). After centrifugation, the liquid in the centrifuge tube was divided into PBS, a serum layer, a leucocyte layer, a lymphocyte isolation solution, and a red blood cell layer from top to bottom. The PBS and serum layer were removed. The leucocyte layer was transferred to a new 50 mL centrifuge tube; PBS was added to 40 mL to wash the cells, and the mixture was centrifuged for 10 min at 450 g. The supernatant was discarded after centrifugation to obtain peripheral blood mononuclear cells. The cells were resuspended and then subjected to cell counting.

(2) Thawing of cryopreserved healthy human PBMCs

Cryopreserved healthy human PBMC cells were thawed in a 37 °C water bath. After complete thawing, the cells were pipetted into a 15 mL centrifuge tube containing 10 mL of X-VIVO15 culture medium containing 10% FBS (purchased from LONZA), and centrifuged for 8 min at 400 g; the supernatant was discarded, 2 mL of X-VIVO15 culture medium (containing 10% FBS and DNase I with a final concentration of 100 $\mu$g/mL) was added, and the cells were incubated at room temperature for 15 min and shaken continuously during incubation; the solution after incubation was filtered by using a 40 $\mu$m filter; 10 mL of PBS buffer was pipetted to resuspend the cells at the bottom, and then the cells were added onto the filter; the cells were centrifuged for 8 min at 400 g after filtration, and the supernatant was discarded after centrifugation; the cells were resuspended and then subjected to cell counting.

(3) Sorting of CD3+ T cells

T cells in the peripheral blood mononuclear cells (PBMCs) were extracted using an EasySep™ human T cell sorting kit (purchased from StemCell Technologies, Catalog No. 17951). The density of PBMCs was adjusted to 5 × 10^7 cells/mL, and a PBS buffer was added in a range of 0.25-2 mL; a cocktail was added firstly and mixed uniformly, and an isolation cocktail was then added at 50 pL/mL; after uniform mixing, the mixture was left at room temperature for 5 min; the RapidSpheres were vortexed by a vortex oscillator for 30 s, added into the cells at 40 μL/mL, and mixed uniformly; the mixture was supplemented with a buffer to the fold of 2.5 mL, and gently pipetted up and down for 2-3 times; the mixture was added into cryopreservation tubes with 2.5 mL in each tube, and the tubes were placed on a magnetic frame and left at room temperature for 3 min; the covers of the cryopreservation tubes were gently removed, and the magnetic frame was carefully picked up by holding two ends of the magnetic frame and inverted for 2-3 s; the cell liquids were poured into new centrifuge tubes at one time; the cells were resuspended in 10-20 mL of a buffer (depending on the number of cells) and then centrifuged for 10 min at 300 g, and the supernatant was discarded to obtain CD3+ T cells.

(4) Activation of T cells

An activating reagent was prepared according to a volume ratio of culture medium:Transact = 99:1. The culture medium was X-VIVO15 culture medium (containing 5% FBS, 200 U/mL IL2, 10 ng/mL IL7, and 5 ng/mL IL15), and Transact was purchased from Miltenyi. The T cells were thoroughly resuspended in 1 mL of activating reagent (containing 10 μL of Transact) per 1 × ^6 cells, and then incubated in an incubator with 5% $CO_2$ at 37 °C for 1 day.

## Example 3

As shown in FIG. 1, in anti-IL13RA2 UCAR-T, TCR and HLA-A of T cells were knocked out through gene editing technology, enabling the T cells to express anti-IL13Rα2 CAR molecules through lentiviral transfection.

### 3.1 Virus Transfer

CD3+ T cells were obtained according to the method in Example 2 (D0) and activated with CD3/CD28 antibody magnetic beads. After activation, lentiviral vectors (IL13Rα2 CAR lentiviral expression vectors prepared in Example 1) were transfected on D1; the lentiviral vectors were washed off on D2, and electroporation was performed on D3.

### 3.2 Gene Knockout

RNP complexes were transferred to the activated T cells prepared in Example 3.1 (the CAR-T cells on D3 were used as starting cells) by electroporation using an electroporation kit (purchased from LONZA, Catalog No. V4XXP-3024). After sampling and counting, the cells were collected and centrifuged, and the cell pellet was resuspended in PBS. A culture medium (X-VIVO15 culture medium + 10% FBS + IL2 (200 U/mL) + IL7 (10 ng/mL) + IL15 (5 ng/mL)) was pre-heated for 30 min in advance in a well plate. An electroporation buffer was prepared according to a ratio of Nucleofector Solution: Supplement = 82:18; the RNP complexes were distributed according to each electroporation system using $1 \times 10^7$ cells (Cas9: sgRNA = 2:1). 10 μg of sgRNA was first added to a PCR tube (without RNase), 20 μg of Cas9 protein (purchased from Thermo, Catalog No. A36499) was then added, and the mixture was mixed gently and incubated at room temperature for 12 min. The cells described above were counted and centrifuged for 8 min at 300 g, and the supernatant was discarded. PBS was added to resuspend the cells; 1E7 cells were pipetted and centrifuged again for 8 min at 300 g, and the supernatant was discarded. The cells were resuspended in 100 μL of the prepared electroporation buffer. The incubated RNP complexes were added to the cell suspension described above. The mixture was gently mixed and gently transferred to an electroporation cuvette. The electroporation cuvette was placed on a Lonza-4D electroporation apparatus and subjected to electroporation using an EO-115 electroporation program. A pre-heated culture medium was added into the electroporation cuvette. The cells were transferred into the pre-heated culture medium in the well plate by using a matched pipette and then placed in an incubator with 5% $CO_2$ at 37 °C for 48 h, and then the cells were collected. The editing efficiency was assayed by Sanger sequencing, and the knockout efficiency of the collected cells was assayed by FACS.

sgRNA sequences were as follows: TRAC sgRNA: AGAGTCTCTCAGCTGGTACA (SEQ ID NO: 186), A02 sgRNA: CTGACCATGAAGCCACCCTG (SEQ ID NO: 203), and A11 sgRNA: GGCCCCTCCTGCTCTATCCA (SEQ ID NO: 233).

### 3.3 Sorting of CD3-negative T cells

CD3-negative T cells were sorted. The cells were counted and centrifuged, and the supernatant was discarded; the cells were resuspended in a buffer and mixed uniformly; CD3 magnetic beads were added according to 20 $\mu$L of CD3 magnetic beads per $10^7$ cells, and the mixture was mixed uniformly and incubated in a refrigerator at 4 °C; the cells were washed with the buffer and centrifuged, and then the magnetic beads were isolated; a column was first put on a magnetic pole, and a centrifuge tube was correspondingly put below the magnetic pole; the column (LD) was infiltrated in the buffer, and the cells were added onto the column without generating bubbles; the column was washed 2 times with the buffer, the washed liquid (CD3-T) was collected in a 15 mL centrifuge tube, and a part of the cells were subjected to cell counting.

### 3.4 Cell Culturing

The cell state was observed under a microscope. The cells were diluted, counted, and supplemented with a full culture medium to maintain the cell density at 3 × 10^5 to 1 × 10^6 cells/mL. Liquid was supplemented/changed in the middle of the process, and the cells were cultured at 37 °C with 5% $CO_2$. Cell harvesting: the cell suspension was collected in a cell centrifuge tube and centrifuged, and the supernatant was discarded. The cells were washed with normal saline again and centrifuged. A cryopreservation solution was prepared, and the centrifuged cells were resuspended in the cryopreservation solution. The cell suspension was pipetted to a cell cryopreservation bag for a final product by using a syringe, and the cell cryopreservation bag was labeled for the later cryopreservation.

### 3.5 Assay of Gene Knockout Efficiency

(1) Sanger sequencing assay

The cells were counted. 3 × $10^4$ to 5 × $10^4$ cells were centrifuged for 5 min at 2000 r/min, and the supernatant was discarded as much as possible. 20 $\mu$L of DE lysis buffer was added into each tube. The lysed cells were added into a PCR tube, centrifuged instantaneously, and then placed into a PCR apparatus with the following conditions: 65 °C for 30 min, 4 °C for 30 s, 95 °C for 2 min, and 16 °C for infinite time. PCR was performed by using primer pairs TRAC-For/TRAC-Rev or HLA-A For/HLA-A Rev, and the lysed product was used as a template. The PCR product was sent to Genewiz for Sanger sequencing. After obtaining the Sanger sequencing results, the editing site and the editing efficiency were predicted with the EditR editor on the website: https://moriaritylab.shinyapps.io/editr_v10/.

(2) Cell counting by flow cytometry

10E5 to 10E8 cells were centrifuged for 5 min at 2000 rpm, and the supernatant was discarded. 100 $\mu$L of PBS buffer was added to each tube to resuspend the cells, and 5 $\mu$L of anti-human AB TCR-APC antibody (purchased from eBioscience), 5 $\mu$L of HLA-A02 monoclonal antibody (BB7.2), APC, and eBioscince™ antibody (purchased from Invitrogen) were added. The mixture was mixed uniformly and incubated at room temperature for 10 min. After being centrifuged for 5 min at 2000 rpm, the cells were washed 2 times with the PBS buffer, resuspended, and assayed by a BD FACSAria flow cytometer. The positive expression rates of TCR and HLA-A02 on the cell surface could be obtained. Knockout efficiency = (A - B)/A × 100%, wherein A was the positive expression rate of the control group; B was the positive expression rate of the knockout group.

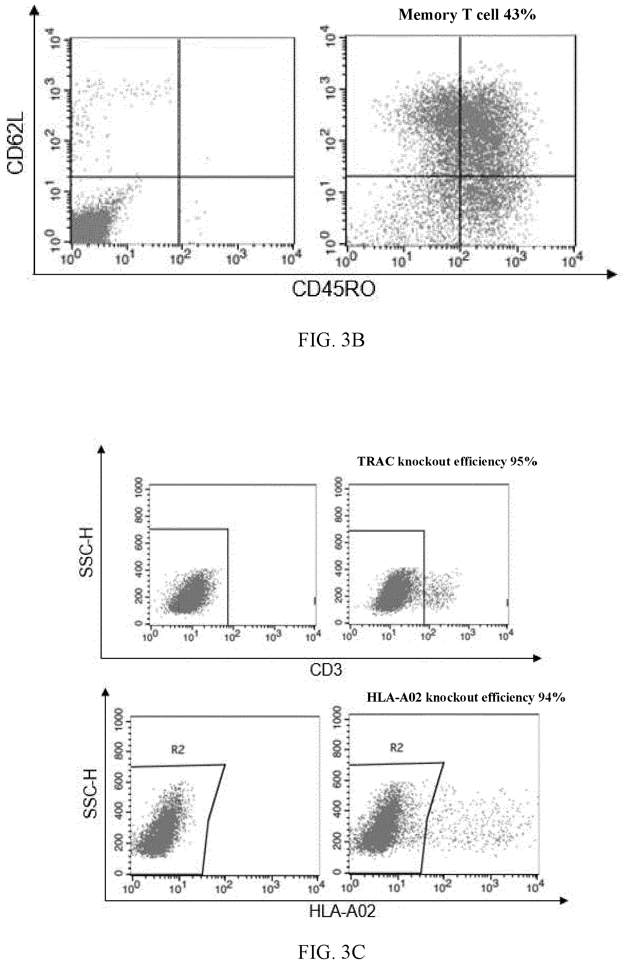

The results are shown in FIGs. 3A-3C. The CAR positive rate of anti- IL13Rα2 UCAR-T cells can be more than 45% (FIG. 3A), the central memory ratio of the anti-IL13Rα2 UCAR-T cells is about 43% (FIG. 3B), and the double knockout efficiency of the anti-IL13Rα2 UCAR-T cells is up to 94% (FIG. 3C).

### Example 4. *In Vitro* Cytotoxicity Analysis of Anti-IL13Rα2 UCAR-T Cells (IL13Rα2 CAR Murine)

### 4.1 Killing of IL13Rα2 UCAR-T Cells for Target Cells

(1) IL13Rα2 target cells: U251-LG, luciferase + GFP; the state of the target cells was adjusted to the log phase, and the cells were continuously passaged 2 times before experiments;

(2) the IL13Rα2 UCAR-T cells and T cells in a control group IL13Rα2 CAR-T were prepared. The knockout efficiency, transfection efficiency, CD3-T sorting efficiency and the proportion of memory T cells were assayed by flow cytometry, and the amplification fold was counted;

(3) several groups of prepared cells were collected by centrifugation, each group of 6 × 10^6 cells;

(4) the target cells were resuspended in 1640 + 10% FBS. For each target, three 24-well plates were taken, and the target cells were seeded at 2 × 10^5 cells/well (both target and effector cells were seeded at a density of 2 × 10^6 cells/mL). Effector cells were then added in an E/T (effector-to-target ratio, effector cell:target cell) ratio. Each well was supplemented to a maximum volume (e.g., 600 μL). The same amount of target cells were seeded in the control group, without effector cells (600 μL). The well plates were incubated in an incubator with 5% $CO_2$ at 37 °C for 24 h. The cells were plated in the following E/T: 1:2, 1:1, 2:1, 5:1, and 10: 1 and repeated three times; and

(5) After 24 h of culture, the well plates were taken out of the incubator, and 200 μL of supernatant was collected. The lysis capacity of the recombinant CAR-T cells on the target cells was then reflected by the assay of Luciferase activity.

The calculation formula for the lysis percentage of the target cells was as follows:

$$Lysis\% = (1 - \frac{Luc\text{Activity}_{\text{Mixed sample}}}{Luc\text{Activity}_{\text{Control sample}}}) \times 100\%$$

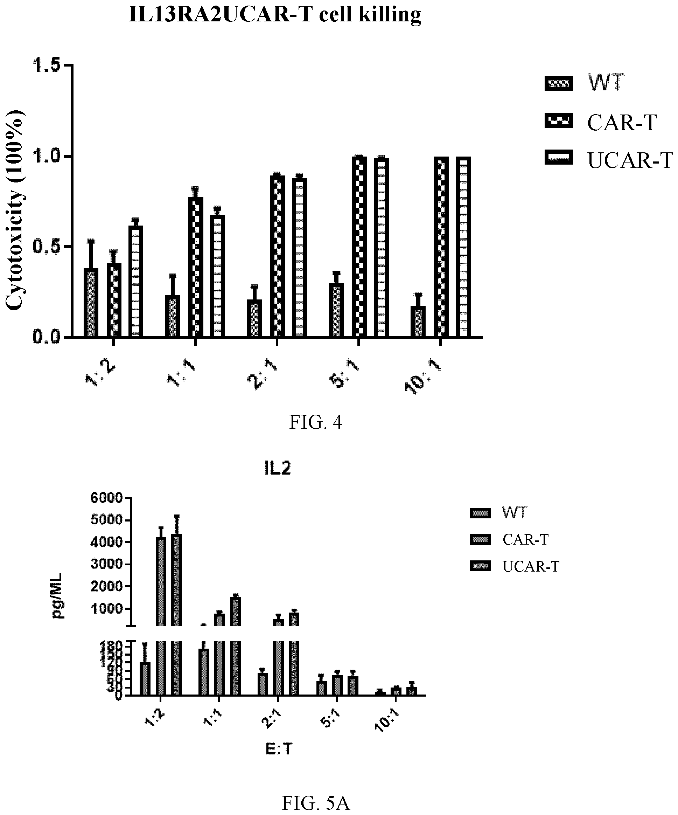

Analysis of results (FIG. 4): the anti-IL13RA2 UCAR-T cells and T cells were separately co-incubated with the target cells U251, and the killing efficiency of more than 90% could be achieved when the effector-to-target ratio was 2:1.

**4.2 Cytokine Secretion Assay of IL13Rα2 UCAR-T Cells Co-Cultured with Target Cells**

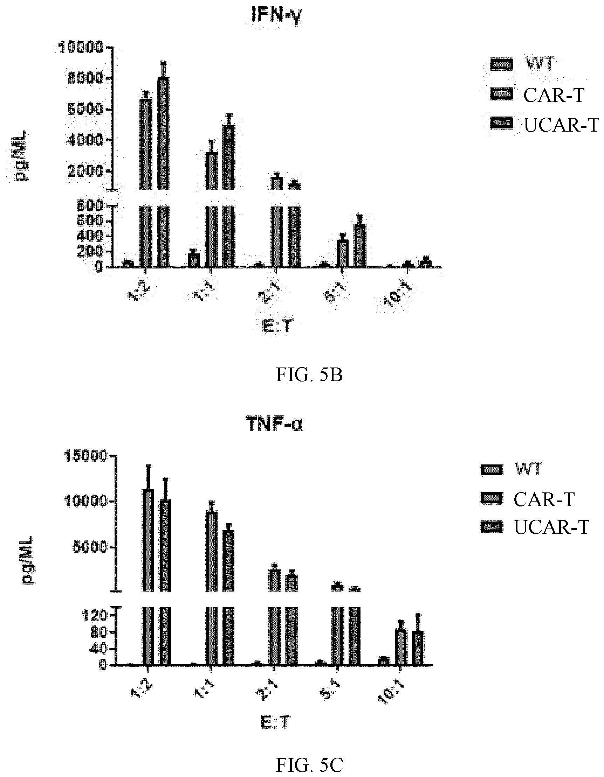

The supernatant of the co-culture system described above was collected, and the cytokine secretion level was assayed.
Analysis of results (FIGs. 5A-5C): anti-IL13Rα2 UCAR-T was activated significantly, and IL-2, IFN-γ and TNF-α cytokines were secreted in large quantities.

**Example 5. *In Vivo* Anti-Tumor Effect of Anti-IL13Rα2 UCAR-T Cells (IL13Rα2 CAR Murine)**

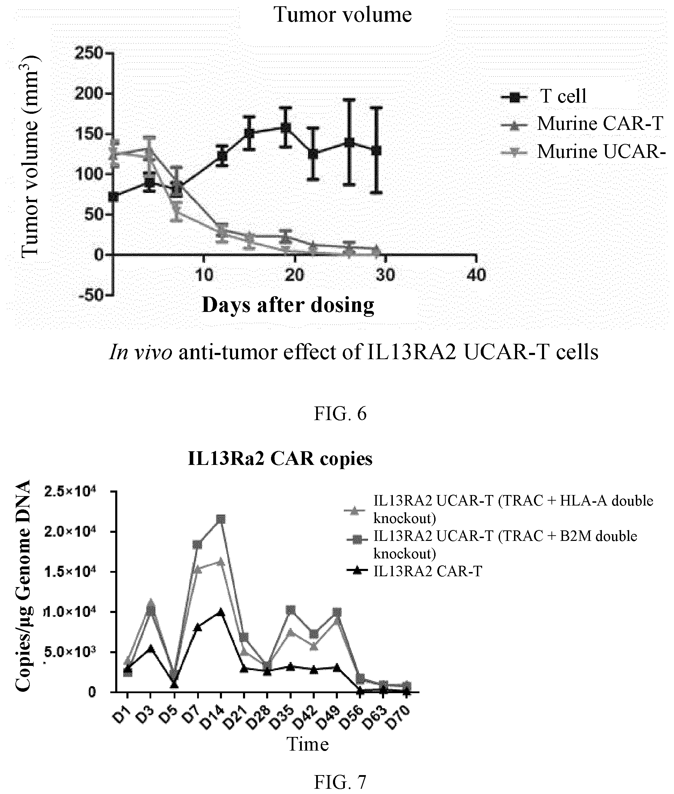

NSG mice aged 8-10 weeks were injected subcutaneously with tumor cells U251-LG (5 × 10^6) and divided into three groups of 5 mice per group, and the tumor formation time was generally 2-4 weeks. 5E6 IL13Rα2 UCAR-T cells, IL13Rα2 CAR-T cells, and T cells without gene knockout were separately injected intratumorally into each group of mice by single-point injection with an injection volume of 50 μL. Tumor regression in mice was monitored by luciferase.
Analysis of results (FIG. 6): the mice to which the anti-IL13Rα2 UCAR-T and anti-IL13Rα2 CAR-T cells were reinfused exhibited a significantly slower growth rate of the tumor.

**Example 6. *In Vivo* Half-Life Assay of Anti-IL13Rα2 UCAR-T Cells (IL13Rα2 CAR Murine)**

15 humanized immune system mice (hHSC-NCG) were prepared and divided into 3 groups. Cells were prepared as follows: an experimental group: IL13Rα2 UCAR-T cells (TRAC + HLA-A02 knocked out); control group 1: IL13Rα2 CAR-T; and control group 2: IL13Rα2 UCAR-T cells (TRAC + B2M knocked out). Each mouse was injected with 1 × 10^7 cells, and blood was collected at different time points: D0, 2 h, D3, D7, D14, D21, D28, D35, D42, D49, D56, and D60. Genomes in blood samples at different time points were extracted, and copy/ng genome DNA was calculated by QPCR absolute quantification method. UCAR-T cells harvested on day 14 were used as a positive control, and DEPC water was used as a negative control.
Analysis of results (FIG. 7): the anti-IL13Rα2 UCAR-T cells (TRAC + HLA-A02 knocked out) survived in the mice for about 70 days and amplified twice in week 4-week 8.

**Example 7. *In Vitro* Safety Validation of Universal T Cells**

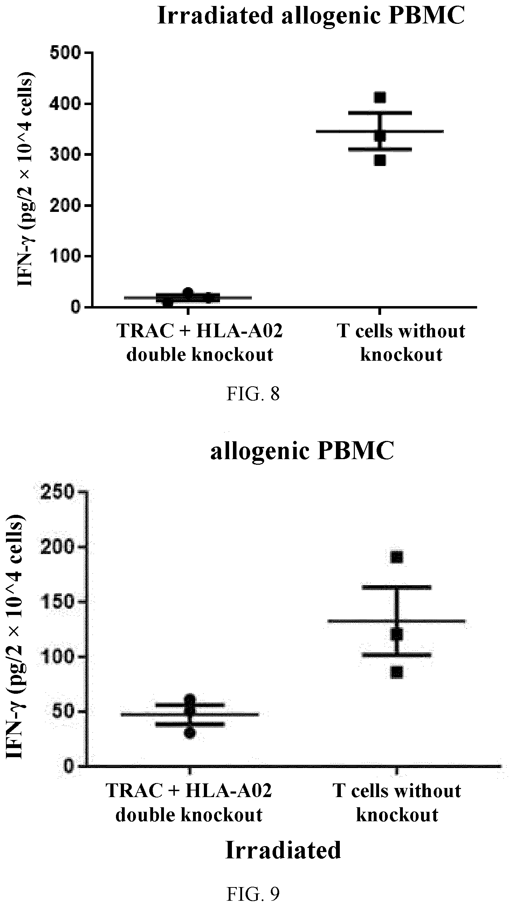

(1) GVHD response: T cells with double knockout of TRAC and HLA-A and T cells without gene knockout were prepared; allogeneic PBMCs were irradiated; 2 groups of prepared cells were stimulated separately, and IFN-γ levels were assayed. Analysis of results (FIG. 8): the T cell group with double knockout of TRAC and HLA-A had very low IFN-γ secretion level, indicating that the knockout of TRAC reduces the GVHD response.

(2) Allogeneic response: after the allogeneic PBMCs were stimulated and irradiated, 2 groups of cells were assayed for the IFN-γ level.

Analysis of results (FIG. 9): the T cell group with double knockout of TRAC and HLA-A had very low IFN-γ secretion level, indicating that the knockout of HLA-A reduces the allogeneic response.

**Example 8. *In Vivo* Safety Validation of Universal T Cells**

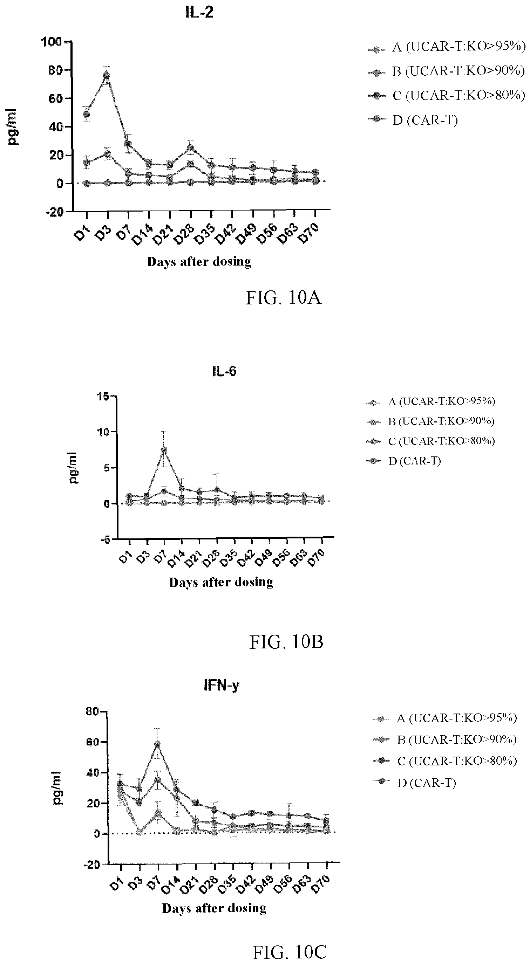

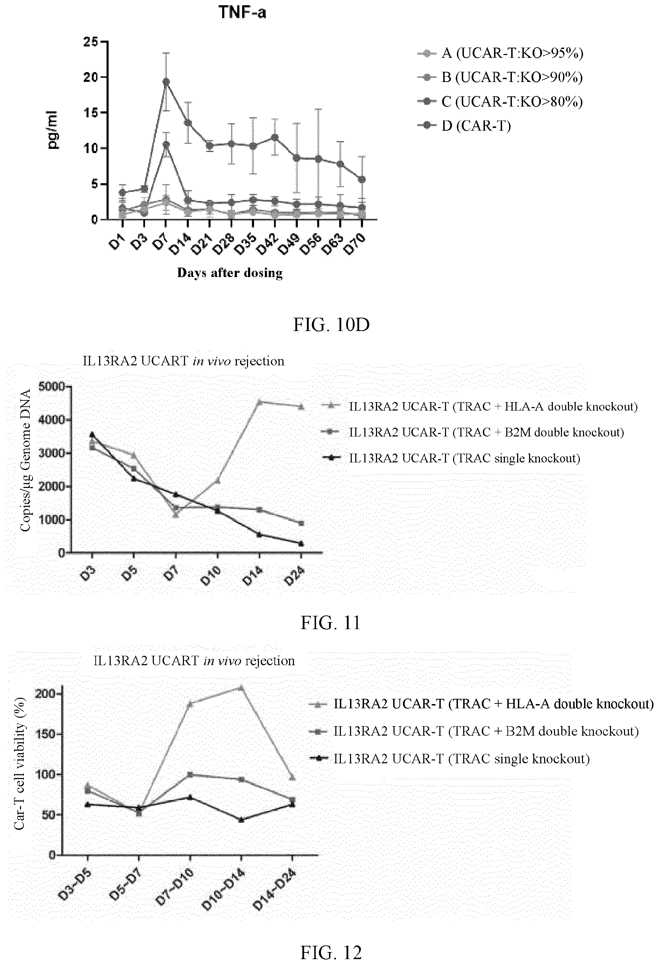

(1) GVHD response: T cells with double knockout of TRAC and HLA-A and T cells without gene knockout were prepared. NSG mice aged 8-10 weeks were injected with $1 \times 10^7$ cells, and according to clinical criteria, such as survival rate, coat texture, skin integrity, and the like, graft versus host response was observed. Assay of cytokines: peripheral blood serum was collected to assay the levels of cytokines such as IL6, IL-2, TNF-α, IFN-y, and the like. The blood collection time points were as follows: 24 h, D3, D7, D14, D28, and 2 M before reinfusion. Analysis of results (FIGs. 10A-10D): three groups of UCAR-T with a knockout efficiency of more than 80% all had very low cytokine secretion level, particularly two groups with a knockout efficiency of more than 90%, indicating that the GVHD response is greatly reduced in the T cell group with double knockout of TRAC and HLA-A.
(2) Allogeneic response: CAR-T cells with double knockout of TRAC and HLA-A were prepared, and $1 \times 10^7$ TCR-HLA-A double-knockout CAR-T cells (irradiated) and $2 \times 10^6$ allogeneic T cells were co-injected into the NSG mice. Control group 1: $5 \times 10^6$ TCR-B2M anti-IL13RA2 UCAR-T cells and $5 \times 10^6$ allogeneic T cells were injected into NSG mice. Control group 2: $5 \times 10^6$ TCR-IL13RA2 UCAR-T cells and $5 \times 10^6$ allogeneic T cells were injected into NSG mice.

Blood was collected at different time points to determine CAR copy number. The changes in the copy number were compared for both groups of CARs. The time points were as follows: D1, D5, D7, D10, and D14.

Conclusion: on D24, the rejection response of the mice in the two control groups was significant, and the copy number was basically undetectable. However, the copy number of the experimental group was still at a relatively high level, indicating that the rejection response is significantly weakened; the survival time of the cells in the experimental group was prolonged in the mice (see FIGs. 11-12).

**Example 9. Safety Analysis of Gene Editing**

T cells with double knockout of TRAC and HLA-A and T cells without gene knockout were prepared, and after the assay of knockout efficiency, the following analyses were performed:

(1) Off-target:

Control group: transgenic CAS9 + ODN tag

Experimental group: transgenic CAS9 + sgRNA (TRAC + HLA-A) + ODN tag

On-target and off-target-WGS (Whole gene sequencing): $1 \times 10^6$ of T cells without gene knockout and T cells with double knockout of TRAC and HLA-A were each collected and sent to Suzhou Genewiz Biological Technology Co., Ltd for assay.

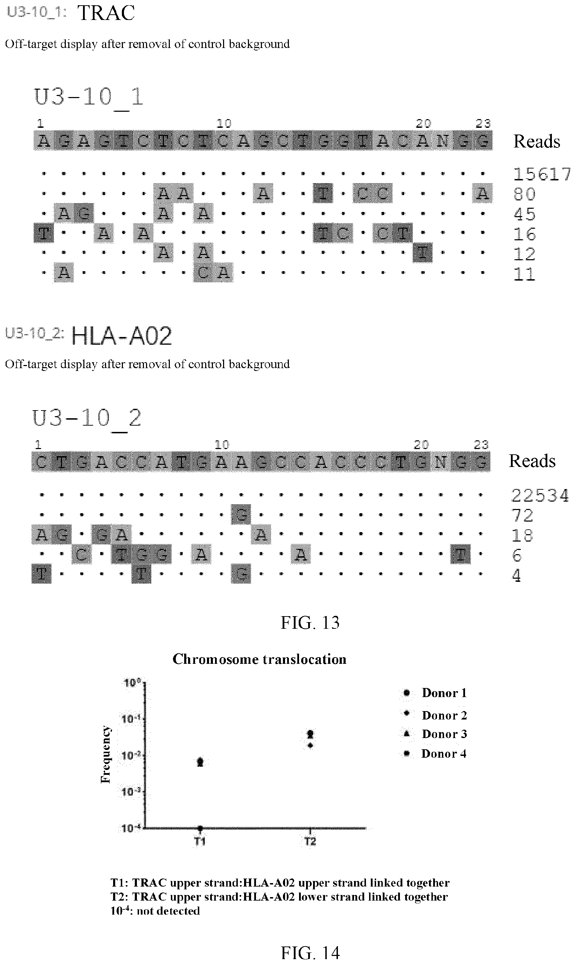

Analysis of results: the off-target rate of the experimental group was very low, and the off-target was mainly concentrated among genes and on introns, so that the effect on the gene function is not great (see FIG. 13).

(2) Chromosome translocation: the qPCR method was used to quantify rearrangements that may occur when editing both TRAC and HLA loci simultaneously. The two translocations were labeled as TRAC:HLA and HLA:TRAC. Positive reference samples in the synthesized template plasmid were evaluated as assay controls. Amplified fragments on both sides of the target region of the HLA genome were used as internal controls. The genome DNA was extracted to perform real-time quantitative PCR, and the gene copy number of the genome DNA was calculated according to the standard curve and Cq value. Analysis of results: the T cells with double knockout (TRAC + HLA-A) were assayed for the occurrence of chromosome translocation on D14 (harvest), and the assay results showed that the assay values for two types of translocation methods were closed to zero, suggesting that there is no rearrangement of the locus (see FIG. 14).

(3) Karyotyping: 1 × 10^6 of T cells without gene knockout and T cells with double knockout of TRAC and HLA-A, which had a confluence of 70%-80%, were each put into two T25 bottles. The bottles were filled with a culture medium, covered with a fully sealed lid, wrapped with a sealing film, and sent to Zhejiang Ruyao Biotech Co., Ltd. for assay.

Analysis of results: compared with the control group, the experimental group was normal in the karyotype (see 15).

(4) Cas9 protein residue: when the cells were prepared, 1 × 10^6 cells at three time points, before knockout, after knockout, and before harvest, were separately collected for lysis, then a protein quantification kit (NOVATEINBIO, Catalog No. NB-E1372PR) was used for quantification, and each group of samples were adjusted to be 2 μg of the same sample loading amount and were assayed by a CRISPR/Cas9 protein ELISA kit according to the instruction. The Cas9 protein in the sample was firmly and stably attached to a test paper hole. The bound Cas9 protein was then recognized using an assay antibody and then developed with a developing agent. The Cas9 ratio was directly proportional to the absorbance, and absolute amounts of Cas9 protein were quantified by comparison to Cas9 control samples.

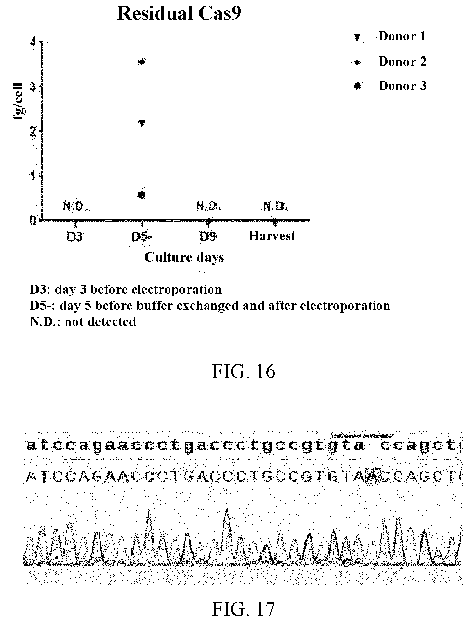

Analysis of results: the T cells with double knockout (TRAC + HLA-A) were assayed for the residue of spCas9 at four time points, i.e., before electroporation (D3), before buffer exchange and after electroporation (D5), D9, and D14 (harvest). The residues were not detected at all the other three time points except for the assay of trace residue before buffer exchange and after electroporation (D5) (see FIG. 16).

## Example 10. Preparation of T Cells with Single-Gene Knockout

RNP complexes were transferred to the activated T cells prepared in Example 2 by electroporation using an electroporation kit (purchased from LONZA, Catalog No. V4XXP-3024). A culture medium (X-VIVO15 culture medium + 10% FBS + IL2 (200 U/mL) + IL7 (10 ng/mL) + IL15 (5 ng/mL)) was pre-heated for 30 min in advance in a well plate. An electroporation buffer was prepared according to a ratio of Nucleofector Solution: Supplement = 82:18. Preparation of RNP complexes: the sgRNA sequence of TRAC was sg9, and the sgRNA sequence of HLA-A was HLA-A02 Sg2, HLA-A02 Sg5, HLA-A11 sg21, or HLA-A11 Rsg2. 20 μg of sgRNA was first added into a PCR tube (without RNase), 10 μg of Cas9 protein (purchased from Thermo, Catalog No. A36499) was then added, and after being mixed gently, the mixture was incubated at room temperature for 12 min. The activated T cells cultured in Example 2 were counted and centrifuged for 8 min at 300 g, and the supernatant was discarded. PBS was added to resuspend the cells, 1E7 cells were pipetted and centrifuged again for 8 min at 300 g, and the supernatant was discarded. The cells were resuspended in 100 μL of the prepared electroporation buffer. The incubated RNP complexes were added to the cell suspension described above. The mixture was gently mixed and gently transferred to an electroporation cuvette. The electroporation cuvette was placed on a Lonza-4D electroporation apparatus and subjected to electroporation using an EO-115 electroporation program. A pre-heated culture medium was added into the electroporation cuvette, and the cells were transferred into the pre-heated culture medium in the well plate by using a matched pipette and then placed in an incubator with 5% $CO_2$ at 37 °C.

## Example 11. Comparison of Gene Knockout Efficiency Assay Methods

(1) Sanger sequencing assay

The cells were counted. $3 \times 10^4$ to $5 \times 10^4$ cells were centrifuged for 5 min at 2000 r/min, and the supernatant was discarded as much as possible. 20 μL of DE lysis buffer was added into each tube. The lysed cells were added into a PCR tube, centrifuged instantaneously, and then placed into a PCR apparatus with the following conditions: 65 °C for 30 min, 4 °C for 30 s, 95 °C for 2 min, and 16 °C for infinite time. PCR was performed by using primer pairs TRAC-For/TRAC-Rev or HLA-A For/HLA-A Rev, and the lysed product was used as a template. The PCR product was sent to Genewiz for Sanger sequencing. After obtaining the Sanger sequencing results, the editing site and the editing efficiency were predicted with the EditR editor on the website: https://moriaritylab.shinyapps.io/editr_v10/.

(2) TA cloning sequencing assay

The PCR product was purified using AxyPrepTM PCR product cleaning kit (purchased from AXYGEN), and then a sticky end was added to the purified PCR product using a kit (DNA A-Tailing Kit, purchased from TaKaRa). The product was ligated to T vector (pMDTM19-T Vector Cloning Kit, purchased from TaKaRa) by a DNA Ligation Kit Ver2.1 (purchased from TaKaRa), and the ligated product was transformed into competent cells (DH5 alpha). The cells were coated on an LB plate containing ampicillin resistance, and the plate was incubated in an incubator at 37 °C for about 12 h. Subsequently, a single colony was picked, and the single colony was sent to Genewiz for sequencing. Knockout efficiency = number of mutated clones/total clones.

(3) Cell counting by flow cytometry

10E5 to 10E8 cells were centrifuged for 5 min at 2000 rpm, and the supernatant was discarded. 100 μL of PBS buffer was added to each tube to resuspend the cells, and 5 μL of anti-human AB TCR-APC antibody (purchased from eBioscience), 5 μL of HLA-A02 monoclonal antibody (BB7.2), APC, and eBioscince™ antibody (purchased from Invitrogen) were added. The mixture was mixed uniformly and incubated at room temperature for 10 min. After being centrifuged for 5 min at 2000 rpm, the cells were washed 2 times with the PBS buffer, resuspended, and assayed by a BD FACSAria flow cytometer. The positive expression rates of TCR and HLA-A02 on the cell surface could be obtained. Knockout efficiency = (A - B)/A × 100%, wherein A was the positive expression rate of the control group; B was the positive expression rate of the knockout group.

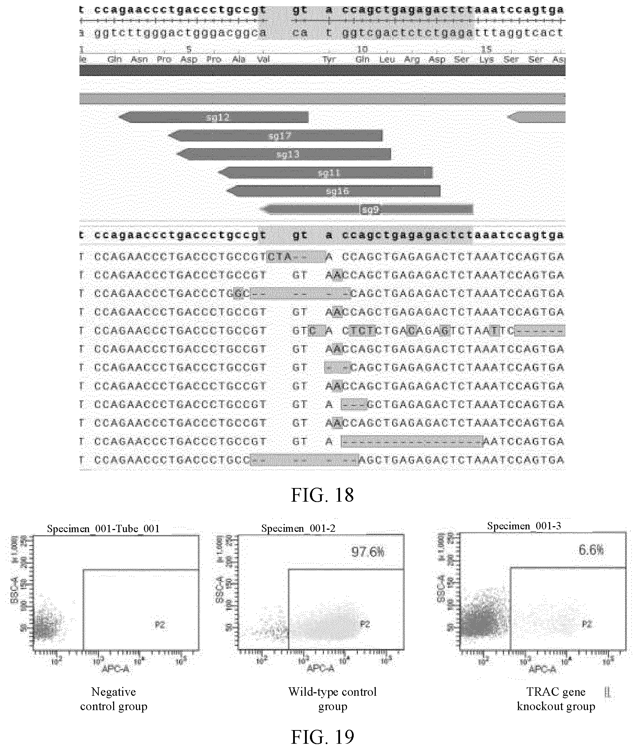

Three assay results of TRAC single-gene knockout are shown in FIGs. 17 to 19, and calculation results of knockout efficiency are shown in Table 1. The three assay methods were basically the same, and the editing efficiency was assayed only by Sanger sequencing in subsequent experiments.

Table 1. Assay method results of gene knockout efficiency

| Target gene | sgRNA | Assay method | Knockout efficiency % |
|---|---|---|---|
| TRAC | Sg9 | Sanger sequencing | 90 |
| | | TA cloning sequencing | 95 |
| | | Flow cytometry | 93 |

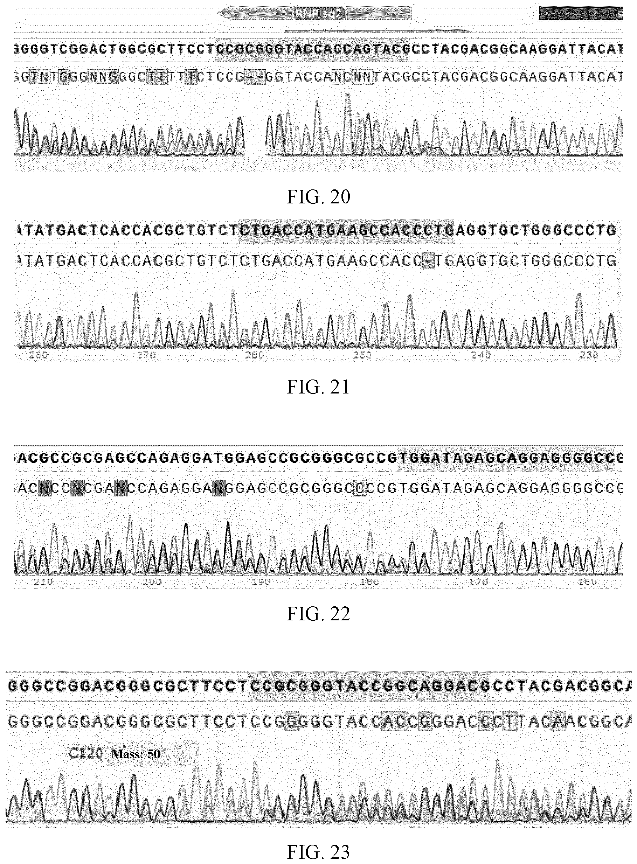

The results of the Sanger sequencing method for HLA-A02 gene editing are shown in FIGs. 20-21, and the editing efficiencies are both 90%; the results of the Sanger sequencing method for HLA-A11 gene editing are shown in FIGs. 22-23.

**Example 12. Preparation of T Cells with Double-Gene Knockout of TRAC Gene and HLA-A Gene**

RNP complexes were transferred to the activated T cells prepared in Example 2 by electroporation using an electroporation kit (purchased from LONZA, Catalog No. V4XXP-3024). A culture medium (X-VIVO15 culture medium + 10% FBS + IL2 (200 U/mL) + IL7 (10 ng/mL) + IL15 (5 ng/mL)) was pre-heated for 30 min in advance in a well plate. An electroporation buffer was prepared according to a ratio of Nucleofector Solution: Supplement = 82:18. Preparation of RNP complexes: 20 μg of TRAC sgRNA (TRAC Sg9) and 20 μg of HLA-A sgRNA (HLA-A02 Sg2, HLA-A02 Sg5, HLA-A11 sg21, or sgRNAs targeting HLA-A*24:02:01, HLA-A*30:01:01:01, HLA-A*33:01:01:01, HLA-A*03:01:01:01, HLA-A*01:01:01:01, or HLA-A*26:01:01:01) were separately added to PCR tubes (without RNA). 10 μg of Cas9 protein (purchased from Thermo, Catalog No. A36499) were added to each tube, and the tubes were mixed gently and incubated at room temperature for 12 min. The activated T cells cultured in Example 2 were counted and centrifuged for 8 min at 300 g, and the supernatant was discarded. PBS was added to resuspend the cells, 1E7 cells were pipetted and centrifuged again for 8 min at 300 g, and the supernatant was discarded. The cells were resuspended in 100 μL of the prepared electroporation buffer. The incubated RNP complexes of TRAC and HLA-A were added to the cell suspension described above. The mixture was gently mixed and gently transferred to an electroporation cuvette. The electroporation cuvette was placed on a Lonza-4D electroporation apparatus and subjected to electroporation using an EO-115 electroporation program. A pre-heated culture medium was added into the electroporation cuvette, and the cells were transferred into the pre-heated culture medium in the well plate by using a matched pipette and then placed in an incubator with 5% $CO_2$ at 37 °C.

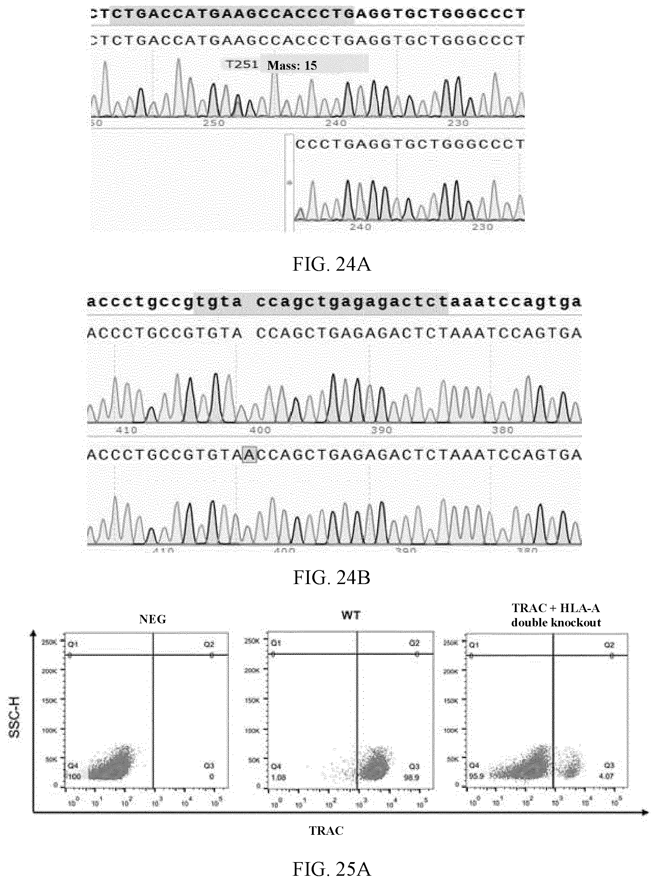

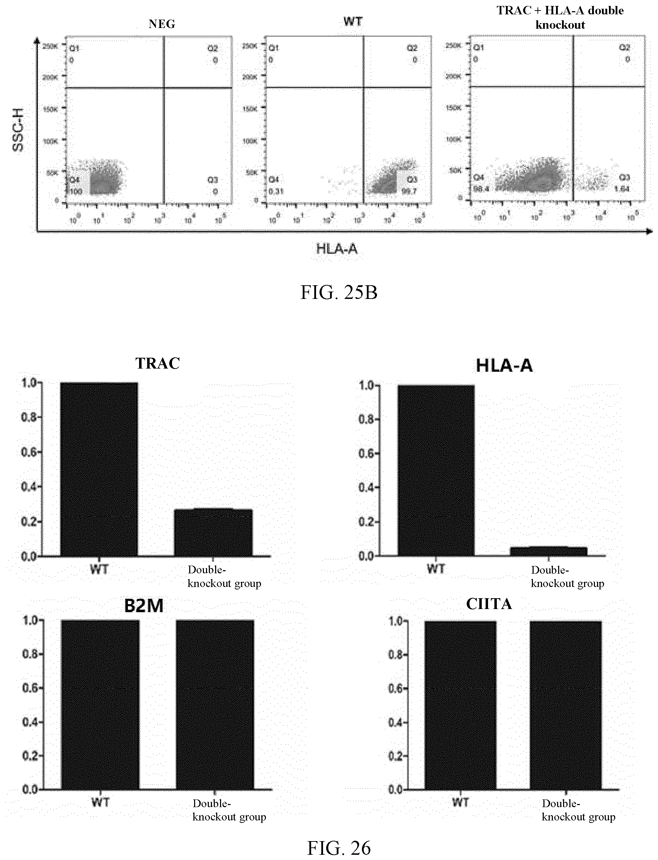

Double-gene knockout efficiency was assayed by sequencing, and TRAC-negative and HLA-A-negative T cells with double-gene knockout efficiency of not less than 80% could be obtained. The results are shown in FIGs. 24-25. FIG. 24A shows the results of HLA-A02 knockout using HLA-A02 Sg5, wherein the upper row shows the results of the control group (i.e., HLA-A02 Sg5 was not used for knockout); the next row shows the results of simultaneous knockout of HLA-A02 and TRAC. FIG. 24B shows the results of TRAC knockout using TRAC Sg9, wherein the upper row shows the results of the control group (i.e., TRAC Sg9 was not used for knockout); the next row shows the results of simultaneous knockout of HLA-A02 and TRAC. FIGs. 25A-25B show the knockout at protein levels after the knockout of HLA-A02 and TRAC, wherein NEG refers to a negative control, WT refers to the absence of any knockouts, and TRAC + HLA-A double knockout refers to the results of simultaneous knockout of HLA-A02 and TRAC.

**Example 13. Differences in Expression of TRAC, HLA-A, B2M, and CIITA Genes in T Cells with Double-Gene Knockout and Corresponding Genes in Corresponding Cells**

(1) The activated T cells prepared in Example 2 were used and divided into two groups. One was used as a control, and the other was prepared into T cells with double-gene knockout of TRAC gene and HLA-A gene according to the method in Example 5. Sanger sequencing was performed according to the method in step (1) of Example 4. The cells with double-gene knockout of TRAC and HLA-A were obtained according to the sequencing result. The prepared T cells with double-gene knockout were incubated with corresponding TRAC and HLA-A antibodies, and a cell strain with double-gene knockout was obtained by flow cytometry sorting or magnetic bead sorting.

(2) A change in mRNA expression level in the T cells with double-gene knockout was assayed compared to the control group. RNA was extracted using an RNA extraction kit (purchased from QIAGEN, Catalog No. 74004), and reverse transcription was performed on RNA using a reverse transcription kit (purchased from Applied Biosystems, Catalog No. 4368814) to obtain cDNA. Quantitative PCR assay was performed using the cDNA as a template.

(3) A change in protein expression level in the T cells with double-gene knockout was assayed compared to the control group. Proteins were extracted using a whole protein extraction reagent (purchased from Thermo Scientific, Catalog No. 87787), and the protein expression level was assayed by Western Blot method or flow cytometry using TRAC antibody (purchased from eBioscience, Catalog No. 17-9986-42), HLA-A antibody (purchased from Merck, Catalog No. 17-9876-41), B2M antibody (purchased from Invitrogen, Catalog No. A15770), and CIITA antibody (purchased from OriGene, Catalog No. CF812200).

The Sanger sequencing assay found that the nucleotide sequence of TRAC and/or HLA-A genes in the T cells with double-gene knockout is changed relative to the control group; the quantitative PCR showed that the mRNA expression level of TRAC and/or HLA-A genes is down-regulated in the T cells with double-gene knockout, but the mRNA expression level of B2M and/or CIITA genes is not down-regulated. FACS and Western Blot results showed that the protein expression amount in the T cells with double-gene knockout is down-regulated, and the protein expression amount of B2M and/or CIITA is not down-regulated.

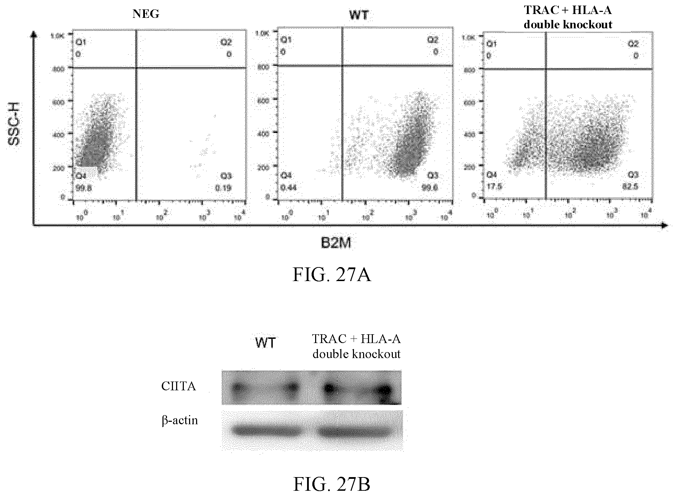

The results are shown in FIGs. 27-28. FIG. 26 shows mRNA level determination of gene expression, and FIG. 26 shows mRNA levels of TRAC, HLA-A, B2M, and CIITA, wherein WT refers to a case without any knockout treatment, and the double-knockout group refers to a result of T cells with double-gene knockout of the TRAC gene and the HLA-A gene. FIG. 27 shows protein level determination of gene expression, wherein FIGs. 27A-27B show protein expression levels of B2M and CIITA, respectively; NEG refers to a negative control, WT refers to a case without any knockout treatment, and TRAC + HLA-A double knockout refers to a result of T cells with double-gene knockout of the TRAC gene and the HLA-A gene.

**Example 14. Preparation of T Cells with Triple-Gene Knockout of TRAC Gene, HLA-A/B2M Gene, and CIITA Gene and Verification of Changes in Expression of Respective Three Genes**

(1) A control group, cells with triple-gene knockout of TRAC gene, HLA-A gene, and CIITA gene, and cells with triple-gene knockout of TRAC gene, B2M gene, and CIITA gene were prepared according to the method in step (1) of Example 15.

(2) Changes in protein expression levels were assayed by FACS and Western Blot methods according to the method in step (3) of Example 15.

The protein expression levels of TRAC, HLA-A, and CIITA genes in the T cells with triple-gene knockout of TRAC, HLA-A, and CIITA were down-regulated relative to the cells in the control group; the protein expression levels of TRAC, HLA-A, and CIITA genes in the T cells with triple-gene knockout of TRAC, B2M, and CIITA were down-regulated relative to the cells in the control group.

(3) The knockout efficiencies of the cells with double-gene knockout in Example 13 and the two cells with triple-gene knockout in this example were assayed by flow cytometry using TRAC antibody (purchased from eBioscience, Catalog No. 17-9986-42), HLA-A antibody (purchased from Merck, Catalog No. 17-9876-41), and B2M antibody (purchased from Invitrogen, Catalog No. A15770), and the results showed that the efficiency of multiple gene knockout was achieved simultaneously at the single cell level, and that the efficiency of the double-gene knockout was significantly higher than that of the triple-gene knockout.

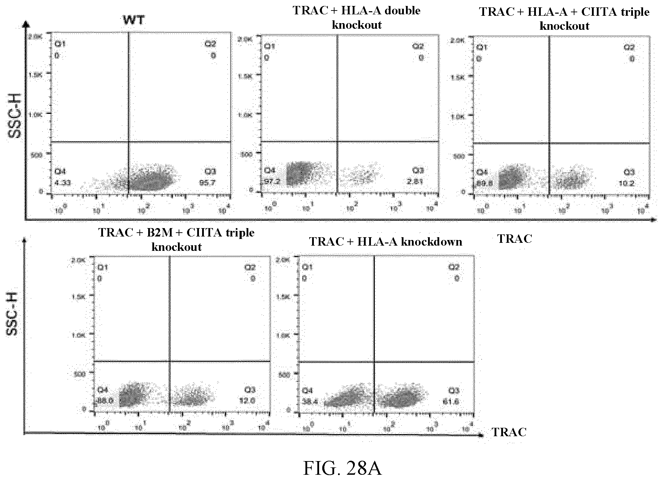

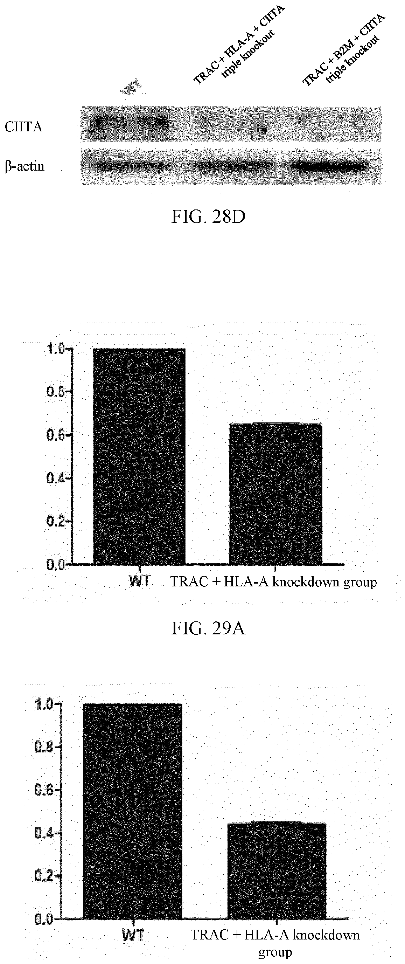

The results are shown in FIGs. 28A-28D. FIGs. 28A-28C show the knockout of TRAC, HLA-A and B2M at protein levels in sequence, wherein WT refers to a case without any knockout treatment, TRAC + HLA-A double knockout refers to the result of T cells with double-gene knockout of TRAC and HLA-A genes; TRAC + HLA-A + CIITA triple knockout refers to the result of T cells with triple-gene knockout of TRAC, HLA-A, and CIITA; TRAC + B2M + CIITA triple knockout refers to the result of T cells with triple-gene knockout of B2M, CIITA, and TRAC; and TRAC + HLA-A knockdown refers to the result of T cells with knockdown of TRAC and HLA-A genes prepared in Example 18. FIG. 28D shows the knockout of CIITA at the protein level.

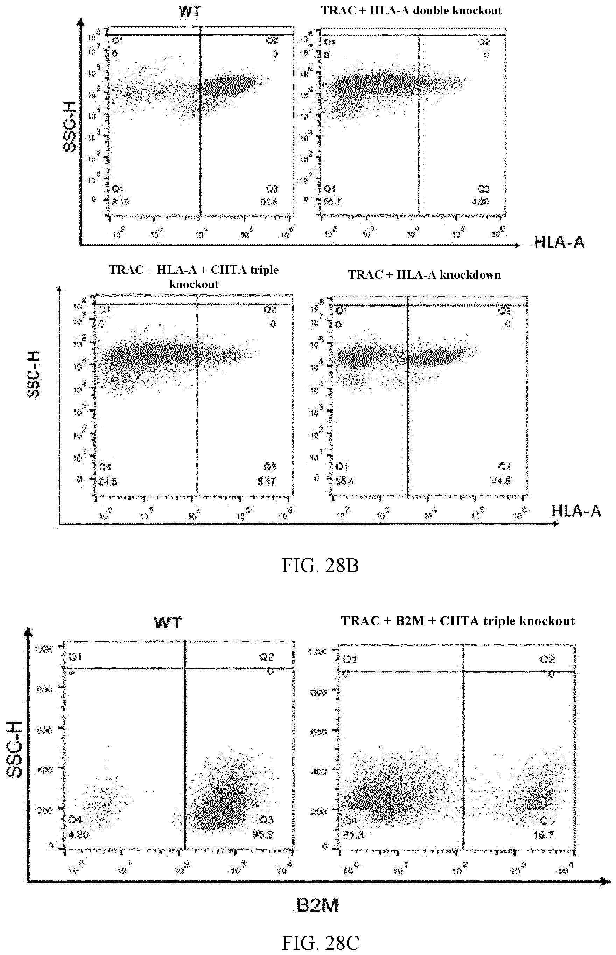

The results in FIG. 28 showed that the protein levels of TRAC, HLA-A, CIITA, and B2M are down-regulated compared to the WT control group. Meanwhile, compared to TRAC + HLA-A + CIITA triple knockout or TRAC + B2M + CIITA triple knockout, the knockout efficiency of TRAC + HLA-A double knockout is higher.

## Example 15. Design of Antisense RNA Sequence

The transcription RNA sequences of the corresponding genes (TRAC gene and HLA-A gene) were obtained by the database https://www.ncbi.nlm.nih.gov/ or www.ensembl.org/, and siRNA was designed with reference to the following principles:

Sequences of 50-100 nucleotides downstream of the start codon and 100 nucleotides upstream of the stop codon were avoided as much as possible; sequences with less than 30 nucleotides in length were selected; 4 or more consecutive identical bases were avoided; intron regions were avoided; repetitive sequences were avoided; single nucleotide polymorphism (SNP) sites were avoided; sequences had a GC content ranging 30% to 60%; sequence patterns AA (N$_{19}$), NA (N$_{21}$), or NAR (N$_{17}$) YNN were preferably selected, wherein A was adenosine, T was thymidine, R was adenosine or guanosine (purines), Y was thymidine or cytidine (pyrimidines), N was adenosine, thymidine, guanosine, or cytidine; homology comparison and analysis were performed on the selected sequences to avoid significant homology of the antisense RNA to other genes or sequences and the resulting off-target effects. The homology analysis was performed using NCBI Blast tool: Nucleotide-nucleotide BLAST (blastn), UCSC Blat tool, or Ensembl Blast.

The antisense RNA sequences obtained by design included HLA-A-homo-551, HLA-A-homo-NEG, TRAC-homo-375, and TRAC-homo-NEG.

## Example 16. Preparation of T Cells with Knockdown of TRAC Gene and HLA-A Gene

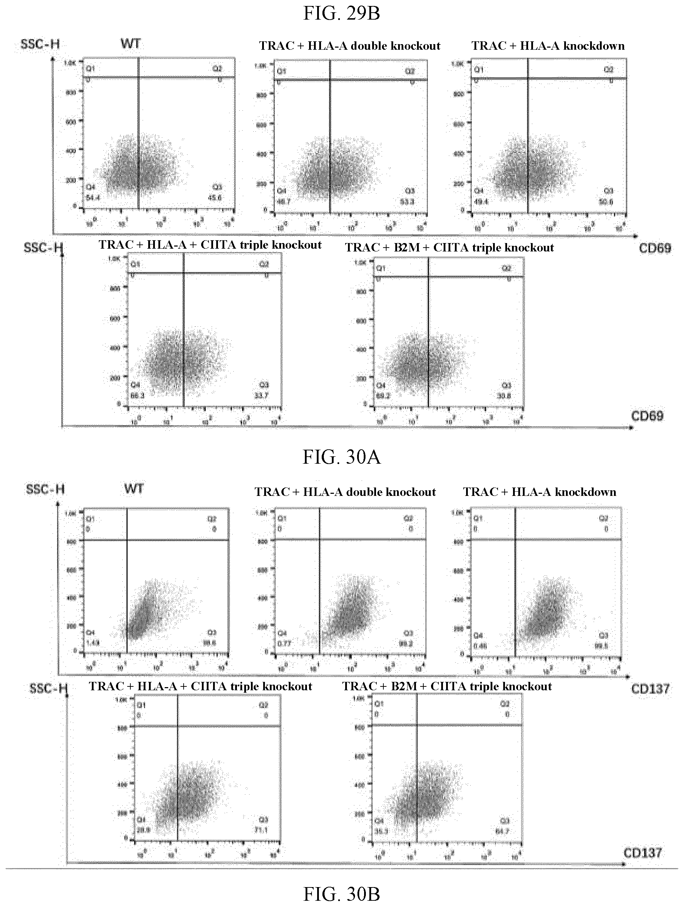

Double-gene knockdown was performed using the antisense RNA designed in Example 17. A lentivirus comprising the antisense RNA sequences of the TRAC gene and HLA-A gene was prepared by a company (Genepharma). CD3$^+$ T cells were prepared according to the method in Example 2 (D0) and activated with CD3/CD28 antibody magnetic beads. The lentivirus carrying the antisense RNA sequences of the TRAC gene and HLA-A gene was transfected into the activated T cells (D1). On D2, the lentiviral vector was washed off, and the cells were continued to be cultured until D5. The T cells cultured until D5 were collected, and gene knockdown efficiency was assayed by quantitative PCR or Western Blot, etc. The obtained T cells were labeled with corresponding TRAC and HLA-A antibodies, and the T cells with knockdown of the TRAC gene and HLA-A gene could be obtained by flow cytometry sorting or magnetic bead sorting methods. The results showed that both the mRNA and protein expression levels of TRAC and HLA-A were down-regulated in the TRAC and HLA-A gene-knockdown group. FIGs. 29A-29B show the knockout of TRAC and HLA-A at mRNA levels in sequence, wherein WT refers to a case without any knockout treatment, and TRAC + HLA-A double knockout refers to the result of T cells with double-gene knockout of the TRAC gene and the HLA-A gene. Among these, the knockout level of TRAC and HLA-A at protein levels can be found in the results shown in FIG. 28.

## Example 17. Difference in Activities of Different T Cells

The T cells without gene knockout, with double-gene knockout, with triple-gene knockout, and with double-gene knockdown in Examples 2, 14, 16, and 18 were prepared, and several T cell activities were compared. Each group of cells was counted and seeded in 24-well plates with $1 \times 10^6$ cells, and PHA (0.3 $\mu$g/mL) (ionomycin+) or 5 ng/mL PMA and 50 ng/mL ionomycin were added to the cells per well. The cells were cultured for another 5 h, and then the activation state of the cells was assayed using CD69 (early activated) (purchased from BD Biosciences, Catalog No. FN50) and CD137 (later stage) (purchased from BD Biosciences, Catalog No. 4B4-1) antibodies by flow cytometry. The results showed that the activities of the T cells with double-gene knockout and double-gene knockdown were superior to that of the T cells with triple-gene knockout.

The expression of CD69 and CD137 at protein levels is shown in FIGs. 30A-30B, respectively, wherein WT refers to a case without any knockout treatment, TRAC + HLA-A double knockout refers to the result of T cells with double-gene knockout of TRAC and HLA-A genes; TRAC + HLA-A + CIITA triple knockout refers to the result of T cells with triple-gene knockout of TRAC, HLA-A, and CIITA; TRAC + B2M + CIITA triple knockout refers to the result of T cells with triple-gene knockout of B2M, CIITA, and TRAC; and TRAC + HLA-A knockdown refers to the result of T cells with knockdown of TRAC and HLA-A genes prepared in Example 18.

**Example 18. Difference in Reactivity of Different T Cells to Allogeneic NK Cells**

CFSE (Invitrogen, C34554) labeling was performed on the T cells without gene knockout, with double-gene knockout, with triple-gene knockout, and with double-gene knockdown in Examples 2, 14, 16, and 18. The cells were counted, and $1 \times 10^6$ cells were collected and co-cultured with NK cells (NK92MI) at a ratio of 1:1. After 24 h, the co-cultured cells were collected from each group, and the ratio of CFSE-positive cells in the mixed cells was determined by flow cytometry.

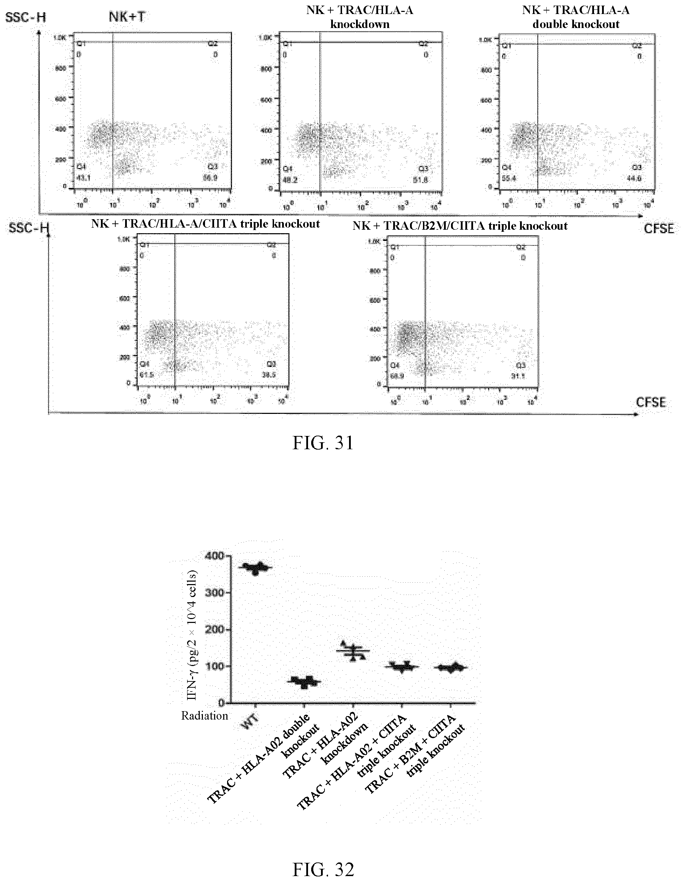

The results showed that the killing toxicity of the NK cells to the T cells with double-gene knockout and double-gene knockdown was lower than that of the T cells with triple-gene knockout. The results are shown in FIG. 31, wherein NK + T refers to a case where the NK cells were co-cultured with the T cells without any knockout treatment; NK + TRAC + HLA-A knockdown refers to a case where the NK cells were co-cultured with the resulting T cells with knockdown of the TRAC gene and HLA-A gene prepared in Example 18; NK + TRAC + HLA-A double knockout refers to a case where the NK cells were co-cultured with the T cells with double-gene knockout of the TRAC gene and HLA-A gene; NK + TRAC + HLA-A + CIITA triple knockout refers to a case where the NK cells were co-cultured with the T cells with triple-gene knockout of TRAC, HLA-A, and CIITA; NK + TRAC + B2M + CIITA triple knockout refers to a case where the NK cells were co-cultured with the T cells with triple-gene knockout of B2M, CIITA, and TRAC.

**Example 19. Difference in Allogeneic Immune Rejection Response of Different T Cells**

Peripheral blood originating from donor 1 was used to prepare T cells without gene knockout, with double-gene knockout, with triple-gene knockout, and with double-gene knockdown in Examples 2, 14, 16, and 18. Peripheral blood originating from donor 2 was used to prepare CD3+ T cells. Each group of cells prepared from the peripheral blood of donor 1 was mixed with the CD3+ T cells prepared from the peripheral blood of donor 2 according to Example 2 in an equal proportion. After 24 h, the expression level of IFN-γ in the cell mixture system was assayed. The results showed that the expression level of IFN-γ in the T-cell group with double-gene knockout was lower than that in the T-cell group with triple-gene knockout.

The results are shown in FIG. 32. WT refers to a case without any knockout treatment, TRAC + HLA-A double knockout refers to the result of T cells with double-gene knockout of TRAC and HLA-A genes; TRAC + HLA-A + CIITA triple knockout refers to the result of T cells with triple-gene knockout of TRAC, HLA-A, and CIITA; TRAC + B2M + CIITA triple knockout refers to the result of T cells with triple-gene knockout of B2M, CIITA, and TRAC; and TRAC + HLA-A knockdown refers to the result of T cells with knockdown of TRAC and HLA-A genes prepared in Example 16.

**Example 20. Preparation of CAR-T Cells with Double-Gene Knockout of TRAC Gene and HLA-A Gene, CAR-T Cells with Triple-Gene Knockout of TRAC Gene, HLA-A Gene, and CIITA Gene, and CAR-T Cells with Knockout of TRAC Gene, B2M Gene, and CIITA Gene**

(1) CD3+ T cells were obtained according to the method in Example 2 (D0) and activated with CD3/CD28 antibody magnetic beads. After activation, lentiviral vectors (lentiviruses comprising CD19-CAR, CD20-CAR, or BCMA-CAR) were transfected on D1, the lentiviral vectors were washed off on D2, CAR-positive T cells were sorted on D3, and the cells were continued to be cultured until D5.

(2) The CAR-T cells obtained on D5 were used as starting cells, and cells with double-gene knockout of the TRAC gene and HLA-A gene, CAR-T cells with triple-gene knockout of the TRAC gene, HLA-A gene, and CIITA gene, and CAR-T cells with triple-gene knockout of the TRAC gene, B2M gene, and CIITA gene were prepared according to the methods in Examples 14 and 16, respectively.

(3) The CAR-T cells with double-gene knockout and triple-gene knockout described above could be obtained by flow cytometry assay, wherein the yield of the CAR-T cells with double-gene knockout was higher than that of the CAR-T cells with triple-gene knockout.

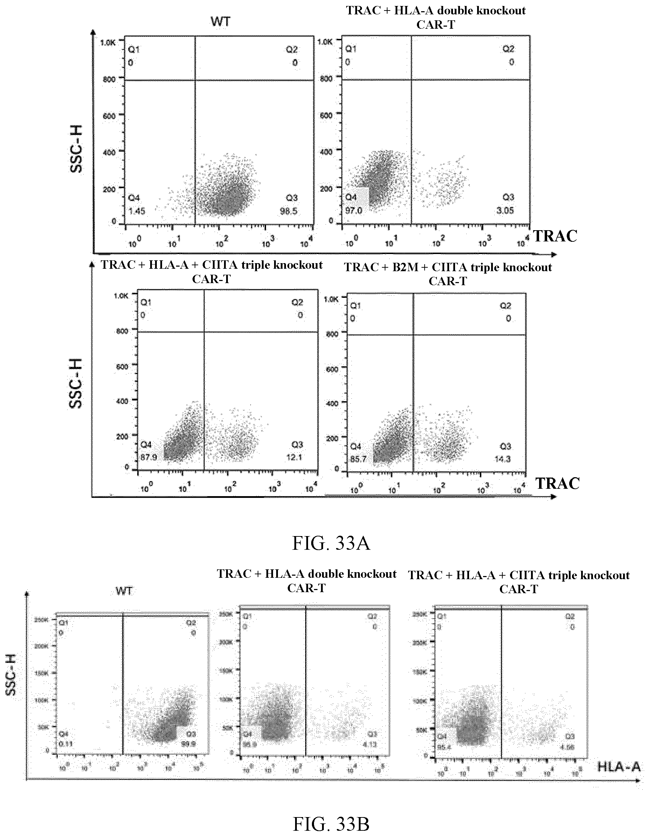

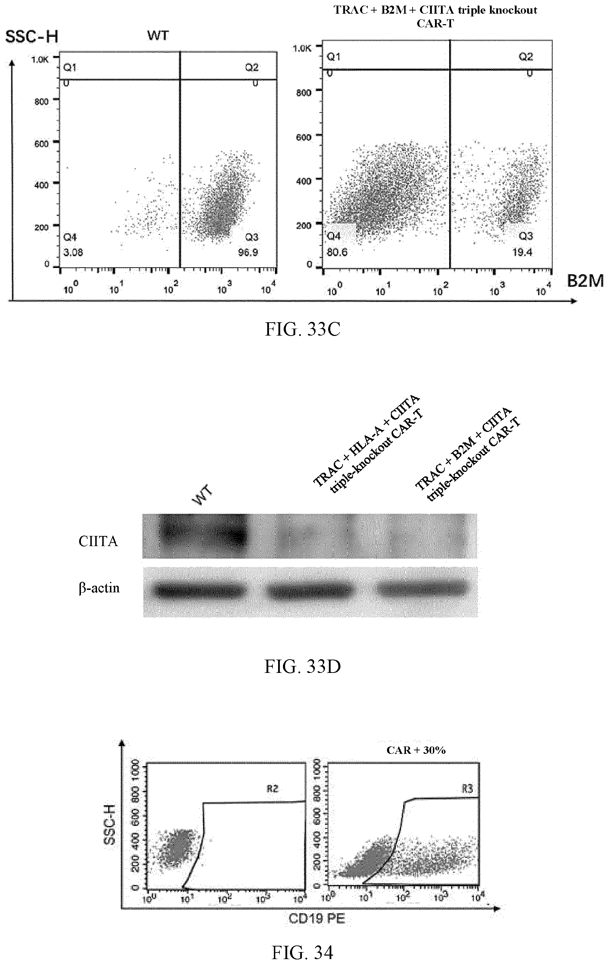

The results are shown in FIGs. 33A-33D. FIGs. 33A-33C show the knockout of TRAC, HLA-A and B2M at protein levels in sequence. FIG. 33D shows the knockout of CIITA at the protein level, wherein WT refers to a case without any knockout treatment, TRAC + HLA-A double knockout refers to the result of CAR-T cells with double-gene knockout of TRAC and HLA-A genes; TRAC + HLA-A + CIITA triple knockout refers to the result of CAR-T cells with triple-gene knockout of TRAC, HLA-A, and CIITA; TRAC + B2M + CIITA triple knockout refers to the result of CAR-T cells with triple-gene knockout of B2M, CIITA, and TRAC.

Among these, the transfection efficiency of CD19CAR is shown in FIG. 34, wherein CAR30%+ represented the transfection efficiency of CD19 CAR.

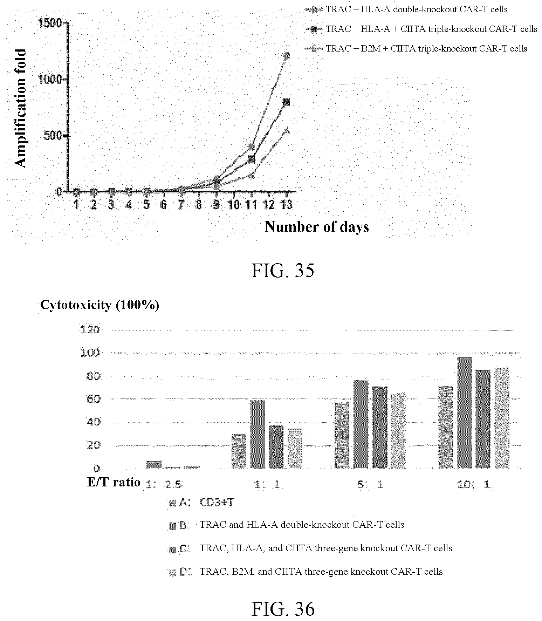

FIG. 35 shows the amplification fold of different cells, wherein the CAR-T cells with double-gene knockout of the TRAC gene and HLA-A gene had the highest amplification fold.

**Example 21. Anti-Tumor Effect of CAR-T Cells with Double-Gene Knockout of TRAC Gene and HLA-A Gene**

The CAR-T cells with double knockout of the TRAC gene and HLA-A gene (targeting CD19, CD20, or BCMA) were prepared in Example 23. Target cells expressing the luciferase gene (target gene-positive leukemia or lymphoma cell lines, such as Raji, Jurkat, MM1S, and the like) were seeded to a well plate. The CAR-T cells with double-gene knockout, CAR-T cells with triple-gene knockout, or T cells without gene knockout were added at different effector-to-target ratios (1:2.5, 1:1, 5:1, and 10:1), respectively. After 24 h of co-culture, the cells were transferred to an assay well plate, luciferase substrate was added, and fluorescence value was detected by a microplate reader. Killing efficiency = 1 - fluorescence value of T cells co-cultured with target cells/fluorescence value of target cells cultured alone.

The results showed that the CAR-T cells with double knockout of the TRAC gene and HLA-A gene had a significant killing effect on tumor cells.

FIG. 36 shows the killing effect on CD19 target cell Raji-Luciferase, wherein the CAR-T cells with double knockout of the TRAC gene and HLA-A gene exhibit the most significant killing effect. At each E/T ratio, the results corresponding to notes A-D were shown from left to right.

**Example 22. Anti-Tumor Effect of CAR-T Cells with Double-Gene Knockout of TRAC Gene and HLA-A Gene**

NSG mice were injected with tumor cells intravenously. After the tumor was successfully established, the CAR-T cells with double-gene knockout of the TRAC gene and HLA-A gene, CAR-T cells with triple-gene knockout, and T cells without gene knockout were reinfused to the mice. The tumor volume of the mice was monitored.

The mice to which the CAR-T cells with double-gene knockout were reinfused exhibited a significantly slower growth rate of the tumor.

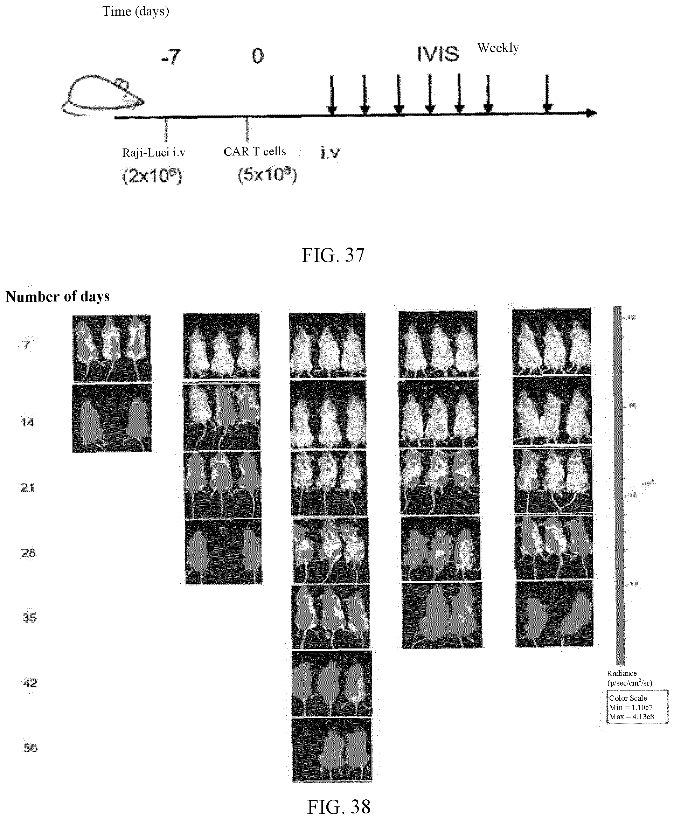

The results are shown in FIGs. 37-38. wherein FIG. 37 shows the administration mode in mice, i.v. representing intravenous injection, and CAR-T cells representing CAR-T cells with double-gene knockout and CAR-T cells with triple-gene knockout expressing CD19 CAR. FIG. 38 shows the tumor volume in the mice after the CAR-T cells were administered, wherein FIG. 38 shows, from left column to right column, the tumor volume in the mice after normal saline, unmodified T cells, CD19 CAR-T cells with double-gene knockout of the TRAC gene and HLA-A gene, CD19 CAR-T cells with triple-gene knockout of TRAC, HLA-A, and CIITA, and CD19 CAR-T cells with triple-gene knockout of B2M, CIITA, and TRAC were separately administered in sequence. The results showed that the mice to which the CAR-T cells with double-gene knockout of the TRAC gene and HLA-A gene were reinfused exhibited a significantly slower growth rate of the tumor.

In summary:

1. The present application firstly constructs IL13Rα2-UCAR-T cells with high efficient double knockout of TCR and HLA-A, achieves a safe shelf ready-to-use therapeutic agent, improves the anti-tumor effect, and is used for treating tumors such as cerebral glioma.

2. The present application provides a lentiviral expression vector. pCDH-CMV-MCS-EF1-copGFP is used as a backbone, and an ampicillin resistance gene β-lactamase on the vector is replaced with aminoglycoside phosphotransferase derived from Tn5 to enable the vector to have kanamycin resistance; the CMV promoter and its adjacent downstream multiple cloning site, which are potentially threatening in *in vivo* applications, are deleted; the copGFP gene that is started to express by the EF1 promoter in the original vector is deleted; a SalI enzyme digestion site is retained, and a SmaI enzyme digestion site is added to the 5' end of SalI for vector construction to form a final target vector.

3. The present application optimizes the protein-RNA complex electrotransfection technology. More than 90% of double-gene knockout efficiency in primary T cells is obtained.

4. In the present application, the donor source is based on HLA-B homozygotes that occur frequently in the population, and one of the alleles of HLA-B in the patient is consistent with the homozygotes in the donor, so that cells from these donors can cover a great number of patient populations, and the rejection response caused by HLA-B can be reduced.

5. According to the present application, HLA-A molecules highly related to rejection are screened out for knockout, and other HLA-I molecules are retained, so that the rejection of allogeneic cells is reduced, and the complete knockout of HLA molecules and elimination of HLA molecules by NK cells are avoided, thereby greatly prolonging the half-life of allogeneic CAR-T cells *in vivo.*

**Claims**

1. An immune effector cell, wherein the functions of a T cell antigen receptor (TCR) and major histocompatibility complexes (MHCI, MHCII) in the immune effector cell are inhibited in a cell, and the immune effector cell comprises a chimeric antigen receptor (CAR) targeting IL13Rα2.

2. The immune effector cell according to claim 1, wherein the chimeric antigen receptor (CAR) targeting IL13Rα2 comprises a targeting moiety comprising at least one complementarity-determining region (CDR) of an antibody heavy chain variable region (VH), and the VH comprises an amino acid sequence set forth in SEQ ID NO: 18.

3. The immune effector cell according to claim 2, wherein the VH comprises an amino acid sequence set forth in SEQ ID NO: 16 or SEQ ID NO: 17.

4. The immune effector cell according to any one of claims 1-3, wherein the chimeric antigen receptor (CAR) targeting IL13Rα2 comprises a targeting moiety comprising a VH, the VH comprises a heavy chain complementarity-determining region 1 (HCDR1), a heavy chain complementarity-determining region 2 (HCDR2), and a heavy chain complementarity-determining region 3 (HCDR3), and the HCDR3 comprises an amino acid sequence set forth in SEQ ID NO: 3.

5. The immune effector cell according to claim 4, wherein the HCDR2 comprises an amino acid sequence set forth in SEQ ID NO: 2.

6. The immune effector cell according to any one of claims 4-5, wherein the HCDR1 comprises an amino acid sequence set forth in SEQ ID NO: 1.

7. The immune effector cell according to any one of claims 4-6, wherein the VH comprises: the HCDR1 comprising the amino acid sequence set forth in SEQ ID NO: 1, the HCDR2 comprising the amino acid sequence set forth in SEQ ID NO: 2, and the HCDR3 comprising the amino acid sequence set forth in SEQ ID NO: 3.

8. The immune effector cell according to any one of claims 4-7, wherein the VH comprises a heavy chain framework region 1 (HFR1), a heavy chain framework region 2 (HFR2), a heavy chain framework region 3 (HFR3), and a heavy chain framework region 4 (HFR4), and the HFR1 comprises an amino acid sequence set forth in SEQ ID NO: 4.

9. The immune effector cell according to claim 8, wherein the HFR1 comprises an amino acid sequence set forth in SEQ ID NO: 8 or SEQ ID NO: 12.

10. The immune effector cell according to any one of claims 8-9, wherein the HFR2 comprises an amino acid sequence set forth in SEQ ID NO: 5.

11. The immune effector cell according to any one of claims 8-10, wherein the HFR2 comprises an amino acid sequence set forth in SEQ ID NO: 9 or SEQ ID NO: 13.

12. The immune effector cell according to any one of claims 8-11, wherein the HFR3 comprises an amino acid sequence set forth in SEQ ID NO: 6.

13. The immune effector cell according to any one of claims 8-12, wherein the HFR3 comprises an amino acid sequence set forth in SEQ ID NO: 10 or SEQ ID NO: 14.

14. The immune effector cell according to any one of claims 8-13, wherein the HFR4 comprises an amino acid sequence set forth in SEQ ID NO: 7.

15. The immune effector cell according to any one of claims 8-14, wherein the HFR4 comprises an amino acid sequence set forth in SEQ ID NO: 11 or SEQ ID NO: 15.

16. The immune effector cell according to any one of claims 4-15, wherein the VH comprises HFR1, HFR2, HFR3, and HFR4, and the HFR1, HFR2, HFR3, and HFR4 are selected from

i) the HFR1 comprising the amino acid sequence set forth in SEQ ID NO: 8, the HFR2 comprising the amino acid sequence set forth in SEQ ID NO: 9, the HFR3 comprising the amino acid sequence set forth in SEQ ID NO: 10, and the HFR4 comprising the amino acid sequence set forth in SEQ ID NO: 11; and
ii) the HFR1 comprising the amino acid sequence set forth in SEQ ID NO: 12, the HFR2 comprising the amino acid sequence set forth in SEQ ID NO: 13, the HFR3 comprising the amino acid sequence set forth in SEQ ID NO: 14, and the HFR4 comprising the amino acid sequence set forth in SEQ ID NO: 15.

17. The immune effector cell according to any one of claims 4-16, wherein the VH comprises the amino acid sequence set forth in SEQ ID NO: 18.

18. The immune effector cell according to any one of claims 4-17, wherein the VH comprises the amino acid sequence set forth in SEQ ID NO: 16 or SEQ ID NO: 17.

19. The immune effector cell according to any one of claims 2-19, wherein the targeting moiety further comprises at least one CDR of an antibody light chain variable region (VL), and the VL comprises an amino acid sequence set forth in SEQ ID NO: 36.

20. The immune effector cell according to claim 19, wherein the VL comprises an amino acid sequence set forth in SEQ ID NO: 34 or SEQ ID NO: 35.

21. The immune effector cell according to claim 20, wherein the targeting moiety comprises at least one CDR of the VH and at least one CDR of the VL, and the VH and the VL are selected from:

i) the VH comprising the amino acid sequence set forth in SEQ ID NO: 16, and the VL comprising the amino acid sequence set forth in SEQ ID NO: 34; and
ii) the VH comprising the amino acid sequence set forth in SEQ ID NO: 17, and the VL comprising the amino acid sequence set forth in SEQ ID NO: 35.

22. The immune effector cell according to any one of claims 4-21, wherein the chimeric antigen receptor (CAR) targeting IL13Rα2 comprises a targeting moiety comprising a VL, the VL comprises a light chain complementarity-determining region 1 (LCDR1), a light chain complementarity-determining region 2 (LCDR2), and a light chain complementarity-determining region 3 (LCDR3), and the LCDR1 comprises an amino acid sequence set forth in SEQ ID NO: 19.

23. The immune effector cell according to any one of claims 4-22, wherein the LCDR2 comprises an amino acid sequence set forth in SEQ ID NO: 20.

24. The immune effector cell according to any one of claims 4-23, wherein the LCDR3 comprises an amino acid sequence set forth in SEQ ID NO: 21.

25. The immune effector cell according to any one of claims 4-24, wherein the VL comprises: the LCDR1 comprising the amino acid sequence set forth in SEQ ID NO: 19, the LCDR2 comprising the amino acid sequence set forth in SEQ ID NO: 20, and the LCDR3 comprising the amino acid sequence set forth in SEQ ID NO: 21.

26. The immune effector cell according to any one of claims 4-25, wherein the VL comprises a light chain framework region 1 (LFR1), a light chain framework region 2 (LFR2), a light chain framework region 3 (LFR3), and a light chain framework region 4 (LFR4), and the LFR1 comprises an amino acid sequence set forth in SEQ ID NO: 22.

27. The immune effector cell according to claim 26, wherein the LFR1 comprises an amino acid sequence set forth in SEQ ID NO: 26 or SEQ ID NO: 30.

28. The immune effector cell according to any one of claims 26-27, wherein the LFR2 comprises an amino acid sequence set forth in SEQ ID NO: 23.

29. The immune effector cell according to any one of claims 26-28, wherein the LFR2 comprises an amino acid sequence set forth in SEQ ID NO: 27 or SEQ ID NO: 31.

30. The immune effector cell according to any one of claims 26-29, wherein the LFR3 comprises an amino acid sequence set forth in SEQ ID NO: 24.

31. The immune effector cell according to any one of claims 26-30, wherein the LFR3 comprises an amino acid sequence set forth in SEQ ID NO: 28 or SEQ ID NO: 32.

32. The immune effector cell according to any one of claims 26-31, wherein the LFR4 comprises an amino acid sequence set forth in SEQ ID NO: 25.

33. The immune effector cell according to any one of claims 26-32, wherein the LFR4 comprises an amino acid sequence set forth in SEQ ID NO: 29 or SEQ ID NO: 33.

34. The immune effector cell according to any one of claims 22-33, wherein the VL comprises LFR1, LFR2, LFR3, and LFR4, and the LFR1, LFR2, LFR3, and LFR4 are selected from

i) the LFR1 comprising the amino acid sequence set forth in SEQ ID NO: 26, the LFR2 comprising the amino acid sequence set forth in SEQ ID NO: 27, the LFR3 comprising the amino acid sequence set forth in SEQ ID NO: 28, and the LFR4 comprising the amino acid sequence set forth in SEQ ID NO: 29; and
ii) the LFR1 comprising the amino acid sequence set forth in SEQ ID NO: 30, the LFR2 comprising the amino acid sequence set forth in SEQ ID NO: 31, the LFR3 comprising the amino acid sequence set forth in SEQ ID NO: 32, and the LFR4 comprising the amino acid sequence set forth in SEQ ID NO: 33.

35. The immune effector cell according to any one of claims 22-34, wherein the VL comprises the amino acid sequence set forth in SEQ ID NO: 36.

36. The immune effector cell according to any one of claims 22-35, wherein the VL comprises the amino acid sequence set forth in SEQ ID NO: 34 or SEQ ID NO: 35.

37. The immune effector cell according to any one of claims 2-36, wherein the targeting moiety comprises the VH comprising the amino acid sequence set forth in SEQ ID NO: 18 and the VL comprising the amino acid sequence set forth in SEQ ID NO: 36.

38. The immune effector cell according to claim 37, wherein the VH and the VL are selected from:

i) the VH comprising the amino acid sequence set forth in SEQ ID NO: 16, and the VL comprising the amino acid sequence set forth in SEQ ID NO: 34; and
ii) the VH comprising the amino acid sequence set forth in SEQ ID NO: 17, and the VL comprising the amino acid sequence set forth in SEQ ID NO: 35.

39. The immune effector cell according to any one of claims 2-38, wherein the targeting moiety comprises a full-length antibody, a Fab, a single-chain variable fragment (scFv), or a single-domain antibody (VHH).

40. The immune effector cell according to any one of claims 2-39, wherein the targeting moiety comprises the scFv.

41. The immune effector cell according to any one of claims 2-40, wherein the targeting moiety comprises an amino acid sequence set forth in SEQ ID NO: 41 or SEQ ID NO: 44.

42. The immune effector cell according to any one of claims 2-41, wherein the targeting moiety comprises an amino acid sequence set forth in SEQ ID NO: 39, SEQ ID NO: 40, SEQ ID NO: 42, or SEQ ID NO: 43.

43. The immune effector cell according to any one of claims 2-42, wherein the chimeric antigen receptor (CAR) targeting IL13Rα2 comprises the targeting moiety comprising at least one complementarity-determining region (CDR) of the antibody heavy chain variable region (VH), and the VH comprises an amino acid sequence set forth in SEQ ID NO: 56.

44. The immune effector cell according to claim 43, wherein the chimeric antigen receptor (CAR) targeting IL13Rα2 comprises the targeting moiety comprising the VH, the VH comprises the heavy chain complementarity-determining region 1 (HCDR1), the heavy chain complementarity-determining region 2 (HCDR2), and the heavy chain complementarity-determining region 3 (HCDR3), and the HCDR3 comprises an amino acid sequence set forth in SEQ ID NO: 55.

45. The immune effector cell according to claim 44, wherein the HCDR2 comprises an amino acid sequence set forth in SEQ ID NO: 54.

46. The immune effector cell according to any one of claims 44-45, wherein the HCDR1 comprises an amino acid sequence set forth in SEQ ID NO: 53.

47. The immune effector cell according to any one of claims 44-46, wherein the VH comprises: the HCDR1 comprising the amino acid sequence set forth in SEQ ID NO: 53, the HCDR2 comprising the amino acid sequence set forth in SEQ ID NO: 54, and the HCDR3 comprising the amino acid sequence set forth in SEQ ID NO: 55.

48. The immune effector cell according to any one of claims 44-47, wherein the VH comprises the amino acid sequence set forth in SEQ ID NO: 56.

49. The immune effector cell according to any one of claims 43-48, wherein the targeting moiety further comprises at least one CDR of the antibody light chain variable region (VL), and the VL comprises an amino acid sequence set forth in SEQ ID NO: 60.

50. The immune effector cell according to any one of claims 43-49, wherein the targeting moiety comprises at least one CDR of the VH and at least one CDR of the VL, the VH comprises the amino acid sequence set forth in SEQ ID NO: 56, and the VL comprises the amino acid sequence set forth in SEQ ID NO: 60.

51. The immune effector cell according to any one of claims 44-50, wherein the chimeric antigen receptor (CAR) targeting IL13Rα2 comprises the targeting moiety comprising the VL, the VL comprises the light chain complementarity-determining region 1 (LCDR1), the light chain complementarity-determining region 2 (LCDR2), and the light chain complementarity-determining region 3 (LCDR3), and the LCDR1 comprises an amino acid sequence set forth in SEQ ID NO: 57.

52. The immune effector cell according to claim 51, wherein the LCDR2 comprises an amino acid sequence set forth in SEQ ID NO: 58.

53. The immune effector cell according to claim 52, wherein the LCDR3 comprises an amino acid sequence set forth in SEQ ID NO: 59.

54. The immune effector cell according to any one of claims 51-53, wherein the VL comprises: the LCDR1 comprising the amino acid sequence set forth in SEQ ID NO: 57, the LCDR2 comprising the amino acid sequence set forth in SEQ ID NO: 58, and the LCDR3 comprising the amino acid sequence set forth in SEQ ID NO: 59.

55. The immune effector cell according to any one of claims 51-54, wherein the VL comprises the amino acid sequence set forth in SEQ ID NO: 60.

56. The immune effector cell according to any one of claims 44-55, wherein the targeting moiety comprises the VH comprising the amino acid sequence set forth in SEQ ID NO: 56 and the VL comprising the amino acid sequence set forth in SEQ ID NO: 60.

57. The immune effector cell according to any one of claims 43-56, wherein the targeting moiety comprises the full-length antibody, the Fab, the single-chain variable fragment (scFv), or the single-domain antibody (VHH).

58. The immune effector cell according to any one of claims 43-57, wherein the targeting moiety comprises the scFv.

59. The immune effector cell according to any one of claims 43-58, wherein the targeting moiety comprises an amino acid sequence set forth in SEQ ID NO: 61 or SEQ ID NO: 62.

60. The immune effector cell according to any one of claims 43-59, wherein the CAR comprises a transmembrane domain, and the transmembrane domain comprises a transmembrane domain derived from one or more proteins selected from the group consisting of: CD8A, CD8B, CD28, CD3ε (CD3e), 4-1BB, CD4, CD27, CD7, PD-1, TRAC, TRBC, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L (CD154), TIM1, CD226, DR3, CD45, CD80, CD86, CD9, CD16, CD22, CD33, CD37, CD64, and SLAM.

61. The immune effector cell according to claim 60, wherein the transmembrane domain comprises a transmembrane domain derived from CD8A.

62. The immune effector cell according to any one of claims 60-61, wherein the transmembrane domain comprises an amino acid sequence set forth in any one of SEQ ID NO: 69 to SEQ ID NO: 117.

63. The immune effector cell according to any one of claims 60-62, wherein the CAR comprises an intracellular co-stimulatory signaling domain, and the intracellular co-stimulatory signaling domain comprises an intracellular co-stimulatory signaling domain derived from one or more proteins selected from the group consisting of: CD28, 4-1BB (CD137), CD27, CD2, CD7, CD8A, CD8B, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88.

64. The immune effector cell according to claim 63, wherein the intracellular co-stimulatory signaling domain is derived from a co-stimulatory signaling domain of 4-1BB.

65. The immune effector cell according to any one of claims 63-64, wherein the intracellular co-stimulatory signaling domain comprises an amino acid sequence set forth in any one of SEQ ID NO: 118 to SEQ ID NO: 150.

66. The immune effector cell according to any one of claims 60-65, wherein the CAR comprises an intracellular signaling domain, and the intracellular signaling domain comprises an intracellular signaling domain derived from one or more proteins selected from the group consisting of: CD3ζ, CD3δ, CD3γ, CD3ε, CD79a, CD79b, FceRIγ, FceRIβ, FcyRIIa, bovine leukemia virus gp30, Epstein-Barr virus (EBV) LMP2A, simian immunodeficiency virus PBj14 Nef, DAP10, DAP-12, and a domain comprising at least one ITAM.

67. The immune effector cell according to claim 66, wherein the intracellular signaling domain comprises a signaling domain derived from CD3ζ.

68. The immune effector cell according to any one of claims 66-67, wherein the intracellular signaling domain comprises an amino acid sequence set forth in any one of SEQ ID NO: 134, SEQ ID NO: 138, SEQ ID NO: 139, and SEQ ID NO: 151 to SEQ ID NO: 161.

69. The immune effector cell according to any one of claims 60-68, wherein the CAR comprises a hinge region between the targeting moiety and the transmembrane domain, and the hinge region comprises a hinge region derived from one or more proteins selected from the group consisting of: CD28, IgG1, IgG4, IgD, 4-1BB, CD4, CD27, CD7, CD8A, PD-1, ICOS, OX40, NKG2D, NKG2C, FcεRIγ, BTLA, GITR, DAP10, TIM1, SLAM, CD30, and LIGHT.

70. The immune effector cell according to claim 69, wherein the hinge region comprises a hinge region derived from CD8A.

71. The immune effector cell according to any one of claims 69-70, wherein the hinge region comprises an amino acid sequence set forth in any one of SEQ ID NO: 162 to SEQ ID NO: 183.

**72.** The immune effector cell according to any one of claims 60-71, wherein a non-targeting moiety of the chimeric antigen receptor comprises the transmembrane domain of CD8A molecule, the hinge region of CD8A, the intracellular co-stimulatory signaling domain of 4-1BB, and the intracellular signaling domain of CD3ζ.

**73.** The immune effector cell according to claim 72, wherein the non-targeting moiety of the chimeric antigen receptor comprises an amino acid sequence set forth in SEQ ID NO: 67.

**74.** The immune effector cell according to any one of claims 60-73, wherein the chimeric antigen receptor further comprises a signal peptide fragment, and the C-terminus of the signal peptide fragment is linked to the N-terminus of the targeting moiety.

**75.** The immune effector cell according to claim 74, wherein the signal peptide fragment comprises a CD8A signal peptide fragment.

**76.** The immune effector cell according to any one of claims 74-75, wherein the signal peptide fragment comprises an amino acid sequence set forth in SEQ ID NO: 184.

**77.** The immune effector cell according to any one of claims 1-76, wherein the chimeric antigen receptor comprises an amino acid sequence set forth in any one of SEQ ID NO: 45 and SEQ ID NO: 46.

**78.** The immune effector cell according to any one of claims 1-77, wherein the immune effector cell comprises a human cell.

**79.** The immune effector cell according to any one of claims 1-78, wherein the immune effector cell comprises a T cell, a B cell, a natural killer cell (NK cell), a macrophage, an NKT cell, a monocyte, a dendritic cell, a granulocyte, a lymphocyte, a leukocyte, and/or a peripheral blood mononuclear cell.

**80.** The immune effector cell according to any one of claims 1-79, comprising an autologous or non-autologous immune effector cell.

**81.** The immune effector cell according to any one of claims 1-80, comprising a modified immune effector cell, wherein the modification comprises down-regulation of the expression and/or activity of one or more of immune rejection-related genes.

**82.** The immune effector cell according to claim 81, wherein the immune rejection-related gene is selected from one or more of the following groups: TRAC, TRBC, HLA-A, HLA-B, B2M, and CIITA.

**83.** The immune effector cell according to any one of claims 81-82, wherein the expression and/or activity of the TRAC gene and the HLA-A gene in the modified immune effector cell is down-regulated as compared to a corresponding unmodified cell.

**84.** The immune effector cell according to any one of claims 81-83, wherein the expression and/or activity of the CIITA gene in the modified immune effector cell is not down-regulated as compared to the corresponding unmodified cell.

**85.** The immune effector cell according to any one of claims 81-84, wherein the expression and/or activity of the B2M gene in the modified immune effector cell is not down-regulated as compared to the corresponding unmodified cell.

**86.** The immune effector cell according to any one of claims 81-85, wherein the expression and/or activity of the TRAC gene and the HLA-A gene in the modified immune effector cell is down-regulated as compared to a corresponding wild-type cell.

**87.** The immune effector cell according to any one of claims 81-86, wherein the expression and/or activity of the B2M gene in the modified immune effector cell is not down-regulated as compared to the corresponding wild-type cell.

**88.** The immune effector cell according to any one of claims 81-87, wherein the expression and/or activity of the CIITA gene in the modified immune effector cell is not down-regulated as compared to the corresponding wild-type cell.

**89.** The immune effector cell according to any one of claims 81-88, wherein the down-regulation of the expression level and/or activity of the gene comprises down-regulating the expression and/or activity of a nucleic acid molecule encoding the gene; and/or down-regulating the expression and/or activity of a protein product encoded by the gene.

90. The immune effector cell according to any one of claims 81-89, wherein the modification comprises: gene knockout, gene mutation, and/or gene silencing.

91. The immune effector cell according to any one of claims 81-90, wherein the modification comprises knocking out either of two TRAC alleles and knocking out either of two HLA-A alleles in the immune effector cell.

92. The immune effector cell according to any one of claims 81-91, wherein the modification comprises knocking out the two TRAC alleles and knocking out either of the two HLA-A alleles in the immune cell.

93. The immune effector cell according to any one of claims 81-92, wherein the modification comprises knocking out an exon of the TRAC gene and knocking out an exon of the HLA-A gene in the immune cell.

94. The immune effector cell according to any one of claims 81-93, wherein the modification comprises administering to the immune effector cell one or more substances selected from the group consisting of: antisense RNA, siRNA, shRNA, and a CRISPR/Cas9 system.

95. The immune effector cell according to any one of claims 81-94, wherein the modification comprises administering to the immune effector cell the CRISPR/Cas9 system.

96. The immune effector cell according to claim 95, wherein the modification further comprises administering to the immune effector cell sgRNA targeting an exon portion of the TRAC gene.

97. The immune effector cell according to claim 96, wherein the sgRNA targeting the exon portion of the TRAC gene comprises a nucleotide sequence set forth in any one of SEQ ID NO: 186 to SEQ ID NO: 200.

98. The immune effector cell according to any one of claims 95-97, wherein the modification comprises administering to the immune effector cell sgRNA targeting an exon portion of the HLA-A gene.

99. The immune effector cell according to claim 98, wherein the sgRNA targeting the exon portion of the HLA-A gene comprises a nucleotide sequence set forth in any one of SEQ ID NO: 201 to SEQ ID NO: 241.

100. The immune effector cell according to any one of claims 95-99, wherein the modification further comprises administering to the cell a Cas enzyme.

101. The immune effector cell according to claim 100, wherein the Cas enzyme comprises a Cas9 protein.

102. The immune effector cell according to claim 94, wherein the antisense RNA comprises a nucleotide sequence set forth in any one of SEQ ID NO: 242 to SEQ ID NO: 245.

103. The immune effector cell according to any one of claims 1-102, wherein the immune effector cell is an HLA-B homozygous cell.

104. The immune effector cell according to claim 103, wherein the HLA-B homozygote comprises HLA-B*40 homozygote, HLA-B* 15 homozygote, HLA-B*46 homozygote, HLA-B * 13 homozygote, HLA-B * 51 homozygote, HLA-B * 58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

105. The immune effector cell according to any one of claims 1-104, wherein the immune effector cell is an HLA-A homozygous or heterozygous cell.

106. The immune effector cell according to claim 105, wherein the HLA-A homozygote or heterozygote comprises HLA-A*02 homozygote, HLA-A* 11 homozygote, HLA-A*02/A* 11 heterozygote, or HLA-A*24 homozygote.

107. A method for preparing an immune effector cell, comprising: modifying the immune effector cell before/after introducing a polynucleotide sequence encoding the CAR targeting IL13Rα2 according to any one of claims 1-106 or a vector comprising the polynucleotide sequence encoding the CAR targeting IL13Rα2 according to any one of claims 1-106 into the immune effector cell, wherein the modification comprises down-regulation of the expression and/or activity of one or more of immune rejection-related genes.

108. The method according to claim 107, wherein the vector is an expression vector.

109. The method according to any one of claims 107-108, wherein the vector is selected from a DNA vector, an RNA vector, a plasmid, a lentiviral vector, an adenoviral vector, an adeno-associated viral vector, and a retroviral vector.

110. The method according to any one of claims 107-109, wherein the immune rejection-related genes are selected from one or more of the following genes: TRAC, TRBC, HLA-A, HLA-B, B2M, and CIITA.

111. The method according to any one of claims 107-110, wherein the expression and/or activity of the TRAC gene and the HLA-A gene in the immune effector cell is down-regulated as compared to the expression and/or activity of a corresponding gene in a corresponding unmodified cell.

112. The method according to any one of claims 107-111, wherein the expression and/or activity of the CIITA gene is not down-regulated as compared to the expression and/or activity of the corresponding gene in the corresponding unmodified cell.

113. The method according to any one of claims 107-112, wherein the expression and/or activity of the B2M gene is not down-regulated as compared to the expression and/or activity of the corresponding gene in the corresponding unmodified cell.

114. The method according to any one of claims 107-113, wherein the expression and/or activity of the TRAC gene and the HLA-A gene in the immune effector cell is down-regulated as compared to a corresponding wild-type cell.

115. The method according to any one of claims 107-114, wherein the expression and/or activity of the CIITA gene is not down-regulated as compared to the corresponding wild-type cell.

116. The method according to any one of claims 107-115, wherein the expression and/or activity of the B2M gene is not down-regulated as compared to the corresponding wild-type cell.

117. The method according to any one of claims 107-116, wherein the down-regulation of the expression level and/or activity of the gene comprises down-regulating the expression and/or activity of a nucleic acid molecule encoding the gene; and/or down-regulating the expression and/or activity of a protein product encoded by the gene.

118. The method according to any one of claims 107-117, wherein the modification comprises: gene knockout, gene mutation, and/or gene silencing.

119. The method according to any one of claims 107-118, wherein the modification comprises knocking out either of two TRAC alleles and knocking out either of two HLA-A alleles in the immune effector cell.

120. The method according to any one of claims 107-119, wherein the modification comprises knocking out the two TRAC alleles and knocking out either of the two HLA-A alleles in the immune cell.

121. The method according to any one of claims 107-120, wherein the modification comprises knocking out an exon of the TRAC gene and knocking out an exon of the HLA-A gene in the immune cell.

122. The method according to any one of claims 107-121, wherein the modification comprises administering to the immune effector cell one or more substances selected from the group consisting of: antisense RNA, siRNA, shRNA, and a CRISPR/Cas9 system.

123. The method according to any one of claims 107-122, wherein the modification comprises administering to the immune effector cell the CRISPR/Cas9 system.

124. The method according to claim 123, wherein the modification comprises administering to the immune effector cell sgRNA targeting an exon portion of the TRAC gene.

125. The method according to claim 124, wherein the sgRNA targeting the exon portion of the TRAC gene comprises a nucleotide sequence set forth in any one of SEQ ID NO: 186 to SEQ ID NO: 200.

126. The method according to any one of claims 123-125, wherein the modification comprises administering to the immune effector cell sgRNA targeting an exon portion of the HLA-A gene.

127. The method according to claim 126, wherein the sgRNA targeting the exon portion of the HLA-A gene comprises a nucleotide sequence set forth in any one of SEQ ID NO: 201 to SEQ ID NO: 241.

128. The method according to any one of claims 123-127, wherein the modification further comprises administering to the cell a Cas enzyme.

129. The method according to claim 128, wherein the Cas enzyme comprises a Cas9 protein.

130. The method according to claim 122, wherein the antisense RNA comprises a nucleotide sequence set forth in any one of SEQ ID NO: 242 to SEQ ID NO: 245.

131. The method according to any one of claims 107-130, wherein the immune effector cell comprises a human cell.

132. The method according to any one of claims 107-131, wherein the immune effector cell comprises a T cell, a B cell, a natural killer cell (NK cell), a macrophage, an NKT cell, a monocyte, a dendritic cell, a granulocyte, a lymphocyte, a leukocyte, and/or a peripheral blood mononuclear cell.

133. The method according to any one of claims 107-132, wherein the immune effector cell comprises an autologous or non-autologous immune effector cell.

134. The method according to any one of claims 107-133, wherein the cell is an HLA-B homozygous cell.

135. The method according to claim 134, wherein the HLA-B homozygote comprises HLA-B*40 homozygote, HLA-B*15 homozygote, HLA-B*46 homozygote, HLA-B*13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

136. The method according to any one of claims 107-135, wherein the cell is an HLA-A homozygous or heterozygous cell.

137. The method according to claim 136, wherein the HLA-A homozygote or heterozygote comprises HLA-A*02 homozygote, HLA-A* 11 homozygote, HLA-A*02/A* 11 heterozygote, or HLA-A*24 homozygote.

138. Use of the immune effector cell according to any one of claims 1-106 in the preparation of a CAR-T cell.

139. A pharmaceutical composition comprising the immune effector cell according to any one of claims 1-106, and optionally a pharmaceutically acceptable carrier.

140. Use of the immune effector cell according to any one of claims 1-106 and/or the pharmaceutical composition according to claim 139 in the treatment of a disease or disorder associated with the expression of IL13R$\alpha$2.

141. The use according to claim 140, wherein the disease or disorder associated with the expression of IL13R$\alpha$2 comprises a disease or disorder associated with up-regulation of the expression of IL13R$\alpha$2.

142. The use according to any one of claims 140-141, wherein the disease or disorder associated with the expression of IL13R$\alpha$2 comprises cancer.

143. The use according to claim 142, wherein the cancer comprises an IL13R$\alpha$-positive tumor.

144. The use according to claim 143, wherein the IL13R$\alpha$-positive tumor comprises malignant gliomas, colorectal cancer, cervical cancer, pancreatic cancer, cutaneous melanoma, ovarian cancer, breast cancer, adrenocortical carcinoma, thymoma, uterine cancer, bladder cancer, esophageal cancer, head and neck squamous cell carcinoma, or gastric cancer.

145. Use of the immune effector cell according to any one of claims 1-106 and/or the pharmaceutical composition according to claim 139 in the preparation of a medicament for treating a disease or disorder associated with the expression of IL13Rα2.

146. The use according to claim 145, wherein the disease or disorder associated with the expression of IL13Rα2 comprises a disease or disorder associated with up-regulation of the expression of IL13Rα2.

147. The use according to any one of claims 145-146, wherein the disease or disorder associated with the expression of IL13Rα2 comprises cancer.

148. The use according to claim 147, wherein the cancer comprises an IL13Rα-positive tumor.

149. The use according to claim 148, wherein the IL13Rα-positive tumor comprises malignant gliomas, colorectal cancer, cervical cancer, pancreatic cancer, cutaneous melanoma, ovarian cancer, breast cancer, adrenocortical carcinoma, thymoma, uterine cancer, bladder cancer, esophageal cancer, head and neck squamous cell carcinoma, or gastric cancer.

150. A method for preventing or treating a disease or disorder associated with the expression of IL13Rα2, comprising administering to a subject in need thereof an effective amount of the immune effector cell according to any one of claims 1-106 and/or the pharmaceutical composition according to claim 139.

151. The method according to claim 150, wherein the disease or disorder associated with the expression of IL13Rα2 comprises a disease or disorder associated with up-regulation of the expression of IL13Rα2.

152. The method according to any one of claims 150-151, wherein the disease or disorder associated with the expression of IL13Rα2 comprises cancer.

153. The method according to any one of claim 152, wherein the cancer comprises an IL13Rα-positive tumor.

154. The method according to claim 153, wherein the IL13Rα-positive tumor comprises malignant gliomas, colorectal cancer, cervical cancer, pancreatic cancer, cutaneous melanoma, ovarian cancer, breast cancer, adrenocortical carcinoma, thymoma, uterine cancer, bladder cancer, esophageal cancer, head and neck squamous cell carcinoma, or gastric cancer.

FIG. 1

FIG. 2

FIG. 3A

FIG. 3B

FIG. 3C

## IL13RA2UCAR-T cell killing

FIG. 4

FIG. 5A

FIG. 5B

FIG. 5C

## Tumor volume

*In vivo* anti-tumor effect of IL13RA2 UCAR-T cells

FIG. 6

## IL13Ra2 CAR copies

FIG. 7

**Irradiated allogenic PBMC**

FIG. 8

**allogenic PBMC**

**Irradiated**

FIG. 9

## IL-2

FIG. 10A

## IL-6

FIG. 10B

## IFN-γ

FIG. 10C

## TNF-a

A (UCAR-T:KO>95%)
B (UCAR-T:KO>90%)
C (UCAR-T:KO>80%)
D (CAR-T)

FIG. 10D

IL13RA2 UCART *in vivo* rejection

IL13RA2 UCAR-T (TRAC + HLA-A double knockout)
IL13RA2 UCAR-T (TRAC + B2M double knockout)
IL13RA2 UCAR-T (TRAC single knockout)

FIG. 11

IL13RA2 UCART *in vivo* rejection

IL13RA2 UCAR-T (TRAC + HLA-A double knockout)
IL13RA2 UCAR-T (TRAC + B2M double knockout)
IL13RA2 UCAR-T (TRAC single knockout)

FIG. 12

U3-10_1: TRAC

Off-target display after removal of control background

U3-10_1

U3-10_2: HLA-A02

Off-target display after removal of control background

U3-10_2

FIG. 13

Chromosome translocation

**T1: TRAC upper strand:HLA-A02 upper strand linked together**
**T2: TRAC upper strand:HLA-A02 lower strand linked together**
**10⁻⁴: not detected**

FIG. 14

FIG. 15

**Residual Cas9**

D3: day 3 before electroporation
D5-: day 5 before buffer exchanged and after electroporation
N.D.: not detected

FIG. 16

FIG. 17

FIG. 18

| Specimen_001-Tube_001 | Specimen_001-2 | Specimen_001-3 |
|---|---|---|
| Negative control group | Wild-type control group | TRAC gene knockout group |

FIG. 19

RNP sg2

GGGGTCGGACTGGCGCTTCCTCCGCGGGTACCACCAGTACGCCTACGACGGCAAGGATTACAT

GTNTGGGNNGGGCTTTTTCTCCG--GGTACCANCNNTACGCCTACGACGGCAAGGATTACAT

FIG. 20

ATATGACTCACCACGCTGTCTCTGACCATGAAGCCACCCTGAGGTGCTGGGCCCTG

ATATGACTCACCACGCTGTCTCTGACCATGAAGCCACC-TGAGGTGCTGGGCCCTG

280    270    260    250    240    230

FIG. 21

ACGCCGCGAGCCAGAGGATGGAGCCGCGGGCGCCGTGGATAGAGCAGGAGGGGCCG

ACNCCNCGANCCAGAGGANGGAGCCGCGGGCCCCGTGGATAGAGCAGGAGGGGCCG

210    200    190    180    170    160

FIG. 22

GGGCCGGACGGGCGCTTCCTCCGCGGGTACCGGCAGGACGCCTACGACGGCA

GGGCCGGACGGGCGCTTCCTCCGGGGGTACCACCGGGACCCTTACAACGGCA

C120  Mass: 50

FIG. 23

FIG. 24A

FIG. 24B

FIG. 25A

FIG. 25B

FIG. 26

FIG. 27A

FIG. 27B

FIG. 28A

FIG. 28B

FIG. 28C

FIG. 28D

FIG. 29A

FIG. 29B

FIG. 30A

FIG. 30B

FIG. 31

FIG. 32

FIG. 33A

FIG. 33B

FIG. 33C

FIG. 33D

FIG. 34

FIG. 35

**Cytotoxicity (100%)**

FIG. 36

Time (days)

FIG. 37

**Number of days**

FIG. 38

## INTERNATIONAL SEARCH REPORT

| International application No. |
|---|
| **PCT/CN2022/103037** |

### A. CLASSIFICATION OF SUBJECT MATTER

A61K 35/17(2015.01)i; C07K 14/54(2006.01)i

According to International Patent Classification (IPC) or to both national classification and IPC

### B. FIELDS SEARCHED

Minimum documentation searched (classification system followed by classification symbols)

A61K; C07K

Documentation searched other than minimum documentation to the extent that such documents are included in the fields searched

Electronic data base consulted during the international search (name of data base and, where practicable, search terms used)

CNTXT, ENTXT, VEN, ISI, CNKI, 万方数据库, WANFANG DATABASE, ISI, NCBI, STN, 百度学术, BAIDU SCHOLAR: 序列检索, Sequence search, CAR, 嵌合抗原受体, CAR-T, 免疫效应细胞, TCR, MHC, HLA-A, 敲除, 抑制, knockout, deficient, 免疫排斥, GVHD, IL13Rα2

### C. DOCUMENTS CONSIDERED TO BE RELEVANT

| Category* | Citation of document, with indication, where appropriate, of the relevant passages | Relevant to claim No. |
|---|---|---|
| Y | CN 112236151 A (SEATTLE CHILDREN'S HOSPITAL (DBA SEATTLE CHILDREN'S RESEARCH INSTITUTE)) 15 January 2021 (2021-01-15) claims 1-66, description, paragraphs 7-45, 113-114, 135, 140-141, and 172, and table 2 | 1-154 |
| Y | CN 104936621 A (PFIZER INC.) 23 September 2015 (2015-09-23) description, paragraphs 106 and 162-168 | 2-154 |
| Y | CN 107723275 A (CHONGQING PRECISION BIOTECH CO., LTD.) 23 February 2018 (2018-02-23) claims 1-20, and description, paragraphs 7-11 | 1-154 |
| Y | CN 111094345 A (U.S. DEPARTMENT OF HEALTH AND HUMAN SERVICES et al.) 01 May 2020 (2020-05-01) tables A, SEQ ID NOs: 21-24 | 60-154 |
| Y | CN 109456943 A (GRACELL BIOTECHNOLOGIES (SHANGHAI) CO., LTD.) 12 March 2019 (2019-03-12) claims 1-10, and description, paragraph 125 | 1-154 |

☑ Further documents are listed in the continuation of Box C.  ☑ See patent family annex.

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| Date of the actual completion of the international search | Date of mailing of the international search report |
|---|---|
| **14 September 2022** | **10 October 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/103037**

### C.    DOCUMENTS CONSIDERED TO BE RELEVANT

| Category* | Citation of document, with indication, where appropriate, of the relevant passages | Relevant to claim No. |
|---|---|---|
| Y | US 2014349402 A1 (BOARD OF REGENTS, THE UNIVERSITY OF TEXAS SYSTEM) 27 November 2014 (2014-11-27)<br>    embodiments 8 and 9 | 1-154 |
| Y | 方雅玲 等 (FANG, Yaling et al.). "同种异体CAR-T细胞的研究进展 (Research Progress in Allogeneic CAR-T Cells)"<br>肿瘤 (Tumor), Vol. 41, 31 May 2021 (2021-05-31),<br>    pp. 363-370 | 1-154 |
| Y | LIU, Xiaojuan et al. "CRISPR-Cas9-Mediated Multiplex Gene Editing in CAR-T Cells"<br>Cell Research, Vol. 27, 02 December 2016 (2016-12-02),<br>    pp. 154-157 | 1-154 |

Form PCT/ISA/210 (second sheet) (January 2015)

**INTERNATIONAL SEARCH REPORT**

International application No.

**PCT/CN2022/103037**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

        ☑ in the form of an Annex C/ST.25 text file.

        ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

| International application No. |
|---|
| **PCT/CN2022/103037** |

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **150-154**
because they relate to subject matter not required to be searched by this Authority, namely:

[1] Claims 150-154 relate to a method for preventing or treating diseases or disorders related to expression of IL13Rα2. That is, claims 150-154 relate to the subject matter defined in PCT Rule 39.1 that does not warrant a search conducted by the international searching authority: (4) methods for the treatment of a human or animal body by surgery or therapy, as well as diagnostic methods implemented on a human or animal body. An international search was conducted on the basis of a use of an effective amount of the immune effector cell according to any one of claims 1-106 and/or the pharmaceutical composition according to claim 139 in the preparation of a drug for preventing or treating the diseases or disorders related to the expression of IL13Rα2.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

<table>
<tr><td colspan="5" align="center">**INTERNATIONAL SEARCH REPORT**<br>Information on patent family members</td><td colspan="2">International application No.<br>**PCT/CN2022/103037**</td></tr>
<tr><td colspan="3" align="center">Patent document<br>cited in search report</td><td align="center">Publication date<br>(day/month/year)</td><td colspan="2" align="center">Patent family member(s)</td><td align="center">Publication date<br>(day/month/year)</td></tr>
<tr><td>CN</td><td>112236151</td><td>A</td><td>15 January 2021</td><td>AU</td><td>2019234573 A1</td><td>08 October 2020</td></tr>
<tr><td></td><td></td><td></td><td></td><td>US</td><td>2021000875 A1</td><td>07 January 2021</td></tr>
<tr><td></td><td></td><td></td><td></td><td>IL</td><td>277267 A</td><td>29 October 2020</td></tr>
<tr><td></td><td></td><td></td><td></td><td>WO</td><td>2019178078 A1</td><td>19 September 2019</td></tr>
<tr><td></td><td></td><td></td><td></td><td>EA</td><td>202091982 A1</td><td>10 June 2021</td></tr>
<tr><td></td><td></td><td></td><td></td><td>EP</td><td>3765041 A1</td><td>20 January 2021</td></tr>
<tr><td></td><td></td><td></td><td></td><td>SG</td><td>11202008795 S A</td><td>29 October 2020</td></tr>
<tr><td></td><td></td><td></td><td></td><td>KR</td><td>20200131279 A</td><td>23 November 2020</td></tr>
<tr><td></td><td></td><td></td><td></td><td>CA</td><td>3093810 A1</td><td>19 September 2019</td></tr>
<tr><td></td><td></td><td></td><td></td><td>JP</td><td>2021518108 A</td><td>02 August 2021</td></tr>
<tr><td></td><td></td><td></td><td></td><td>BR</td><td>112020018670 A2</td><td>05 January 2021</td></tr>
<tr><td>CN</td><td>104936621</td><td>A</td><td>23 September 2015</td><td>JP</td><td>2016503295 A</td><td>04 February 2016</td></tr>
<tr><td></td><td></td><td></td><td></td><td>MX</td><td>2015005582 A</td><td>14 August 2015</td></tr>
<tr><td></td><td></td><td></td><td></td><td>HK</td><td>1214161 A1</td><td>22 July 2016</td></tr>
<tr><td></td><td></td><td></td><td></td><td>CA</td><td>2890256 A1</td><td>15 May 2014</td></tr>
<tr><td></td><td></td><td></td><td></td><td>SG</td><td>11201503431 T A</td><td>28 May 2015</td></tr>
<tr><td></td><td></td><td></td><td></td><td>PE</td><td>20150891 A1</td><td>11 June 2015</td></tr>
<tr><td></td><td></td><td></td><td></td><td>EP</td><td>2916875 A1</td><td>16 September 2015</td></tr>
<tr><td></td><td></td><td></td><td></td><td>KR</td><td>20150060980 A</td><td>03 June 2015</td></tr>
<tr><td></td><td></td><td></td><td></td><td>PH</td><td>12015500977 A1</td><td>13 July 2015</td></tr>
<tr><td></td><td></td><td></td><td></td><td>WO</td><td>2014072888 A1</td><td>15 May 2014</td></tr>
<tr><td></td><td></td><td></td><td></td><td>US</td><td>2015266962 A1</td><td>24 September 2015</td></tr>
<tr><td></td><td></td><td></td><td></td><td>IL</td><td>238566 A</td><td>30 June 2015</td></tr>
<tr><td></td><td></td><td></td><td></td><td>AU</td><td>2013343111 A1</td><td>14 May 2015</td></tr>
<tr><td></td><td></td><td></td><td></td><td>RU</td><td>2015116485 A</td><td>27 December 2016</td></tr>
<tr><td>CN</td><td>107723275</td><td>A</td><td>23 February 2018</td><td>EP</td><td>3699268 A1</td><td>26 August 2020</td></tr>
<tr><td></td><td></td><td></td><td></td><td>WO</td><td>2019076149 A1</td><td>25 April 2019</td></tr>
<tr><td>CN</td><td>111094345</td><td>A</td><td>01 May 2020</td><td>JP</td><td>2020529841 A</td><td>15 October 2020</td></tr>
<tr><td></td><td></td><td></td><td></td><td>WO</td><td>2019006072 A1</td><td>03 January 2019</td></tr>
<tr><td></td><td></td><td></td><td></td><td>AU</td><td>2018291128 A1</td><td>23 January 2020</td></tr>
<tr><td></td><td></td><td></td><td></td><td>SG</td><td>11201913175 T A</td><td>30 January 2020</td></tr>
<tr><td></td><td></td><td></td><td></td><td>US</td><td>2020138865 A1</td><td>07 May 2020</td></tr>
<tr><td></td><td></td><td></td><td></td><td>IL</td><td>271597 A</td><td>27 February 2020</td></tr>
<tr><td></td><td></td><td></td><td></td><td>KR</td><td>20200049757 A</td><td>08 May 2020</td></tr>
<tr><td></td><td></td><td></td><td></td><td>EP</td><td>3645568 A1</td><td>06 May 2020</td></tr>
<tr><td></td><td></td><td></td><td></td><td>CA</td><td>3068444 A1</td><td>03 January 2019</td></tr>
<tr><td>CN</td><td>109456943</td><td>A</td><td>12 March 2019</td><td>CN</td><td>111051502 A</td><td>21 April 2020</td></tr>
<tr><td></td><td></td><td></td><td></td><td>WO</td><td>2019047899 A1</td><td>14 March 2019</td></tr>
<tr><td></td><td></td><td></td><td></td><td>EP</td><td>3680328 A1</td><td>15 July 2020</td></tr>
<tr><td>US</td><td>2014349402</td><td>A1</td><td>27 November 2014</td><td>WO</td><td>2013074916 A1</td><td>23 May 2013</td></tr>
<tr><td></td><td></td><td></td><td></td><td>US</td><td>2019388472 A1</td><td>26 December 2019</td></tr>
</table>

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014072888 A1
- WO 2021041725 A1

**Non-patent literature cited in the description**

- **MILLER et al.** *Jour. of Immunology,* 2003, vol. 170, 4854-4861
- **WARD et al.** Binding Activities of a Repertoire of Single Immunoglobulin Variable Domains Secreted From Escherichia coli. *Nature,* 1989, vol. 341, 544-546
- **HUSTON et al.** Protein Engineering of Antibody Binding Sites: Recovery of Specific Activity in an Anti-Digoxin Single-Chain Fv Analogue Produced in Escherichia coli. *Proc. Natl. Acad. Sci. USA,* 1988, vol. 85, 5879-5883
- **NGUYEN V. K. et al.** *The EMBO Journal,* 2000, vol. 19, 921-930
- **MUYLDERMANS S.** *J Biotechnol.,* 2001, vol. 74, 277-302
- **VANLANDSCHOOT P. et al.** *Antiviral Research,* 2011, vol. 92, 389-407
- **KANG XIAOZHEN et al.** *Chinese Journal of Biotechnology,* 2018, vol. 34 (12), 1974-1984
- **KABAT et al.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 877-883
- **PADLAN.** *FASEB J.,* 1995, vol. 9, 133-139
- **MACCALLUM.** *J Mol Biol,* 1996, vol. 262 (5), 732-45
- *PLoS One.,* 16 October 2013, vol. 8 (10), e77719
- *Breast Cancer Research,* 2015, vol. 17 (1